# EUROPEAN PATENT APPLICATION

(11) **EP 2 093 281 A1**
(43) Date of publication of application: **26.08.2009**
(21) Application number: 08380046.6
(22) Date of filing: 19.02.2008
(51) Int. Cl.: C12N 7/04, C07K 14/08, C12N 15/62

(54) **Protein nanocarriers, process for obtaining them and applications**

(71) Applicant: Kapsid Link, S.L., 28017 Madrid (ES)
(72) Inventor: Rodríguez Aguirre, José Francisco, 28049 Madrid (ES); Oña Blanco, Ana Maria, 28049 Madrid (ES)
(74) Representative: ABG Patentes, S.L.

(57) **Abstract**

The invention relates to nanocarriers formed by protein assemblies, obtained by means of the assembly of a genetically modified self assembly protein in order to couple products of interest bound to a ligand. These protein nanocarriers can be used as a system for carrying and presenting products of interest.

SEQUENCE LISTING

<110>  KAPSID LINK, S.L.

<120>  Protein nanocarriers, process for obtaining them and applications

<130>  P3134EP00

<160>  24

<170>  PatentIn version 3.3

<210>  1
<211>  452
<212>  PRT
<213>  Infectious bursal disease virus (IBDV)

<220>
<223> IBDV pVP2_452

<400>  1

Met Thr Asn Leu Ser Asp Gln Thr Gln Gln Ile Val Pro Phe Ile Arg
1               5                   10                  15

Ser Leu Leu Met Pro Thr Thr Gly Pro Ala Ser Ile Pro Asp Asp Thr
            20                  25                  30

Leu Glu Lys His Thr Leu Arg Ser Glu Thr Ser Thr Tyr Asn Leu Thr
        35                  40                  45

Val Gly Asp Thr Gly Ser Gly Leu Ile Val Phe Phe Pro Gly Phe Pro
    50                  55                  60

Gly Ser Ile Val Gly Ala His Tyr Thr Leu Gln Gly Asn Gly Asn Tyr
65                  70                  75                  80

Lys Phe Asp Gln Met Leu Leu Thr Ala Gln Asn Leu Pro Ala Ser Tyr
                85                  90                  95

Asn Tyr Cys Arg Leu Val Ser Arg Ser Leu Thr Val Arg Ser Ser Thr
            100                 105                 110

Leu Pro Gly Gly Val Tyr Ala Leu Asn Gly Thr Ile Asn Ala Val Thr
        115                 120                 125

Phe Gln Gly Ser Leu Ser Glu Leu Thr Asp Val Ser Tyr Asn Gly Leu
    130                 135                 140

Met Ser Ala Thr Ala Asn Ile Asn Asp Lys Ile Gly Asn Val Leu Val
145                 150                 155                 160

Gly Glu Gly Val Thr Val Leu Ser Leu Pro Thr Ser Tyr Asp Leu Gly
                165                 170                 175

Tyr Val Arg Leu Gly Asp Pro Ile Pro Ala Ile Gly Leu Asp Pro Lys
            180                 185                 190

Met Val Ala Thr Cys Asp Ser Ser Asp Arg Pro Arg Val Tyr Thr Ile
        195                 200                 205

Thr Ala Ala Asp Asp Tyr Gln Phe Ser Ser Gln Tyr Gln Pro Gly Gly
    210                 215                 220

Val Thr Ile Thr Leu Phe Ser Ala Asn Ile Asp Ala Ile Thr Ser Leu
225                 230                 235                 240

Ser Val Gly Gly Glu Leu Val Phe Arg Thr Ser Val His Gly Leu Val
                245                 250                 255

Leu Gly Ala Thr Ile Tyr Leu Ile Gly Phe Asp Gly Thr Thr Val Ile
            260                 265                 270

Thr Arg Ala Val Ala Ala Asn Asn Gly Leu Thr Thr Gly Thr Asp Asn
        275                 280                 285

Leu Met Pro Phe Asn Leu Val Ile Pro Thr Asn Glu Ile Thr Gln Pro
    290                 295                 300

Ile Thr Ser Ile Lys Leu Glu Ile Val Thr Ser Lys Ser Gly Gly Gln
305                 310                 315                 320

Ala Gly Asp Gln Met Ser Trp Ser Ala Arg Gly Ser Leu Ala Val Thr
                325                 330                 335

Ile His Gly Gly Asn Tyr Pro Gly Ala Leu Arg Pro Val Thr Leu Val
            340                 345                 350

Ala Tyr Glu Arg Val Ala Thr Gly Ser Val Val Thr Val Ala Gly Val
        355                 360                 365

Ser Asn Phe Glu Leu Ile Pro Asn Pro Glu Leu Ala Lys Asn Leu Val
    370                 375                 380

Thr Glu Tyr Gly Arg Phe Asp Pro Gly Ala Met Asn Tyr Thr Lys Leu
385                 390                 395                 400

Ile Leu Ser Glu Arg Asp Arg Leu Gly Ile Lys Thr Val Trp Pro Thr
                405                 410                 415

Arg Glu Tyr Thr Asp Phe Arg Glu Tyr Phe Met Glu Val Ala Asp Leu
            420                 425                 430

Asn Ser Pro Leu Lys Ile Ala Gly Ala Phe Gly Phe Lys Asp Ile Ile
        435                 440                 445

Arg Ala Ile Arg
    450

<210>  2
<211>
<212>  PRT
<213>

<220>
<221> region
<222> 2,3
<223> Xaa in position 2 is any amino acid; in position 3 it is any amino
acid other than Leu, Val, Ile, Trp, Phe or Tyr.

<220>
<221> region
<222> 5, 6, 7, 8
<223> Xaa in position 5 is Phe or Leu, in position 6 it is Glu or Asp, in
position 7 it is Ala, Gly, Ser or Thr, in position 8

<220>
<221> region
<222> 10, 11, 12, 13
<223> Xaa in position 10 is Ile, Met or Val; in position 11 it is Glu,
Leu, Val, Tyr or Ile, in position 12 it is Thr, Tyt, Val, Phe and Leu

<400>  2

Leu Xaa Xaa Ile Xaa Xaa Xaa Xaa Lys Xaa Xaa Xaa Xaa
                5                   10

<210>  3
<211>  15
<212>  PRT
<213>  Artificial

<220>
<223>  Amino acid sequence corresponding to the BAP peptide

<400>  3

Gly Leu Asn Asp Ile Phe Glu Ala Gln Lys Ile Glu Trp His Glu
1               5                   10                  15

<210>  4
<211>  466
<212>  PRT
<213>  Artificial

<220>
<223>  Fusion protein SAP_BAP (pVP2-452-BAP)

<400>  4

Met Thr Asn Leu Ser Asp Gln Thr Gln Gln Ile Val Pro Phe Ile Arg
1               5                   10                  15

Ser Leu Leu Met Pro Thr Thr Gly Pro Ala Ser Ile Pro Asp Asp Thr
            20                  25                  30

Leu Glu Lys His Thr Leu Arg Ser Glu Thr Ser Thr Tyr Asn Leu Thr
        35                  40                  45

Val Gly Asp Thr Gly Ser Gly Leu Ile Val Phe Phe Pro Gly Phe Pro
    50                  55                  60

Gly Ser Ile Val Gly Ala His Tyr Thr Leu Gln Gly Asn Gly Asn Tyr
65                  70                  75                  80

Lys Phe Asp Gln Met Leu Leu Thr Ala Gln Asn Leu Pro Ala Ser Tyr
                85                  90                  95

Asn Tyr Cys Arg Leu Val Ser Arg Ser Leu Thr Val Arg Ser Ser Thr
            100                 105                 110

Leu Pro Gly Gly Val Tyr Ala Leu Asn Gly Thr Ile Asn Ala Val Thr
        115                 120                 125

Phe Gln Gly Ser Leu Ser Glu Leu Thr Asp Val Ser Tyr Asn Gly Leu
    130                 135                 140

Met Ser Ala Thr Ala Asn Ile Asn Asp Lys Ile Gly Asn Val Leu Val
145                 150                 155                 160

Gly Glu Gly Val Thr Val Leu Ser Leu Pro Thr Ser Tyr Asp Leu Gly
                165                 170                 175

Tyr Val Arg Leu Gly Asp Pro Ile Pro Ala Ile Gly Leu Asp Pro Lys
            180                 185                 190

Met Val Ala Thr Cys Asp Ser Ser Asp Arg Pro Arg Val Tyr Thr Ile
        195                 200                 205

Thr Ala Ala Asp Asp Tyr Gln Phe Ser Ser Gln Tyr Gln Pro Gly Gly
    210                 215                 220

Val Thr Ile Thr Leu Phe Ser Ala Asn Ile Asp Ala Ile Thr Ser Leu
225                 230                 235                 240

Ser Val Gly Gly Glu Leu Val Phe Arg Thr Ser Val His Gly Leu Val
                245                 250                 255

Leu Gly Ala Thr Ile Tyr Leu Ile Gly Phe Asp Gly Thr Thr Val Ile
            260                 265                 270

Thr Arg Ala Val Ala Ala Asn Asn Gly Leu Thr Thr Gly Thr Asp Asn
        275                 280                 285

Leu Met Pro Phe Asn Leu Val Ile Pro Thr Asn Glu Ile Thr Gln Pro
    290                 295                 300

Ile Thr Ser Ile Lys Leu Glu Ile Val Thr Ser Lys Ser Gly Gly Gly
305                 310                 315                 320

Leu Asn Asp Ile Phe Glu Ala Gln Lys Ile Glu Trp His Glu Ala Gly
                325                 330                 335

Asp Gln Met Ser Trp Ser Ala Arg Gly Ser Leu Ala Val Thr Ile His
            340                 345                 350

Gly Gly Asn Tyr Pro Gly Ala Leu Arg Pro Val Thr Leu Val Ala Tyr
        355                 360                 365

Glu Arg Val Ala Thr Gly Ser Val Val Thr Val Ala Gly Val Ser Asn
    370                 375                 380

Phe Glu Leu Ile Pro Asn Pro Glu Leu Ala Lys Asn Leu Val Thr Glu
385                 390                 395                 400

Tyr Gly Arg Phe Asp Pro Gly Ala Met Asn Tyr Thr Lys Leu Ile Leu
                405                 410                 415

Ser Glu Arg Asp Arg Leu Gly Ile Lys Thr Val Trp Pro Thr Arg Glu
            420                 425                 430

Tyr Thr Asp Phe Arg Glu Tyr Phe Met Glu Val Ala Asp Leu Asn Ser
        435                 440                 445

Pro Leu Lys Ile Ala Gly Ala Phe Gly Phe Lys Asp Ile Ile Arg Ala
    450                 455                 460

Ile Arg
465

<210>  5
<211>  1401
<212>  DNA
<213>  Artificial

<220>
<223>  Open reading phase corresponding to chimeric gene SAP_BAP
(pVP2_452-BAP)

<400>  5
atgacaaacc tgtcagatca aacccagcag attgttccgt tcatacggag ccttctgatg      60

ccaacaaccg gaccggcgtc cattccggac gacaccctgg agaagcacac tctcaggtca     120

gagacctcga cctacaattt gactgtgggg gacacagggt cagggctaat tgtctttttc     180

cctggattcc ctggctcaat tgtgggtgct cactacacac tgcagggcaa tgggaactac     240

aagttcgatc agatgctcct gactgcccag aacctaccgg ccagttacaa ctactgcagg     300

ctagtgagtc ggagtctcac agtgaggtca agcacacttc ctggtggcgt ttatgcacta     360

aacggcacca taaacgccgt gaccttccaa ggaagcctga gtgaactgac agatgttagc     420

tacaatgggt tgatgtctgc aacagccaac atcaacgaca aaattgggaa cgtcctagta     480

ggggaagggg tcaccgtcct cagcttaccc acatcatatg atcttgggta tgtgaggctt     540

ggtgacccca ttcccgcaat agggcttgac ccaaaaatgg tagccacatg tgacagcagt     600

gacaggccca gagtctacac cataactgca gccgatgatt accaattctc atcacagtac     660

caaccaggtg gggtaacaat cacactgttc tcagccaaca ttgatgccat cacaagcctc     720

agcgttgggg gagagctcgt gtttcgaaca agcgtccacg gccttgtact gggcgccacc     780

atctacctca taggctttga tgggacaacg gtaatcacca gggctgtggc cgcaaacaat     840

gggctgacga ccggcaccga caaccttatg ccattcaatc ttgtgattcc aacaaacgag     900

ataacccagc caatcacatc catcaaactg gagatagtga cctccaaaag tggtggtggc     960

ctgaacgaca tcttcgaggc tcagaaaatc gaatggcacg aagcagggga tcagatgtca    1020

tggtcggcaa gagggagcct agcagtgacg atccatggtg gcaactatcc aggggccctc    1080

cgtcccgtca cgctagtggc ctacgaaaga gtggcaacag gatccgtcgt tacggtcgct    1140

ggggtgagca acttcgagct gatcccaaat cctgaactag caaagaacct ggttacagaa 1200

tacggccgat ttgacccagg agccatgaac tacacaaaat tgatactgag tgagagggac 1260

cgtcttggca tcaagaccgt ctggccaaca agggagtaca ctgactttcg tgaatacttc 1320

atggaggtgg ccgacctcaa ctctcccctg aagattgcag gagcattcgg cttcaaagac 1380

ataatccggg ccataaggta a 1401

<210> 6
<211> 1359
<212> DNA
<213> Artificial

<220>
<223> Open reading phase corresponding to the gene of the SAP (IBDV pVP2-
452)

<400> 6
atgacaaacc tgtcagatca aacccagcag attgttccgt tcatacggag ccttctgatg 60

ccaacaaccg gaccggcgtc cattccggac gacaccctgg agaagcacac tctcaggtca 120

gagacctcga cctacaattt gactgtgggg gacacagggt cagggctaat tgtctttttc 180

cctggattcc ctggctcaat tgtgggtgct cactacacac tgcagggcaa tgggaactac 240

aagttcgatc agatgctcct gactgcccag aacctaccgg ccagttacaa ctactgcagg 300

ctagtgagtc ggagtctcac agtgaggtca agcacacttc ctggtggcgt ttatgcacta 360

aacggcacca taaacgccgt gaccttccaa ggaagcctga gtgaactgac agatgttagc 420

tacaatgggt tgatgtctgc aacagccaac atcaacgaca aaattgggaa cgtcctagta 480

ggggaagggg tcaccgtcct cagcttaccc acatcatatg atcttgggta tgtgaggctt 540

ggtgacccca ttcccgcaat agggcttgac ccaaaaatgg tagccacatg tgacagcagt 600

gacaggccca gagtctacac cataactgca gccgatgatt accaattctc atcacagtac 660

caaccaggtg gggtaacaat cacactgttc tcagccaaca ttgatgccat cacaagcctc 720

agcgttgggg gagagctcgt gtttcgaaca agcgtccacg gccttgtact gggcgccacc 780

atctacctca taggctttga tgggacaacg gtaatcacca gggctgtggc cgcaaacaat 840

gggctgacga ccggcaccga caaccttatg ccattcaatc ttgtgattcc aacaaacgag 900

ataacccagc caatcacatc catcaaactg gagatagtga cctccaaaag tggtggtcag 960

gcaggggatc agatgtcatg gtcggcaaga gggagcctag cagtgacgat ccatggtggc 1020

aactatccag gggccctccg tcccgtcacg ctagtggcct acgaaagagt ggcaacagga 1080

tccgtcgtta cggtcgctgg ggtgagcaac ttcgagctga tcccaaatcc tgaactagca 1140

aagaacctgg ttacagaata cggccgattt gacccaggag ccatgaacta cacaaaattg 1200

atactgagtg agagggaccg tcttggcatc aagaccgtct ggccaacaag ggagtacact 1260

gactttcgtg aatacttcat ggaggtggcc gacctcaact ctcccctgaa gattgcagga 1320

gcattcggct tcaaagacat aatccgggcc ataaggtaa 1359

<210> 7
<211> 18
<212> DNA
<213> Artificial

<220>
<223> Primer Bap 1

<400> 7
tcaccgtcct cagcttac 18

<210> 8
<211> 18
<212> DNA
<213> Artificial

<220>
<223> Primer Bap 4

<400> 8
tcaggatttg ggatcagc 18

<210> 9
<211> 59
<212> DNA
<213> Artificial

<220>
<223> Primer Bap 2

<400> 9
cgattttctg agcctcgaag atgtcgttca ggccaccacc acttttggag gtcactatc 59

<210> 10
<211> 58
<212> DNA
<213> Artificial

<220>
<223> Primer Bap 3

<400> 10
catcttcgag gctcagaaaa tcgaatggca cgaagcaggg gatcagatgt catggtcg 58

<210> 11
<211> 6076

<212>  DNA
<213>  Artificial

<220>
<223>  Nucleotide sequence of plasmid pFB_SAP_BAP

<400>  11
cggccgtcgc ggcgcttgcc ggtggtgctg accccggatg aagtggttcg catcctcggt      60

tttctggaag gcgagcatcg tttgttcgcc caggactcta gctatagttc tagtggttgg     120

ctacgtatac tccggaatat taatagatca tggagataat taaaatgata accatctcgc     180

aaataaataa gtattttact gttttcgtaa cagttttgta ataaaaaaac ctataaatat     240

tccggattat tcataccgtc ccaccatcgg gcgcggatct atgacaaacc tgtcagatca     300

aacccagcag attgttccgt tcatacggag ccttctgatg ccaacaaccg gaccggcgtc     360

cattccggac gacaccctgg agaagcacac tctcaggtca gagacctcga cctacaattt     420

gactgtgggg gacacagggt cagggctaat tgtctttttc cctggattcc ctggctcaat     480

tgtgggtgct cactacacac tgcagggcaa tgggaactac aagttcgatc agatgctcct     540

gactgcccag aacctaccgg ccagttacaa ctactgcagg ctagtgagtc ggagtctcac     600

agtgaggtca agcacacttc ctggtggcgt ttatgcacta aacggcacca taaacgccgt     660

gaccttccaa ggaagcctga gtgaactgac agatgttagc tacaatgggt tgatgtctgc     720

aacagccaac atcaacgaca aaattgggaa cgtcctagta ggggaagggg tcaccgtcct     780

cagcttaccc acatcatatg atcttgggta tgtgaggctt ggtgacccca ttcccgcaat     840

agggcttgac ccaaaaatgg tagccacatg tgacagcagt gacaggccca gagtctacac     900

cataactgca gccgatgatt accaattctc atcacagtac caaccaggtg gggtaacaat     960

cacactgttc tcagccaaca ttgatgccat cacaagcctc agcgttgggg gagagctcgt    1020

gtttcgaaca agcgtccacg gccttgtact gggcgccacc atctacctca taggctttga    1080

tgggacaacg gtaatcacca gggctgtggc cgcaaacaat gggctgacga ccggcaccga    1140

caaccttatg ccattcaatc ttgtgattcc aacaaacgag ataacccagc caatcacatc    1200

catcaaactg gagatagtga cctccaaaag tggtggtggc ctgaacgaca tcttcgaggc    1260

tcagaaaatc gaatggcacg aagcagggga tcagatgtca tggtcggcaa gagggagcct    1320

agcagtgacg atccatggtg gcaactatcc aggggccctc cgtcccgtca cgctagtggc    1380

ctacgaaaga gtggcaacag gatccgtcgt tacggtcgct ggggtgagca acttcgagct    1440

gatcccaaat cctgaactag caaagaacct ggttacagaa tacggccgat ttgacccagg    1500

agccatgaac tacacaaaat tgatactgag tgagagggac cgtcttggca tcaagaccgt    1560

ctggccaaca agggagtaca ctgactttcg tgaatacttc atggaggtgg ccgacctcaa    1620

ctctcccctg aagattgcag gagcattcgg cttcaaagac ataatccggg ccataaggta 1680

agcttgtcga gaagtactag aggatcataa tcagccatac cacatttgta gaggttttac 1740

ttgctttaaa aaacctccca cacctccccc tgaacctgaa acataaaatg aatgcaattg 1800

ttgttgttaa cttgtttatt gcagcttata atggttacaa ataaagcaat agcatcacaa 1860

atttcacaaa taaagcattt ttttcactgc attctagttg tggtttgtcc aaactcatca 1920

atgtatctta tcatgtctgg atctgatcac tgcttgagcc taggagatcc gaaccagata 1980

agtgaaatct agttccaaac tattttgtca tttttaattt tcgtattagc ttacgacgct 2040

acacccagtt cccatctatt ttgtcactct tccctaaata atccttaaaa actccatttc 2100

cacccctccc agttcccaac tattttgtcc gcccacagcg gggcattttt cttcctgtta 2160

tgtttttaat caaacatcct gccaactcca tgtgacaaac cgtcatcttc ggctactttt 2220

tctctgtcac agaatgaaaa tttttctgtc atctcttcgt tattaatgtt tgtaattgac 2280

tgaatatcaa cgcttatttg cagcctgaat ggcgaatggg acgcgccctg tagcggcgca 2340

ttaagcgcgg cgggtgtggt ggttacgcgc agcgtgaccg ctacacttgc cagcgcccta 2400

gcgcccgctc ctttcgcttt cttcccttcc tttctcgcca cgttcgccgg ctttccccgt 2460

caagctctaa atcgggggct ccctttaggg ttccgattta gtgctttacg gcacctcgac 2520

cccaaaaaac ttgattaggg tgatggttca cgtagtgggc catcgccctg atagacggtt 2580

tttcgccctt tgacgttgga gtccacgttc tttaatagtg gactcttgtt ccaaactgga 2640

acaacactca accctatctc ggtctattct tttgatttat aagggatttt gccgatttcg 2700

gcctattggt taaaaaatga gctgatttaa caaaaattta acgcgaattt taacaaaata 2760

ttaacgttta caatttcagg tggcactttt cggggaaatg tgcgcggaac ccctatttgt 2820

ttatttttct aaatacattc aaatatgtat ccgctcatga gacaataacc ctgataaatg 2880

cttcaataat attgaaaaag gaagagtatg agtattcaac atttccgtgt cgcccttatt 2940

cccttttttg cggcattttg ccttcctgtt tttgctcacc cagaaacgct ggtgaaagta 3000

aaagatgctg aagatcagtt gggtgcacga gtgggttaca tcgaactgga tctcaacagc 3060

ggtaagatcc ttgagagttt tcgccccgaa gaacgttttc caatgatgag cacttttaaa 3120

gttctgctat gtggcgcggt attatcccgt attgacgccg ggcaagagca actcggtcgc 3180

cgcatacact attctcagaa tgacttggtt gagtactcac cagtcacaga aaagcatctt 3240

acggatggca tgacagtaag agaattatgc agtgctgcca taaccatgag tgataacact 3300

gcggccaact tacttctgac aacgatcgga ggaccgaagg agctaaccgc ttttttgcac 3360

aacatggggg atcatgtaac tcgccttgat cgttgggaac cggagctgaa tgaagccata 3420

ccaaacgacg agcgtgacac cacgatgcct gtagcaatgg caacaacgtt gcgcaaacta 3480

ttaactggcg aactacttac tctagcttcc cggcaacaat taatagactg gatggaggcg 3540

gataaagttg caggaccact tctgcgctcg gcccttccgg ctggctggtt tattgctgat 3600

aaatctggag ccggtgagcg tgggtctcgc ggtatcattg cagcactggg gccagatggt 3660

aagccctccc gtatcgtagt tatctacacg acggggagtc aggcaactat ggatgaacga 3720

aatagacaga tcgctgagat aggtgcctca ctgattaagc attggtaact gtcagaccaa 3780

gtttactcat atatacttta gattgattta aaacttcatt tttaatttaa aaggatctag 3840

gtgaagatcc tttttgataa tctcatgacc aaaatccctt aacgtgagtt ttcgttccac 3900

tgagcgtcag accccgtaga aaagatcaaa ggatcttctt gagatccttt ttttctgcgc 3960

gtaatctgct gcttgcaaac aaaaaaacca ccgctaccag cggtggtttg tttgccggat 4020

caagagctac caactctttt tccgaaggta actggcttca gcagagcgca gataccaaat 4080

actgtccttc tagtgtagcc gtagttaggc caccacttca agaactctgt agcaccgcct 4140

acatacctcg ctctgctaat cctgttacca gtggctgctg ccagtggcga taagtcgtgt 4200

cttaccgggt tggactcaag acgatagtta ccggataagg cgcagcggtc gggctgaacg 4260

gggggttcgt gcacacagcc cagcttggag cgaacgacct acaccgaact gagataccta 4320

cagcgtgagc attgagaaag cgccacgctt cccgaaggga gaaaggcgga caggtatccg 4380

gtaagcggca gggtcggaac aggagagcgc acgagggagc ttccaggggg aaacgcctgg 4440

tatctttata gtcctgtcgg gtttcgccac ctctgacttg agcgtcgatt tttgtgatgc 4500

tcgtcagggg ggcggagcct atggaaaaac gccagcaacg cggccttttt acggttcctg 4560

gccttttgct ggccttttgc tcacatgttc tttcctgcgt tatcccctga ttctgtggat 4620

aaccgtatta ccgcctttga gtgagctgat accgctcgcc gcagccgaac gaccgagcgc 4680

agcgagtcag tgagcgagga agcggaagag cgcctgatgc ggtattttct ccttacgcat 4740

ctgtgcggta tttcacaccg cagaccagcc gcgtaacctg gcaaaatcgg ttacggttga 4800

gtaataaatg gatgccctgc gtaagcgggt gtgggcggac aataaagtct taaactgaac 4860

aaaatagatc taaactatga caataaagtc ttaaactaga cagaatagtt gtaaactgaa 4920

atcagtccag ttatgctgtg aaaaagcata ctggactttt gttatggcta aagcaaactc 4980

ttcattttct gaagtgcaaa ttgcccgtcg tattaaagag gggcgtggcc aagggcatgg 5040

taaagactat attcgcggcg ttgtgacaat ttaccgaaca actccgcggc cgggaagccg 5100

atctcggctt gaacgaattg ttaggtggcg gtacttgggt cgatatcaaa gtgcatcact 5160

tcttcccgta tgcccaactt tgtatagaga gccactgcgg gatcgtcacc gtaatctgct 5220

tgcacgtaga tcacataagc accaagcgcg ttggcctcat gcttgaggag attgatgagc 5280

gcggtggcaa tgccctgcct ccggtgctcg ccggagactg cgagatcata gatatagatc 5340

tcactacgcg gctgctcaaa cctgggcaga acgtaagccg cgagagcgcc aacaaccgct 5400

tcttggtcga aggcagcaag cgcgatgaat gtcttactac ggagcaagtt cccgaggtaa 5460

tcggagtccg gctgatgttg ggagtaggtg gctacgtctc cgaactcacg accgaaaaga 5520

tcaagagcag cccgcatgga tttgacttgg tcagggccga gcctacatgt gcgaatgatg 5580

cccatacttg agccacctaa ctttgtttta gggcgactgc cctgctgcgt aacatcgttg 5640

ctgctgcgta acatcgttgc tgctccataa catcaaacat cgacccacgg cgtaacgcgc 5700

ttgctgcttg gatgcccgag gcatagactg tacaaaaaaa cagtcataac aagccatgaa 5760

aaccgccact gcgccgttac caccgctgcg ttcggtcaag gttctggacc agttgcgtga 5820

gcgcatacgc tacttgcatt acagtttacg aaccgaacag gcttatgtca actgggttcg 5880

tgccttcatc cgtttccacg gtgtgcgtca cccggcaacc ttgggcagca gcgaagtcga 5940

ggcatttctg tcctggctgg cgaacgagcg caaggtttcg gtctccacgc atcgtcaggc 6000

attggcggcc ttgctgttct tctacggcaa ggtgctgtgc acggatctgc cctggcttca 6060

ggagatcgga agacct 6076

<210> 12
<211> 966
<212> DNA
<213> Escherichia coli

<220>
<221> gene BirA

<400> 12
atgaaggata acaccgtgcc actgaaattg attgccctgt tagcgaacgg tgaatttcac 60

tctggcgagc agttgggtga aacgctggga atgagccggg cggctattaa taaacacatt 120

cagacactgc gtgactgggg cgttgatgtc tttaccgttc cgggtaaagg atacagcctg 180

cctgagccta tccagttact taatgctaaa cagatattgg gtcagctgga tggcggtagt 240

gtagccgtgc tgccagtgat tgactccacg aatcagtacc ttcttgatcg tatcggagag 300

cttaaatcgg gcgatgcttg cattgcagaa taccagcagg ctggccgtgg tcgccggggt 360

cggaaatggt tttcgccttt tggcgcaaac ttatatttgt cgatgttctg gcgtctggaa 420

caaggcccgg cggcggcgat tggtttaagt ctggttatcg gtatcgtgat ggcggaagta 480

ttacgcaagc tgggtgcaga taaagttcgt gttaaatggc ctaatgacct ctatctgcag 540

gatcgcaagc tggcaggcat tctggtggag ctgactggca aaactggcga tgcggcgcaa 600

atagtcattg gagccgggat caacatggca atgcgccgtg ttgaagagag tgtcgttaat 660

caggggtgga tcacgctgca ggaagcgggg atcaatctcg atcgtaatac gttggcggcc 720

atgctaatac gtgaattacg tgctgcgttg gaactcttcg aacaagaagg attggcacct 780

tatctgtcgc gctgggaaaa gctggataat tttattaatc gcccagtgaa acttatcatt 840

ggtgataaag aaatatttgg catttcacgc ggaatagaca aacagggggc tttattactt 900

gagcaggatg gaataataaa accctggatg ggcggtgaaa tatccctgcg tagtgcagaa 960

aaataa 966

<210> 13
<211> 30
<212> DNA
<213> Artificial

<220>
<223> Primer BirA 1

<400> 13
cgcgctcgag atgaaggata acaccgtgcc 30

<210> 14
<211> 31
<212> DNA
<213> Artificial

<220>
<223> Primer BirA 2

<400> 14
gcgcgcatgc ttatttttct gcactacgca g 31

<210> 15
<211> 6179
<212> DNA
<213> Artificial

<220>
<223> Nucleotide sequence corresponding to plasmid
pFastBacDual_Bir A

<400> 15
tcgagatgaa ggataacacc gtgccactga aattgattgc cctgttagcg aacggtgaat 60

ttcactctgg cgagcagttg ggtgaaacgc tgggaatgag ccgggcggct attaataaac 120

acattcagac actgcgtgac tggggcgttg atgtctttac cgttccgggt aaaggataca 180

gcctgcctga gcctatccag ttacttaatg ctaaacagat attgggtcag ctggatggcg 240

gtagtgtagc cgtgctgcca gtgattgact ccacgaatca gtaccttctt gatcgtatcg 300

gagagcttaa atcgggcgat gcttgcattg cagaatacca gcaggctggc cgtggtcgcc 360
ggggtcggaa atggttttcg ccttttggcg caaacttata tttgtcgatg ttctggcgtc 420
tggaacaagg cccggcggcg gcgattggtt taagtctggt tatcggtatc gtgatggcgg 480
aagtattacg caagctgggt gcagataaag ttcgtgttaa atggcctaat gacctctatc 540
tgcaggatcg caagctggca ggcattctgg tggagctgac tggcaaaact ggcgatgcgg 600
cgcaaatagt cattggagcc gggatcaaca tggcaatgcg ccgtgttgaa gagagtgtcg 660
ttaatcaggg gtggatcacg ctgcaggaag cggggatcaa tctcgatcgt aatacgttgg 720
cggccatgct aatacgtgaa ttacgtgctg cgttggaact cttcgaacaa gaaggattgg 780
caccttatct gtcgcgctgg gaaaagctgg ataattttat taatcgccca gtgaaactta 840
tcattggtga taaagaaata tttggcattt cacgcggaat agacaaacag gggggcttat 900
tacttgagca ggatggaata ataaaaccct ggatgggcgg tgaaatatcc ctgcgtagtg 960
cagaaaaata agcatgcggt accgggagat gggggaggct aactgaaaca cggaaggaga 1020
caataccgga aggaacccgc gctatgacgg caataaaaag acagaataaa acgcacgggt 1080
gttgggtcgt ttgttcataa acgcggggtt cggtcccagg gctggcactc tgtcgatacc 1140
ccaccgagac cccattggga ccaatacgcc cgcgtttctt ccttttcccc accccaaccc 1200
ccaagttcgg gtgaaggccc agggctcgca gccaacgtcg gggcggcaag ccctgccata 1260
gccactacgg gtacgtaggc caaccactag aactatagct agagtcctgg gcgaacaaac 1320
gatgctcgcc ttccagaaaa ccgaggatgc gaaccacttc atccggggtc agcaccaccg 1380
gcaagcgccg cgacggccga ggtctaccga tctcctgaag ccagggcaga tccgtgcaca 1440
gcaccttgcc gtagaagaac agcaaggccg ccaatgcctg acgatgcgtg gagaccgaaa 1500
ccttgcgctc gttcgccagc caggacagaa atgcctcgac ttcgctgctg cccaaggttg 1560
ccgggtgacg cacaccgtgg aaacggatga aggcacgaac ccagttgaca taagcctgtt 1620
cggttcgtaa actgtaatgc aagtagcgta tgcgctcacg caactggtcc agaaccttga 1680
ccgaacgcag cggtggtaac ggcgcagtgg cggttttcat ggcttgttat gactgttttt 1740
ttgtacagtc tatgcctcgg gcatccaagc agcaagcgcg ttacgccgtg ggtcgatgtt 1800
tgatgttatg gagcagcaac gatgttacgc agcagcaacg atgttacgca gcagggcagt 1860
cgccctaaaa caaagttagg tggctcaagt atgggcatca ttcgcacatg taggctcggc 1920
cctgaccaag tcaaatccat gcgggctgct cttgatcttt tcggtcgtga gttcggagac 1980
gtagccacct actcccaaca tcagccggac tccgattacc tcgggaactt gctccgtagt 2040
aagacattca tcgcgcttgc tgccttcgac caagaagcgg ttgttggcgc tctcgcggct 2100

tacgttctgc ccaggtttga gcagccgcgt agtgagatct atatctatga tctcgcagtc 2160
tccggcgagc accggaggca gggcattgcc accgcgctca tcaatctcct caagcatgag 2220
gccaacgcgc ttggtgctta tgtgatctac gtgcaagcag attacggtga cgatcccgca 2280
gtggctctct atacaaagtt gggcatacgg gaagaagtga tgcactttga tatcgaccca 2340
agtaccgcca cctaacaatt cgttcaagcc gagatcggct tcccggccgc ggagttgttc 2400
ggtaaattgt cacaacgccg cgaatatagt ctttaccatg cccttggcca cgcccctctt 2460
taatacgacg ggcaatttgc acttcagaaa atgaagagtt tgctttagcc ataacaaaag 2520
tccagtatgc tttttcacag cataactgga ctgatttcag tttacaacta ttctgtctag 2580
tttaagactt tattgtcata gtttagatct attttgttca gtttaagact ttattgtccg 2640
cccacacccg cttacgcagg gcatccattt attactcaac cgtaaccgat tttgccaggt 2700
tacgcggctg gtctgcggtg tgaaataccg cacagatgcg taaggagaaa ataccgcatc 2760
aggcgctctt ccgcttcctc gctcactgac tcgctgcgct cggtcgttcg gctgcggcga 2820
gcggtatcag ctcactcaaa ggcggtaata cggttatcca cagaatcagg ggataacgca 2880
ggaaagaaca tgtgagcaaa aggccagcaa aaggccagga accgtaaaaa ggccgcgttg 2940
ctggcgtttt tccataggct ccgcccccct gacgagcatc acaaaaatcg acgctcaagt 3000
cagaggtggc gaaacccgac aggactataa agataccagg cgtttccccc tggaagctcc 3060
ctcgtgcgct ctcctgttcc gaccctgccg cttaccggat acctgtccgc ctttctccct 3120
tcgggaagcg tggcgctttc tcaatgctca cgctgtaggt atctcagttc ggtgtaggtc 3180
gttcgctcca agctgggctg tgtgcacgaa ccccccgttc agcccgaccg ctgcgcctta 3240
tccggtaact atcgtcttga gtccaacccg gtaagacacg acttatcgcc actggcagca 3300
gccactggta acaggattag cagagcgagg tatgtaggcg gtgctacaga gttcttgaag 3360
tggtggccta actacggcta cactagaagg acagtatttg gtatctgcgc tctgctgaag 3420
ccagttacct tcggaaaaag agttggtagc tcttgatccg gcaaacaaac caccgctggt 3480
agcggtggtt tttttgtttg caagcagcag attacgcgca gaaaaaaagg atctcaagaa 3540
gatcctttga tcttttctac ggggtctgac gctcagtgga acgaaaactc acgttaaggg 3600
attttggtca tgagattatc aaaaaggatc ttcacctaga tccttttaaa ttaaaaatga 3660
agttttaaat caatctaaag tatatatgag taaacttggt ctgacagtta ccaatgctta 3720
atcagtgagg cacctatctc agcgatctgt ctatttcgtt catccatagt tgcctgactc 3780
cccgtcgtgt agataactac gatacgggag ggcttaccat ctggccccag tgctgcaatg 3840
ataccgcgag acccacgctc accggctcca gatttatcag caataaacca gccagccgga 3900

agggccgagc gcagaagtgg tcctgcaact ttatccgcct ccatccagtc tattaattgt 3960
tgccgggaag ctagagtaag tagttcgcca gttaatagtt tgcgcaacgt tgttgccatt 4020
gctacaggca tcgtggtgtc acgctcgtcg tttggtatgg cttcattcag ctccggttcc 4080
caacgatcaa ggcgagttac atgatccccc atgttgtgca aaaaagcggt tagctccttc 4140
ggtcctccga tcgttgtcag aagtaagttg gccgcagtgt tatcactcat ggttatggca 4200
gcactgcata attctcttac tgtcatgcca tccgtaagat gcttttctgt gactggtgag 4260
tactcaacca agtcattctg agaatagtgt atgcggcgac cgagttgctc ttgcccggcg 4320
tcaatacggg ataataccgc gccacatagc agaactttaa aagtgctcat cattggaaaa 4380
cgttcttcgg ggcgaaaact ctcaaggatc ttaccgctgt tgagatccag ttcgatgtaa 4440
cccactcgtg cacccaactg atcttcagca tcttttactt tcaccagcgt ttctgggtga 4500
gcaaaaacag gaaggcaaaa tgccgcaaaa aagggaataa gggcgacacg gaaatgttga 4560
atactcatac tcttcctttt tcaatattat tgaagcattt atcagggtta ttgtctcatg 4620
agcggataca tatttgaatg tatttagaaa aataaacaaa taggggttcc gcgcacattt 4680
ccccgaaaag tgccacctga aattgtaaac gttaatattt tgttaaaatt cgcgttaaat 4740
ttttgttaaa tcagctcatt ttttaaccaa taggccgaaa tcggcaaaat cccttataaa 4800
tcaaaagaat agaccgagat agggttgagt gttgttccag tttggaacaa gagtccacta 4860
ttaaagaacg tggactccaa cgtcaaaggg cgaaaaaccg tctatcaggg cgatggccca 4920
ctacgtgaac catcacccta atcaagtttt ttggggtcga ggtgccgtaa agcactaaat 4980
cggaacccta aagggagccc ccgatttaga gcttgacggg gaaagccggc gaacgtggcg 5040
agaaaggaag ggaagaaagc gaaaggagcg ggcgctaggg cgctggcaag tgtagcggtc 5100
acgctgcgcg taaccaccac acccgccgcg cttaatgcgc cgctacaggg cgcgtcccat 5160
tcgccattca ggctgcaaat aagcgttgat attcagtcaa ttacaaacat taataacgaa 5220
gagatgacag aaaaattttc attctgtgac agagaaaaag tagccgaaga tgacggtttg 5280
tcacatggag ttggcaggat gtttgattaa aaacataaca ggaagaaaaa tgccccgctg 5340
tgggcggaca aaatagttgg gaactgggag gggtggaaat ggagttttta aggattattt 5400
agggaagagt gacaaaatag atgggaactg ggtgtagcgt cgtaagctaa tacgaaaatt 5460
aaaaatgaca aaatagtttg gaactagatt tcacttatct ggttcggatc tcctaggctc 5520
aagcagtgat cagatccaga catgataaga tacattgatg agtttggaca aaccacaact 5580
agaatgcagt gaaaaaaatg ctttatttgt gaaatttgtg atgctattgc tttatttgta 5640
accattataa gctgcaataa acaagttaac aacaacaatt gcattcattt tatgtttcag 5700

gttcaggggg aggtgtggga ggttttttaa agcaagtaaa acctctacaa atgtggtatg 5760

gctgattatg atcctctagt acttctcgac aagcttgtcg agactgcagg ctctagattc 5820

gaaagcggcc gcgactagtg agctcgtcga cgtaggcctt tgaattccgc gcgcttcgga 5880

ccgggatccg cgcccgatgg tgggacggta tgaataatcc ggaatattta taggtttttt 5940

tattacaaaa ctgttacgaa aacagtaaaa tacttattta tttgcgagat ggttatcatt 6000

ttaattatct ccatgatcta ttaatattcc ggagtatacg gacctttaat tcaacccaac 6060

acaatatatt atagttaaat aagaattatt atcaaatcat ttgtatatta attaaaatac 6120

tatactgtaa attacatttt atttacaatc actcgacgaa gacttgatca cccgggatc 6179

<210> 16
<211> 7491
<212> DNA
<213> Artificial

<220>
<223> Nucleotide sequence of plasmid pFBD_BirA_SAP_BAP

<400> 16
gatctatgac aaacctgtca gatcaaaccc agcagattgt tccgttcata cggagccttc 60

tgatgccaac aaccggaccg gcgtccattc cggacgacac cctggagaag cacactctca 120

ggtcagagac ctcgacctac aatttgactg tgggggacac agggtcaggg ctaattgtct 180

ttttccctgg attccctggc tcaattgtgg gtgctcacta cacactgcag ggcaatggga 240

actacaagtt cgatcagatg ctcctgactg cccagaacct accggccagt tacaactact 300

gcaggctagt gagtcggagt ctcacagtga ggtcaagcac acttcctggt ggcgtttatg 360

cactaaacgg caccataaac gccgtgacct tccaaggaag cctgagtgaa ctgacagatg 420

ttagctacaa tgggttgatg tctgcaacag ccaacatcaa cgacaaaatt gggaacgtcc 480

tagtagggga aggggtcacc gtcctcagct tacccacatc atatgatctt gggtatgtga 540

ggcttggtga ccccattccc gcaatagggc ttgacccaaa aatggtagcc acatgtgaca 600

gcagtgacag gcccagagtc tacaccataa ctgcagccga tgattaccaa ttctcatcac 660

agtaccaacc aggtggggta acaatcacac tgttctcagc caacattgat gccatcacaa 720

gcctcagcgt tgggggagag ctcgtgtttc gaacaagcgt ccacggcctt gtactgggcg 780

ccaccatcta cctcataggc tttgatggga caacggtaat caccagggct gtggccgcaa 840

acaatgggct gacgaccggc accgacaacc ttatgccatt caatcttgtg attccaacaa 900

acgagataac ccagccaatc acatccatca aactggagat agtgacctcc aaaagtggtg 960

gtggcctgaa cgacatcttc gaggctcaga aaatcgaatg gcacgaagca ggggatcaga 1020

tgtcatggtc ggcaagaggg agcctagcag tgacgatcca tggtggcaac tatccagggg 1080

ccctccgtcc cgtcacgcta gtggcctacg aaagagtggc aacaggatcc gtcgttacgg 1140

tcgctggggt gagcaacttc gagctgatcc caaatcctga actagcaaag aacctggtta 1200

cagaatacgg ccgatttgac ccaggagcca tgaactacac aaaattgata ctgagtgaga 1260

gggaccgtct tggcatcaag accgtctggc caacaaggga gtacactgac tttcgtgaat 1320

acttcatgga ggtggccgac ctcaactctc ccctgaagat tgcaggagca ttcggcttca 1380

aagacataat ccgggccata aggtaagctt gtcgagaagt actagaggat cataatcagc 1440

cataccacat ttgtagaggt tttacttgct ttaaaaaacc tcccacacct ccccctgaac 1500

ctgaaacata aaatgaatgc aattgttgtt gttaacttgt ttattgcagc ttataatggt 1560

tacaaataaa gcaatagcat cacaaatttc acaaataaag catttttttc actgcattct 1620

agttgtggtt tgtccaaact catcaatgta tcttatcatg tctggatctg atcactgctt 1680

gagcctagga gatccgaacc agataagtga aatctagttc caaactattt tgtcattttt 1740

aattttcgta ttagcttacg acgctacacc cagttcccat ctattttgtc actcttccct 1800

aaataatcct taaaaactcc atttccaccc ctcccagttc ccaactattt tgtccgccca 1860

cagcggggca tttttcttcc tgttatgttt ttaatcaaac atcctgccaa ctccatgtga 1920

caaaccgtca tcttcggcta ctttttctct gtcacagaat gaaaattttt ctgtcatctc 1980

ttcgttatta atgtttgtaa ttgactgaat atcaacgctt atttgcagcc tgaatggcga 2040

atgggacgcg ccctgtagcg gcgcattaag cgcggcgggt gtggtggtta cgcgcagcgt 2100

gaccgctaca cttgccagcg ccctagcgcc cgctcctttc gctttcttcc cttcctttct 2160

cgccacgttc gccggctttc cccgtcaagc tctaaatcgg gggctccctt tagggttccg 2220

atttagtgct ttacggcacc tcgaccccaa aaaacttgat tagggtgatg gttcacgtag 2280

tgggccatcg ccctgataga cggtttttcg ccctttgacg ttggagtcca cgttctttaa 2340

tagtggactc ttgttccaaa ctggaacaac actcaaccct atctcggtct attcttttga 2400

tttataaggg attttgccga tttcggccta ttggttaaaa aatgagctga tttaacaaaa 2460

atttaacgcg aattttaaca aaatattaac gtttacaatt tcaggtggca cttttcgggg 2520

aaatgtgcgc ggaaccccta tttgtttatt tttctaaata cattcaaata tgtatccgct 2580

catgagacaa taaccctgat aaatgcttca ataatattga aaaaggaaga gtatgagtat 2640

tcaacatttc cgtgtcgccc ttattccctt ttttgcggca ttttgccttc ctgtttttgc 2700

tcacccagaa acgctggtga aagtaaaaga tgctgaagat cagttgggtg cacgagtggg 2760

ttacatcgaa ctggatctca acagcggtaa gatccttgag agttttcgcc ccgaagaacg 2820

ttttccaatg atgagcactt ttaaagttct gctatgtggc gcggtattat cccgtattga 2880
cgccgggcaa gagcaactcg gtcgccgcat acactattct cagaatgact tggttgagta 2940
ctcaccagtc acagaaaagc atcttacgga tggcatgaca gtaagagaat tatgcagtgc 3000
tgccataacc atgagtgata acactgcggc caacttactt ctgacaacga tcggaggacc 3060
gaaggagcta accgcttttt tgcacaacat gggggatcat gtaactcgcc ttgatcgttg 3120
ggaaccggag ctgaatgaag ccataccaaa cgacgagcgt gacaccacga tgcctgtagc 3180
aatggcaaca acgttgcgca aactattaac tggcgaacta cttactctag cttcccggca 3240
acaattaata gactggatgg aggcggataa agttgcagga ccacttctgc gctcggccct 3300
tccggctggc tggtttattg ctgataaatc tggagccggt gagcgtgggt ctcgcggtat 3360
cattgcagca ctggggccag atggtaagcc ctcccgtatc gtagttatct acacgacggg 3420
gagtcaggca actatggatg aacgaaatag acagatcgct gagataggtg cctcactgat 3480
taagcattgg taactgtcag accaagttta ctcatatata ctttagattg atttaaaact 3540
tcatttttaa tttaaaagga tctaggtgaa gatccttttt gataatctca tgaccaaaat 3600
cccttaacgt gagttttcgt tccactgagc gtcagacccc gtagaaaaga tcaaaggatc 3660
ttcttgagat cctttttttc tgcgcgtaat ctgctgcttg caaacaaaaa aaccaccgct 3720
accagcggtg gtttgtttgc cggatcaaga gctaccaact ctttttccga aggtaactgg 3780
cttcagcaga gcgcagatac caaatactgt ccttctagtg tagccgtagt taggccacca 3840
cttcaagaac tctgtagcac cgcctacata cctcgctctg ctaatcctgt taccagtggc 3900
tgctgccagt ggcgataagt cgtgtcttac cgggttggac tcaagacgat agttaccgga 3960
taaggcgcag cggtcgggct gaacgggggg ttcgtgcaca cagcccagct tggagcgaac 4020
gacctacacc gaactgagat acctacagcg tgagcattga gaaagcgcca cgcttcccga 4080
agggagaaag gcggacaggt atccggtaag cggcagggtc ggaacaggag agcgcacgag 4140
ggagcttcca gggggaaacg cctggtatct ttatagtcct gtcgggtttc gccacctctg 4200
acttgagcgt cgatttttgt gatgctcgtc aggggggcgg agcctatgga aaaacgccag 4260
caacgcggcc tttttacggt tcctggcctt ttgctggcct tttgctcaca tgttctttcc 4320
tgcgttatcc cctgattctg tggataaccg tattaccgcc tttgagtgag ctgataccgc 4380
tcgccgcagc cgaacgaccg agcgcagcga gtcagtgagc gaggaagcgg aagagcgcct 4440
gatgcggtat tttctcctta cgcatctgtg cggtatttca caccgcagac cagccgcgta 4500
acctggcaaa atcggttacg gttgagtaat aaatggatgc cctgcgtaag cgggtgtggg 4560
cggacaataa agtcttaaac tgaacaaaat agatctaaac tatgacaata aagtcttaaa 4620

ctagacagaa tagttgtaaa ctgaaatcag tccagttatg ctgtgaaaaa gcatactgga 4680
cttttgttat ggctaaagca aactcttcat tttctgaagt gcaaattgcc cgtcgtatta 4740
aagaggggcg tggccaaggg catggtaaag actatattcg cggcgttgtg acaatttacc 4800
gaacaactcc gcggccggga agccgatctc ggcttgaacg aattgttagg tggcggtact 4860
tgggtcgata tcaaagtgca tcacttcttc ccgtatgccc aactttgtat agagagccac 4920
tgcgggatcg tcaccgtaat ctgcttgcac gtagatcaca taagcaccaa gcgcgttggc 4980
ctcatgcttg aggagattga tgagcgcggt ggcaatgccc tgcctccggt gctcgccgga 5040
gactgcgaga tcatagatat agatctcact acgcggctgc tcaaacctgg gcagaacgta 5100
agccgcgaga gcgccaacaa ccgcttcttg gtcgaaggca gcaagcgcga tgaatgtctt 5160
actacggagc aagttcccga ggtaatcgga gtccggctga tgttgggagt aggtggctac 5220
gtctccgaac tcacgaccga aaagatcaag agcagcccgc atggatttga cttggtcagg 5280
gccgagccta catgtgcgaa tgatgcccat acttgagcca cctaactttg ttttagggcg 5340
actgccctgc tgcgtaacat cgttgctgct gcgtaacatc gttgctgctc cataacatca 5400
aacatcgacc cacggcgtaa cgcgcttgct gcttggatgc ccgaggcata gactgtacaa 5460
aaaaacagtc ataacaagcc atgaaaaccg ccactgcgcc gttaccaccg ctgcgttcgg 5520
tcaaggttct ggaccagttg cgtgagcgca tacgctactt gcattacagt ttacgaaccg 5580
aacaggctta tgtcaactgg gttcgtgcct tcatccgttt ccacggtgtg cgtcacccgg 5640
caaccttggg cagcagcgaa gtcgaggcat ttctgtcctg gctggcgaac gagcgcaagg 5700
tttcggtctc cacgcatcgt caggcattgg cggccttgct gttcttctac ggcaaggtgc 5760
tgtgcacgga tctgccctgg cttcaggaga tcggtagacc tcggccgtcg cggcgcttgc 5820
cggtggtgct gaccccggat gaagtggttc gcatcctcgg ttttctggaa ggcgagcatc 5880
gtttgttcgc ccaggactct agctatagtt ctagtggttg gcctacgtac ccgtagtggc 5940
tatggcaggg cttgccgccc cgacgttggc tgcgagccct gggccttcac ccgaacttgg 6000
gggttggggt ggggaaaagg aagaaacgcg ggcgtattgg tcccaatggg gtctcggtgg 6060
ggtatcgaca gagtgccagc cctgggaccg aaccccgcgt ttatgaacaa acgacccaac 6120
acccgtgcgt tttattctgt ctttttattg ccgtcatagc gcgggttcct tccggtattg 6180
tctccttccg tgtttcagtt agcctccccc atctcccggt accgcatgct tatttttctg 6240
cactacgcag ggatatttca ccgcccatcc agggttttat tattccatcc tgctcaagta 6300
ataaagcccc ctgtttgtct attccgcgtg aaatgccaaa tatttcttta tcaccaatga 6360
taagtttcac tgggcgatta ataaaattat ccagcttttc ccagcgcgac agataaggtg 6420

ccaatccttc ttgttcgaag agttccaacg cagcacgtaa ttcacgtatt agcatggccg 6480

ccaacgtatt acgatcgaga ttgatccccg cttcctgcag cgtgatccac ccctgattaa 6540

cgacactctc ttcaacacgg cgcattgcca tgttgatccc ggctccaatg actatttgcg 6600

ccgcatcgcc agttttgcca gtcagctcca ccagaatgcc tgccagcttg cgatcctgca 6660

gatagaggtc attaggccat ttaacacgaa ctttatctgc acccagcttg cgtaatactt 6720

ccgccatcac gataccgata accagactta aaccaatcgc cgccgccggg ccttgttcca 6780

gacgccagaa catcgacaaa tataagtttg cgccaaaagg cgaaaaccat ttccgacccc 6840

ggcgaccacg gccagcctgc tggtattctg caatgcaagc atcgcccgat ttaagctctc 6900

cgatacgatc aagaaggtac tgattcgtgg agtcaatcac tggcagcacg gctacactac 6960

cgccatccag ctgacccaat atctgtttag cattaagtaa ctggataggc tcaggcaggc 7020

tgtatccttt acccggaacg gtaaagacat caacgcccca gtcacgcagt gtctgaatgt 7080

gtttattaat agccgcccgg ctcattccca gcgtttcacc caactgctcg ccagagtgaa 7140

attcaccgtt cgctaacagg gcaatcaatt tcagtggcac ggtgttatcc ttcatctcga 7200

gatcccgggt gatcaagtct tcgtcgagtg attgtaaata aaatgtaatt tacagtatag 7260

tattttaatt aatatacaaa tgatttgata ataattctta tttaactata atatattgtg 7320

ttgggttgaa ttaaaggtcc gtatactccg gaatattaat agatcatgga gataattaaa 7380

atgataacca tctcgcaaat aaataagtat tttactgttt tcgtaacagt tttgtaataa 7440

aaaaacctat aaatattccg gattattcat accgtcccac catcgggcgc g 7491

<210> 17
<211> 30
<212> DNA
<213> Artificial

<220>
<223> Primer BirA Yeast 1

<400> 17
cgcggaattc atgaaggata acaccgtgcc 30

<210> 18
<211> 30
<212> DNA
<213> Artificial

<220>
<223> Primer BirA Yeast 2

<400> 18
gcgcagatct tatttttctg cactacgcag 30

<210> 19
<211> 8866
<212> DNA
<213> Artificial

<220>
<223> Nucleotide sequence of plasmid pESCURA_BirA_SAP_BAP

<400> 19
cgaattttca aaaattctta cttttttttt ggatggacgc aaagaagttt aataatcata 60

ttacatggca ttaccaccat atacatatcc atatacatat ccatatctaa tcttacttat 120

atgttgtgga aatgtaaaga gccccattat cttagcctaa aaaaaccttc tctttggaac 180

tttcagtaat acgcttaact gctcattgct atattgaagt acggattaga agccgccgag 240

cgggtgacag ccctccgaag gaagactctc ctccgtgcgt cctcgtcttc accggtcgcg 300

ttcctgaaac gcagatgtgc ctcgcgccgc actgctccga acaataaaga ttctacaata 360

ctagctttta tggttatgaa gaggaaaaat tggcagtaac ctggccccac aaaccttcaa 420

atgaacgaat caaattaaca accataggat gataatgcga ttagtttttt agccttattt 480

ctggggtaat taatcagcga agcgatgatt tttgatctat taacagatat ataaatgcaa 540

aaactgcata accactttaa ctaatacttt caacattttc ggtttgtatt acttcttatt 600

caaatgtaat aaaagtatca acaaaaaatt gttaatatac ctctatactt taacgtcaag 660

gagaaaaaac cccggatcta tgacaaacct gtcagatcaa acccagcaga ttgttccgtt 720

catacggagc cttctgatgc caacaaccgg accggcgtcc attccggacg acaccctgga 780

gaagcacact ctcaggtcag agacctcgac ctacaatttg actgtggggg acacagggtc 840

agggctaatt gtctttttcc ctggattccc tggctcaatt gtgggtgctc actacacact 900

gcagggcaat gggaactaca agttcgatca gatgctcctg actgcccaga acctaccggc 960

cagttacaac tactgcaggc tagtgagtcg gagtctcaca gtgaggtcaa gcacacttcc 1020

tggtggcgtt tatgcactaa acggcaccat aaacgccgtg accttccaag gaagcctgag 1080

tgaactgaca gatgttagct acaatgggtt gatgtctgca acagccaaca tcaacgacaa 1140

aattgggaac gtcctagtag gggaaggggt caccgtcctc agcttaccca catcatatga 1200

tcttgggtat gtgaggcttg gtgaccccat tcccgcaata gggcttgacc caaaaatggt 1260

agccacatgt gacagcagtg acaggcccag agtctacacc ataactgcag ccgatgatta 1320

ccaattctca tcacagtacc aaccaggtgg ggtaacaatc acactgttct cagccaacat 1380

tgatgccatc acaagcctca gcgttggggg agagctcgtg tttcgaacaa gcgtccacgg 1440

ccttgtactg ggcgccacca tctacctcat aggctttgat gggacaacgg taatcaccag 1500

ggctgtggcc gcaaacaatg ggctgacgac cggcaccgac aaccttatgc cattcaatct 1560
tgtgattcca acaaacgaga taacccagcc aatcacatcc atcaaactgg agatagtgac 1620
ctccaaaagt ggtggtggcc tgaacgacat cttcgaggct cagaaaatcg aatggcacga 1680
agcaggggat cagatgtcat ggtcggcaag agggagccta gcagtgacga tccatggtgg 1740
caactatcca ggggccctcc gtcccgtcac gctagtggcc tacgaaagag tggcaacagg 1800
atccgtcgtt acggtcgctg gggtgagcaa cttcgagctg atcccaaatc ctgaactagc 1860
aaagaacctg gttacagaat acggccgatt tgacccagga gccatgaact acacaaaatt 1920
gatactgagt gagagggacc gtcttggcat caagaccgtc tggccaacaa gggagtacac 1980
tgactttcgt gaatacttca tggaggtggc cgacctcaac tctcccctga agattgcagg 2040
agcattcggc ttcaaagaca taatccgggc cataaggtaa gcttggtacc gcggctagct 2100
aagatccgct ctaaccgaaa aggaaggagt tagacaacct gaagtctagg tccctattta 2160
tttttttata gttatgttag tattaagaac gttatttata tttcaaattt ttcttttttt 2220
tctgtacaga cgcgtgtacg catgtaacat tatactgaaa accttgcttg agaaggtttt 2280
gggacgctcg aagatccagc tggcgtaata gcgaagaggc ccgcaccgat cgcccttccc 2340
aacagttgcg cagcctgaat ggcgaatgga cgcgccctgt agcggcgcat taagcgcggc 2400
gggtgtggtg gttacgcgca gcgtgaccgc tacacttgcc agcgccctag cgcccgctcc 2460
tttcgctttc ttcccttcct ttctcgccac gttcgccggc tttccccgtc aagctctaaa 2520
tcgggggctc cctttagggt tccgatttag tgctttacgg cacctcgacc ccaaaaaact 2580
tgattagggt gatggttcac gtagtgggcc atcgccctga tagacggttt ttcgcccttt 2640
gacgttggag tccacgttct ttaatagtgg actcttgttc caaactggaa caacactcaa 2700
ccctatctcg gtctattctt ttgatttata agggattttg ccgatttcgg cctattggtt 2760
aaaaaatgag ctgatttaac aaaaatttaa cgcgaatttt aacaaaatat taacgtttac 2820
aatttcctga tgcggtattt tctccttacg catctgtgcg gtatttcaca ccgcataggg 2880
taataactga tataattaaa ttgaagctct aatttgtgag tttagtatac atgcatttac 2940
ttataataca gttttttagt tttgctggcc gcatcttctc aaatatgctt cccagcctgc 3000
ttttctgtaa cgttcaccct ctaccttagc atcccttccc tttgcaaata gtcctcttcc 3060
aacaataata atgtcagatc ctgtagagac cacatcatcc acggttctat actgttgacc 3120
caatgcgtct cccttgtcat ctaaacccac accgggtgtc ataatcaacc aatcgtaacc 3180
ttcatctctt ccacccatgt ctctttgagc aataaagccg ataacaaaat ctttgtcgct 3240
cttcgcaatg tcaacagtac ccttagtata ttctccagta gatagggagc ccttgcatga 3300

caattctgct aacatcaaaa ggcctctagg ttcctttgtt acttcttctg ccgcctgctt 3360

caaaccgcta acaatacctg ggcccaccac accgtgtgca ttcgtaatgt ctgcccattc 3420

tgctattctg tatacacccg cagagtactg caatttgact gtattaccaa tgtcagcaaa 3480

ttttctgtct tcgaagagta aaaaattgta cttggcggat aatgccttta gcggcttaac 3540

tgtgccctcc atggaaaaat cagtcaagat atccacatgt gtttttagta aacaaatttt 3600

gggacctaat gcttcaacta actccagtaa ttccttggtg gtacgaacat ccaatgaagc 3660

acacaagttt gtttgctttt cgtgcatgat attaaatagc ttggcagcaa caggactagg 3720

atgagtagca gcacgttcct tatatgtagc tttcgacatg atttatcttc gtttcctgca 3780

ggtttttgtt ctgtgcagtt gggttaagaa tactgggcaa tttcatgttt cttcaacact 3840

acatatgcgt atatatacca atctaagtct gtgctccttc cttcgttctt ccttctgttc 3900

ggagattacc gaatcaaaaa aatttcaaag aaaccgaaat caaaaaaaag aataaaaaaa 3960

aaatgatgaa ttgaattgaa aagctgtggt atggtgcact ctcagtacaa tctgctctga 4020

tgccgcatag ttaagccagc cccgacaccc gccaacaccc gctgacgcgc cctgacgggc 4080

ttgtctgctc ccggcatccg cttacagaca agctgtgacc gtctccggga gctgcatgtg 4140

tcagaggttt tcaccgtcat caccgaaacg cgcgagacga aagggcctcg tgatacgcct 4200

atttttatag gttaatgtca tgataataat ggtttcttag tatgatccaa tatcaaagga 4260

aatgatagca ttgaaggatg agactaatcc aattgaggag tggcagcata tagaacagct 4320

aaagggtagt gctgaaggaa gcatacgata ccccgcatgg aatgggataa tatcacagga 4380

ggtactagac tacctttcat cctacataaa tagacgcata taagtacgca tttaagcata 4440

aacacgcact atgccgttct tctcatgtat atatatatac aggcaacacg cagatatagg 4500

tgcgacgtga acagtgagct gtatgtgcgc agctcgcgtt gcattttcgg aagcgctcgt 4560

tttcggaaac gctttgaagt tcctattccg aagttcctat tctctagaaa gtataggaac 4620

ttcagagcgc ttttgaaaac caaaagcgct ctgaagacgc actttcaaaa aaccaaaaac 4680

gcaccggact gtaacgagct actaaaatat tgcgaatacc gcttccacaa acattgctca 4740

aaagtatctc tttgctatat atctctgtgc tatatcccta tataacctac ccatccacct 4800

ttcgctcctt gaacttgcat ctaaactcga cctctacatt ttttatgttt atctctagta 4860

ttactcttta gacaaaaaaa ttgtagtaag aactattcat agagtgaatc gaaaacaata 4920

cgaaaatgta aacatttcct atacgtagta tatagagaca aaatagaaga aaccgttcat 4980

aattttctga ccaatgaaga atcatcaacg ctatcacttt ctgttcacaa agtatgcgca 5040

atccacatcg gtatagaata taatcgggga tgcctttatc ttgaaaaaat gcacccgcag 5100

cttcgctagt aatcagtaaa cgcgggaagt ggagtcaggc tttttttatg gaagagaaaa 5160

tagacaccaa agtagccttc ttctaacctt aacggaccta cagtgcaaaa agttatcaag 5220

agactgcatt atagagcgca caaaggagaa aaaaagtaat ctaagatgct ttgttagaaa 5280

aatagcgctc tcgggatgca tttttgtaga acaaaaaaga agtatagatt ctttgttggt 5340

aaaatagcgc tctcgcgttg catttctgtt ctgtaaaaat gcagctcaga ttctttgttt 5400

gaaaaattag cgctctcgcg ttgcattttt gttttacaaa aatgaagcac agattcttcg 5460

ttggtaaaat agcgctttcg cgttgcattt ctgttctgta aaaatgcagc tcagattctt 5520

tgtttgaaaa attagcgctc tcgcgttgca tttttgttct acaaaatgaa gcacagatgc 5580

ttcgttcagg tggcactttt cggggaaatg tgcgcggaac ccctatttgt ttatttttct 5640

aaatacattc aaatatgtat ccgctcatga gacaataacc ctgataaatg cttcaataat 5700

attgaaaaag gaagagtatg agtattcaac atttccgtgt cgcccttatt cccttttttg 5760

cggcattttg ccttcctgtt tttgctcacc cagaaacgct ggtgaaagta aaagatgctg 5820

aagatcagtt gggtgcacga gtgggttaca tcgaactgga tctcaacagc ggtaagatcc 5880

ttgagagttt tcgccccgaa gaacgttttc caatgatgag cacttttaaa gttctgctat 5940

gtggcgcggt attatcccgt attgacgccg ggcaagagca actcggtcgc cgcatacact 6000

attctcagaa tgacttggtt gagtactcac cagtcacaga aaagcatctt acggatggca 6060

tgacagtaag agaattatgc agtgctgcca taaccatgag tgataacact gcggccaact 6120

tacttctgac aacgatcgga ggaccgaagg agctaaccgc ttttttgcac aacatggggg 6180

atcatgtaac tcgccttgat cgttgggaac cggagctgaa tgaagccata ccaaacgacg 6240

agcgtgacac cacgatgcct gtagcaatgg caacaacgtt gcgcaaacta ttaactggcg 6300

aactacttac tctagcttcc cggcaacaat taatagactg gatggaggcg gataaagttg 6360

caggaccact tctgcgctcg gcccttccgg ctggctggtt tattgctgat aaatctggag 6420

ccggtgagcg tgggtctcgc ggtatcattg cagcactggg gccagatggt aagccctccc 6480

gtatcgtagt tatctacacg acggggagtc aggcaactat ggatgaacga aatagacaga 6540

tcgctgagat aggtgcctca ctgattaagc attggtaact gtcagaccaa gtttactcat 6600

atatacttta gattgattta aaacttcatt tttaatttaa aaggatctag gtgaagatcc 6660

tttttgataa tctcatgacc aaaatccctt aacgtgagtt ttcgttccac tgagcgtcag 6720

accccgtaga aaagatcaaa ggatcttctt gagatccttt ttttctgcgc gtaatctgct 6780

gcttgcaaac aaaaaaacca ccgctaccag cggtggtttg tttgccggat caagagctac 6840

caactctttt tccgaaggta actggcttca gcagagcgca gataccaaat actgtccttc 6900

tagtgtagcc gtagttaggc caccacttca agaactctgt agcaccgcct acatacctcg 6960
ctctgctaat cctgttacca gtggctgctg ccagtggcga taagtcgtgt cttaccgggt 7020
tggactcaag acgatagtta ccggataagg cgcagcggtc gggctgaacg gggggttcgt 7080
gcacacagcc cagcttggag cgaacgacct acaccgaact gagataccta cagcgtgagc 7140
tatgagaaag cgccacgctt cccgaaggga gaaaggcgga caggtatccg gtaagcggca 7200
gggtcggaac aggagagcgc acgagggagc ttccaggggg aaacgcctgg tatctttata 7260
gtcctgtcgg gtttcgccac ctctgacttg agcgtcgatt tttgtgatgc tcgtcagggg 7320
ggcggagcct atggaaaaac gccagcaacg cggccttttt acggttcctg gccttttgct 7380
ggccttttgc tcacatgttc tttcctgcgt tatcccctga ttctgtggat aaccgtatta 7440
ccgcctttga gtgagctgat accgctcgcc gcagccgaac gaccgagcgc agcgagtcag 7500
tgagcgagga agcggaagag cgcccaatac gcaaaccgcc tctccccgcg cgttggccga 7560
ttcattaatg cagctgaatt ggagcgacct catgctatac ctgagaaagc aacctgacct 7620
acaggaaaga gttactcaag aataagaatt ttcgttttaa aacctaagag tcactttaaa 7680
atttgtatac acttattttt tttataactt atttaataat aaaaatcata aatcataaga 7740
aattcgctta tttagaagtg tcaacaacgt atctaccaac gatttgaccc ttttccatct 7800
tttcgtaaat ttctggcaag gtagacaagc cgacaacctt gattggagac ttgaccaaac 7860
ctctggcgaa gaattgttaa ttaagagctc agatcttatt tttctgcact acgcagggat 7920
atttcaccgc ccatccaggg ttttattatt ccatcctgct caagtaataa agccccctgt 7980
ttgtctattc cgcgtgaaat gccaaatatt tctttatcac caatgataag tttcactggg 8040
cgattaataa aattatccag cttttcccag cgcgacagat aaggtgccaa tccttcttgt 8100
tcgaagagtt ccaacgcagc acgtaattca cgtattagca tggccgccaa cgtattacga 8160
tcgagattga tccccgcttc ctgcagcgtg atccacccct gattaacgac actctcttca 8220
acacggcgca ttgccatgtt gatcccggct ccaatgacta tttgcgccgc atcgccagtt 8280
ttgccagtca gctccaccag aatgcctgcc agcttgcgat cctgcagata gaggtcatta 8340
ggccatttaa cacgaacttt atctgcaccc agcttgcgta atacttccgc catcacgata 8400
ccgataacca gacttaaacc aatcgccgcc gccgggcctt gttccagacg ccagaacatc 8460
gacaaatata agtttgcgcc aaaaggcgaa aaccatttcc gaccccggcg accacggcca 8520
gcctgctggt attctgcaat gcaagcatcg cccgatttaa gctctccgat acgatcaaga 8580
aggtactgat tcgtggagtc aatcactggc agcacggcta cactaccgcc atccagctga 8640
cccaatatct gtttagcatt aagtaactgg ataggctcag gcaggctgta tcctttaccc 8700

ggaacggtaa agacatcaac gccccagtca cgcagtgtct gaatgtgttt attaatagcc 8760

gcccggctca ttcccagcgt ttcacccaac tgctcgccag agtgaaattc accgttcgct 8820

aacagggcaa tcaatttcag tggcacggtg ttatccttca tgaatt 8866

<210> 20
<211> 717
<212> DNA
<213> Artificial

<220>
<223> Nucleotide sequence of a synthetic DNA fragment comprising the
sequence encoding protein mAV_NANP

<400> 20
agatctatgg tgcacgccac ctcccctctg ctgctgctgc tgctgctgtc cctggccctg 60

gtcgctcctg gtctgtccgc tcgtaagtgc tccctgaccg gcaagtggac caacgacctg 120

ggttccaaca tgaccatcgg cgccgtcaac tcccgcggtg agttcaccgg cacctacatc 180

accgctgtca ccgctacctc caacgagatc aaggagtccc ctctgcacgg cacccaggct 240

accatcaaca agcgtaccca gcccaccttc ggtttcaccg tgaactggaa gttctccgag 300

tccaccaccg tgttcaccgg tcagtgcttc atcgaccgca acggcaagga ggtgctgaag 360

accatgtggc tgctgcgttc ctccgtgaac gacatcggtg acgacaagaa ggctacccgt 420

gtgggtatca acatcttcac ccgtctgcgt acccagaagg aggccatgga tcccaatgct 480

aatcctaacg ccaaccccaa tgccaaccca aacgccaatc ctaatgctaa ccctaacgct 540

aatcccaatg ccaatcccaa cgccaatcct aatgccaatc caaacgctaa ccctaatgct 600

aatccaaacg ccaatccata aggatccgga attcaaaggc ctacgtcgac gagctcaact 660

agtgcggccg ctttcgaatc tagagcctgc agtctcgagg catgcggtac caagctt 717

<210> 21
<211> 5382
<212> DNA
<213> Artificial

<220>
<223> Nucleotide sequence of plasmid pFB_mAV_NANP

<400> 21
agcttgtcga gaagtactag aggatcataa tcagccatac cacatttgta gaggttttac 60

ttgctttaaa aaacctccca cacctccccc tgaacctgaa acataaaatg aatgcaattg 120

ttgttgttaa cttgtttatt gcagcttata atggttacaa ataaagcaat agcatcacaa 180

atttcacaaa taaagcattt ttttcactgc attctagttg tggtttgtcc aaactcatca 240

atgtatctta tcatgtctgg atctgatcac tgcttgagcc taggagatcc gaaccagata 300

agtgaaatct agttccaaac tattttgtca tttttaattt tcgtattagc ttacgacgct 360

acacccagtt cccatctatt ttgtcactct tccctaaata atccttaaaa actccatttc 420

cacccctccc agttcccaac tattttgtcc gcccacagcg gggcattttt cttcctgtta 480

tgtttttaat caaacatcct gccaactcca tgtgacaaac cgtcatcttc ggctactttt 540

tctctgtcac agaatgaaaa tttttctgtc atctcttcgt tattaatgtt tgtaattgac 600

tgaatatcaa cgcttatttg cagcctgaat ggcgaatggg acgcgccctg tagcggcgca 660

ttaagcgcgg cgggtgtggt ggttacgcgc agcgtgaccg ctacacttgc cagcgcccta 720

gcgcccgctc ctttcgcttt cttcccttcc tttctcgcca cgttcgccgg ctttccccgt 780

caagctctaa atcgggggct ccctttaggg ttccgattta gtgctttacg gcacctcgac 840

cccaaaaaac ttgattaggg tgatggttca cgtagtgggc catcgccctg atagacggtt 900

tttcgccctt tgacgttgga gtccacgttc tttaatagtg gactcttgtt ccaaactgga 960

acaacactca accctatctc ggtctattct tttgatttat aagggatttt gccgatttcg 1020

gcctattggt taaaaaatga gctgatttaa caaaaattta acgcgaattt taacaaaata 1080

ttaacgttta caatttcagg tggcactttt cggggaaatg tgcgcggaac ccctatttgt 1140

ttatttttct aaatacattc aaatatgtat ccgctcatga gacaataacc ctgataaatg 1200

cttcaataat attgaaaaag gaagagtatg agtattcaac atttccgtgt cgcccttatt 1260

cccttttttg cggcattttg ccttcctgtt tttgctcacc cagaaacgct ggtgaaagta 1320

aaagatgctg aagatcagtt gggtgcacga gtgggttaca tcgaactgga tctcaacagc 1380

ggtaagatcc ttgagagttt tcgccccgaa gaacgttttc caatgatgag cacttttaaa 1440

gttctgctat gtggcgcggt attatcccgt attgacgccg ggcaagagca actcggtcgc 1500

cgcatacact attctcagaa tgacttggtt gagtactcac cagtcacaga aaagcatctt 1560

acggatggca tgacagtaag agaattatgc agtgctgcca taaccatgag tgataacact 1620

gcggccaact tacttctgac aacgatcgga ggaccgaagg agctaaccgc ttttttgcac 1680

aacatggggg atcatgtaac tcgccttgat cgttgggaac cggagctgaa tgaagccata 1740

ccaaacgacg agcgtgacac cacgatgcct gtagcaatgg caacaacgtt gcgcaaacta 1800

ttaactggcg aactacttac tctagcttcc cggcaacaat taatagactg gatggaggcg 1860

gataaagttg caggaccact tctgcgctcg gcccttccgg ctggctggtt tattgctgat 1920

aaatctggag ccggtgagcg tgggtctcgc ggtatcattg cagcactggg gccagatggt 1980

aagccctccc gtatcgtagt tatctacacg acggggagtc aggcaactat ggatgaacga 2040

aatagacaga tcgctgagat aggtgcctca ctgattaagc attggtaact gtcagaccaa 2100
gtttactcat atatacttta gattgattta aaacttcatt tttaatttaa aaggatctag 2160
gtgaagatcc tttttgataa tctcatgacc aaaatccctt aacgtgagtt ttcgttccac 2220
tgagcgtcag accccgtaga aaagatcaaa ggatcttctt gagatccttt ttttctgcgc 2280
gtaatctgct gcttgcaaac aaaaaaacca ccgctaccag cggtggtttg tttgccggat 2340
caagagctac caactctttt tccgaaggta actggcttca gcagagcgca gataccaaat 2400
actgtccttc tagtgtagcc gtagttaggc caccacttca agaactctgt agcaccgcct 2460
acatacctcg ctctgctaat cctgttacca gtggctgctg ccagtggcga taagtcgtgt 2520
cttaccgggt tggactcaag acgatagtta ccggataagg cgcagcggtc gggctgaacg 2580
gggggttcgt gcacacagcc cagcttggag cgaacgacct acaccgaact gagataccta 2640
cagcgtgagc attgagaaag cgccacgctt cccgaaggga gaaaggcgga caggtatccg 2700
gtaagcggca gggtcggaac aggagagcgc acgagggagc ttccaggggg aaacgcctgg 2760
tatctttata gtcctgtcgg gtttcgccac ctctgacttg agcgtcgatt tttgtgatgc 2820
tcgtcagggg ggcggagcct atggaaaaac gccagcaacg cggccttttt acggttcctg 2880
gccttttgct ggccttttgc tcacatgttc tttcctgcgt tatcccctga ttctgtggat 2940
aaccgtatta ccgcctttga gtgagctgat accgctcgcc gcagccgaac gaccgagcgc 3000
agcgagtcag tgagcgagga agcggaagag cgcctgatgc ggtattttct ccttacgcat 3060
ctgtgcggta tttcacaccg cagaccagcc gcgtaacctg gcaaaatcgg ttacggttga 3120
gtaataaatg gatgccctgc gtaagcgggt gtgggcggac aataaagtct taaactgaac 3180
aaaatagatc taaactatga caataaagtc ttaaactaga cagaatagtt gtaaactgaa 3240
atcagtccag ttatgctgtg aaaaagcata ctggactttt gttatggcta aagcaaactc 3300
ttcattttct gaagtgcaaa ttgcccgtcg tattaaagag gggcgtggcc aagggcatgg 3360
taaagactat attcgcggcg ttgtgacaat ttaccgaaca actccgcggc cgggaagccg 3420
atctcggctt gaacgaattg ttaggtggcg gtacttgggt cgatatcaaa gtgcatcact 3480
tcttcccgta tgcccaactt tgtatagaga gccactgcgg gatcgtcacc gtaatctgct 3540
tgcacgtaga tcacataagc accaagcgcg ttggcctcat gcttgaggag attgatgagc 3600
gcggtggcaa tgccctgcct ccggtgctcg ccggagactg cgagatcata gatatagatc 3660
tcactacgcg gctgctcaaa cctgggcaga acgtaagccg cgagagcgcc aacaaccgct 3720
tcttggtcga aggcagcaag cgcgatgaat gtcttactac ggagcaagtt cccgaggtaa 3780
tcggagtccg gctgatgttg ggagtaggtg gctacgtctc cgaactcacg accgaaaaga 3840

tcaagagcag cccgcatgga tttgacttgg tcagggccga gcctacatgt gcgaatgatg 3900

cccatacttg agccacctaa ctttgtttta gggcgactgc cctgctgcgt aacatcgttg 3960

ctgctgcgta acatcgttgc tgctccataa catcaaacat cgacccacgg cgtaacgcgc 4020

ttgctgcttg gatgcccgag gcatagactg tacaaaaaaa cagtcataac aagccatgaa 4080

aaccgccact gcgccgttac caccgctgcg ttcggtcaag gttctggacc agttgcgtga 4140

gcgcatacgc tacttgcatt acagtttacg aaccgaacag gcttatgtca actgggttcg 4200

tgccttcatc cgtttccacg gtgtgcgtca cccggcaacc ttgggcagca gcgaagtcga 4260

ggcatttctg tcctggctgg cgaacgagcg caaggtttcg gtctccacgc atcgtcaggc 4320

attggcggcc ttgctgttct tctacggcaa ggtgctgtgc acggatctgc cctggcttca 4380

ggagatcgga agacctcggc cgtcgcggcg cttgccggtg gtgctgaccc cggatgaagt 4440

ggttcgcatc ctcggttttc tggaaggcga gcatcgtttg ttcgcccagg actctagcta 4500

tagttctagt ggttggctac gtatactccg gaatattaat agatcatgga gataattaaa 4560

atgataacca tctcgcaaat aaataagtat tttactgttt tcgtaacagt tttgtaataa 4620

aaaaacctat aaatattccg gattattcat accgtcccac catcgggcgc ggatctatgg 4680

tgcacgccac ctcccctctg ctgctgctgc tgctgctgtc cctggccctg gtcgctcctg 4740

gtctgtccgc tcgtaagtgc tccctgaccg gcaagtggac caacgacctg ggttccaaca 4800

tgaccatcgg cgccgtcaac tcccgcggtg agttcaccgg cacctacatc accgctgtca 4860

ccgctacctc caacgagatc aaggagtccc ctctgcacgg cacccaggct accatcaaca 4920

agcgtaccca gcccaccttc ggtttcaccg tgaactggaa gttctccgag tccaccaccg 4980

tgttcaccgg tcagtgcttc atcgaccgca acggcaagga ggtgctgaag accatgtggc 5040

tgctgcgttc ctccgtgaac gacatcggtg acgacaagaa ggctacccgt gtgggtatca 5100

acatcttcac ccgtctgcgt acccagaagg aggccatgga tcccaatgct aatcctaacg 5160

ccaaccccaa tgccaaccca aacgccaatc ctaatgctaa ccctaacgct aatcccaatg 5220

ccaatcccaa cgccaatcct aatgccaatc caaacgctaa ccctaatgct aatccaaacg 5280

ccaatccata aggatccgga attcaaaggc ctacgtcgac gagctcaact agtgcggccg 5340

ctttcgaatc tagagcctgc agtctcgagg catgcggtac ca 5382

<210> 22
<211> 4
<212> PRT
<213> Artificial

<220>
<223> Amino acid sequence corresponding to the NANP peptide

<400>  22

Asn Ala Asn Pro
1


<210>  23
<211>  204
<212>  PRT
<213>  Artificial

<220>
<223>  Amino acid sequence of chimeric protein mAV_NANP

<400>  23

Met Val His Ala Thr Ser Pro Leu Leu Leu Leu Leu Leu Leu Ser Leu
1               5                   10                  15

Ala Leu Val Ala Pro Gly Leu Ser Ala Arg Lys Cys Ser Leu Thr Gly
            20                  25                  30

Lys Trp Thr Asn Asp Leu Gly Ser Asn Met Thr Ile Gly Ala Val Asn
        35                  40                  45

Ser Arg Gly Glu Phe Thr Gly Thr Tyr Ile Thr Ala Val Thr Ala Thr
    50                  55                  60

Ser Asn Glu Ile Lys Glu Ser Pro Leu His Gly Thr Gln Ala Thr Ile
65                  70                  75                  80

Asn Lys Arg Thr Gln Pro Thr Phe Gly Phe Thr Val Asn Trp Lys Phe
                85                  90                  95

Ser Glu Ser Thr Thr Val Phe Thr Gly Gln Cys Phe Ile Asp Arg Asn
            100                 105                 110

Gly Lys Glu Val Leu Lys Thr Met Trp Leu Leu Arg Ser Ser Val Asn
        115                 120                 125

Asp Ile Gly Asp Asp Lys Lys Ala Thr Arg Val Gly Ile Asn Ile Phe
    130                 135                 140

Thr Arg Leu Arg Thr Gln Lys Glu Ala Met Asp Pro Asn Ala Asn Pro
145                 150                 155                 160

Asn Ala Asn Pro Asn Ala Asn Pro Asn Ala Asn Pro Asn Ala Asn Pro
                165                 170                 175

Asn Ala Asn Pro Asn Ala Asn Pro Asn Ala Asn Pro Asn Ala Asn Pro
            180                 185                 190

Asn Ala Asn Pro Asn Ala Asn Pro Asn Ala Asn Pro
        195                 200

<210>  24
<211>  5228
<212>  DNA
<213>  Artificial

<220>
<223>  Nucleotide sequence of plasmid pFB_mAV

<400>  24
gtatactccg gaatattaat agatcatgga gataattaaa atgataacca tctcgcaaat      60

aaataagtat tttactgttt tcgtaacagt tttgtaataa aaaaacctat aaatattccg     120

gattattcat accgtcccac catcgggcgc ggatctatgg tgcacgccac ctcccctctg     180

ctgctgctgc tgctgctgtc cctggccctg gtcgctcctg gtctgtccgc tcgtaagtgc     240

tccctgaccg gcaagtggac caacgacctg ggttccaaca tgaccatcgg cgccgtcaac     300

tcccgcggtg agttcaccgg cacctacatc accgctgtca ccgctacctc caacgagatc     360

aaggagtccc ctctgcacgg cacccaggct accatcaaca agcgtaccca gcccaccttc     420

ggtttcaccg tgaactggaa gttctccgag tccaccaccg tgttcaccgg tcagtgcttc     480

atcgaccgca acggcaagga ggtgctgaag accatgtggc tgctgcgttc ctccgtgaac     540

gacatcggtg acgacaagaa ggctacccgt gtgggtatca acatcttcac ccgtctgcgt     600

acccagaagg aggccatgga tccggaattc aaaggcctac gtcgacgagc tcaactagtg     660

cggccgcttt cgaatctaga gcctgcagtc tcgaggcatg cggtaccaag cttgtcgaga     720

agtactagag gatcataatc agccatacca catttgtaga ggttttactt gctttaaaaa     780

acctcccaca cctccccctg aacctgaaac ataaaatgaa tgcaattgtt gttgttaact     840

tgtttattgc agcttataat ggttacaaat aaagcaatag catcacaaat ttcacaaata     900

aagcattttt ttcactgcat tctagttgtg gtttgtccaa actcatcaat gtatcttatc     960

atgtctggat ctgatcactg cttgagccta ggagatccga accagataag tgaaatctag    1020

ttccaaacta ttttgtcatt tttaattttc gtattagctt acgacgctac acccagttcc    1080

catctatttt gtcactcttc cctaaataat ccttaaaaac tccatttcca cccctcccag    1140

ttcccaacta ttttgtccgc ccacagcggg gcatttttct tcctgttatg tttttaatca    1200

aacatcctgc caactccatg tgacaaaccg tcatcttcgg ctactttttc tctgtcacag    1260

aatgaaaatt tttctgtcat ctcttcgtta ttaatgtttg taattgactg aatatcaacg 1320

cttatttgca gcctgaatgg cgaatgggac gcgccctgta gcggcgcatt aagcgcggcg 1380

ggtgtggtgg ttacgcgcag cgtgaccgct acacttgcca gcgccctagc gcccgctcct 1440

ttcgctttct tcccttcctt tctcgccacg ttcgccggct ttccccgtca agctctaaat 1500

cgggggctcc ctttagggtt ccgatttagt gctttacggc acctcgaccc caaaaaactt 1560

gattagggtg atggttcacg tagtgggcca tcgccctgat agacggtttt tcgccctttg 1620

acgttggagt ccacgttctt taatagtgga ctcttgttcc aaactggaac aacactcaac 1680

cctatctcgg tctattcttt tgatttataa gggattttgc cgatttcggc ctattggtta 1740

aaaaatgagc tgatttaaca aaaatttaac gcgaatttta acaaaatatt aacgtttaca 1800

atttcaggtg gcacttttcg gggaaatgtg cgcggaaccc ctatttgttt atttttctaa 1860

atacattcaa atatgtatcc gctcatgaga caataaccct gataaatgct tcaataatat 1920

tgaaaaagga agagtatgag tattcaacat ttccgtgtcg cccttattcc cttttttgcg 1980

gcattttgcc ttcctgtttt tgctcaccca gaaacgctgg tgaaagtaaa agatgctgaa 2040

gatcagttgg gtgcacgagt gggttacatc gaactggatc tcaacagcgg taagatcctt 2100

gagagttttc gccccgaaga acgttttcca atgatgagca cttttaaagt tctgctatgt 2160

ggcgcggtat tatcccgtat tgacgccggg caagagcaac tcggtcgccg catacactat 2220

tctcagaatg acttggttga gtactcacca gtcacagaaa agcatcttac ggatggcatg 2280

acagtaagag aattatgcag tgctgccata accatgagtg ataacactgc ggccaactta 2340

cttctgacaa cgatcggagg accgaaggag ctaaccgctt ttttgcacaa catgggggat 2400

catgtaactc gccttgatcg ttgggaaccg gagctgaatg aagccatacc aaacgacgag 2460

cgtgacacca cgatgcctgt agcaatggca acaacgttgc gcaaactatt aactggcgaa 2520

ctacttactc tagcttcccg gcaacaatta atagactgga tggaggcgga taaagttgca 2580

ggaccacttc tgcgctcggc ccttccggct ggctggttta ttgctgataa atctggagcc 2640

ggtgagcgtg ggtctcgcgg tatcattgca gcactggggc cagatggtaa gccctcccgt 2700

atcgtagtta tctacacgac ggggagtcag gcaactatgg atgaacgaaa tagacagatc 2760

gctgagatag gtgcctcact gattaagcat tggtaactgt cagaccaagt ttactcatat 2820

atactttaga ttgatttaaa acttcatttt taatttaaaa ggatctaggt gaagatcctt 2880

tttgataatc tcatgaccaa aatcccttaa cgtgagtttt cgttccactg agcgtcagac 2940

cccgtagaaa agatcaaagg atcttcttga gatccttttt ttctgcgcgt aatctgctgc 3000

ttgcaaacaa aaaaaccacc gctaccagcg gtggtttgtt tgccggatca agagctacca 3060

actctttttc cgaaggtaac tggcttcagc agagcgcaga taccaaatac tgtccttcta 3120

gtgtagccgt agttaggcca ccacttcaag aactctgtag caccgcctac atacctcgct 3180

ctgctaatcc tgttaccagt ggctgctgcc agtggcgata agtcgtgtct taccgggttg 3240

gactcaagac gatagttacc ggataaggcg cagcggtcgg gctgaacggg gggttcgtgc 3300

acacagccca gcttggagcg aacgacctac accgaactga gatacctaca gcgtgagcat 3360

tgagaaagcg ccacgcttcc cgaagggaga aaggcggaca ggtatccggt aagcggcagg 3420

gtcggaacag gagagcgcac gagggagctt ccagggggaa acgcctggta tctttatagt 3480

cctgtcgggt ttcgccacct ctgacttgag cgtcgatttt tgtgatgctc gtcagggggg 3540

cggagcctat ggaaaaacgc cagcaacgcg gcctttttac ggttcctggc cttttgctgg 3600

ccttttgctc acatgttctt tcctgcgtta tcccctgatt ctgtggataa ccgtattacc 3660

gcctttgagt gagctgatac cgctcgccgc agccgaacga ccgagcgcag cgagtcagtg 3720

agcgaggaag cggaagagcg cctgatgcgg tattttctcc ttacgcatct gtgcggtatt 3780

tcacaccgca gaccagccgc gtaacctggc aaaatcggtt acggttgagt aataaatgga 3840

tgccctgcgt aagcgggtgt gggcggacaa taaagtctta aactgaacaa aatagatcta 3900

aactatgaca ataaagtctt aaactagaca gaatagttgt aaactgaaat cagtccagtt 3960

atgctgtgaa aaagcatact ggacttttgt tatggctaaa gcaaactctt cattttctga 4020

agtgcaaatt gcccgtcgta ttaaagaggg gcgtggccaa gggcatggta aagactatat 4080

tcgcggcgtt gtgacaattt accgaacaac tccgcggccg ggaagccgat ctcggcttga 4140

acgaattgtt aggtggcggt acttgggtcg atatcaaagt gcatcacttc ttcccgtatg 4200

cccaactttg tatagagagc cactgcggga tcgtcaccgt aatctgcttg cacgtagatc 4260

acataagcac caagcgcgtt ggcctcatgc ttgaggagat tgatgagcgc ggtggcaatg 4320

ccctgcctcc ggtgctcgcc ggagactgcg agatcataga tatagatctc actacgcggc 4380

tgctcaaacc tgggcagaac gtaagccgcg agagcgccaa caaccgcttc ttggtcgaag 4440

gcagcaagcg cgatgaatgt cttactacgg agcaagttcc cgaggtaatc ggagtccggc 4500

tgatgttggg agtaggtggc tacgtctccg aactcacgac cgaaaagatc aagagcagcc 4560

cgcatggatt tgacttggtc agggccgagc ctacatgtgc gaatgatgcc catacttgag 4620

ccacctaact ttgttttagg gcgactgccc tgctgcgtaa catcgttgct gctgcgtaac 4680

atcgttgctg ctccataaca tcaaacatcg acccacggcg taacgcgctt gctgcttgga 4740

tgcccgaggc atagactgta caaaaaaaca gtcataacaa gccatgaaaa ccgccactgc 4800

gccgttacca ccgctgcgtt cggtcaaggt tctggaccag ttgcgtgagc gcatacgcta 4860

cttgcattac agtttacgaa ccgaacaggc ttatgtcaac tgggttcgtg ccttcatccg 4920

tttccacggt gtgcgtcacc cggcaacctt gggcagcagc gaagtcgagg catttctgtc 4980

ctggctggcg aacgagcgca aggtttcggt ctccacgcat cgtcaggcat tggcggcctt 5040

gctgttcttc tacggcaagg tgctgtgcac ggatctgccc tggcttcagg agatcggaag 5100

acctcggccg tcgcggcgct tgccggtggt gctgaccccg gatgaagtgg ttcgcatcct 5160

cggttttctg gaaggcgagc atcgtttgtt cgcccaggac tctagctata gttctagtgg 5220

ttggctac 5228

## Description

### Field of the Invention

The invention relates to a system for the transport and presentation of products of interest based on nanocarriers (NCs) formed by protein assemblies, obtained by means of the assembly of a genetically modified self assembly protein in order to couple products of interest bound to a ligand. The invention also related to processes for producing them and applications.

### Background of the Invention

The development of molecular biology techniques has allowed conducting systematic studies aimed at characterizing at a molecular level the mechanisms directing and controlling the processes for assembling different types of macromolecules. These types of studies have been especially successful in characterizing essential macromolecular assemblies for the correct functioning of the replicative cycle of a large number of viruses. The study of the processes for assembling viral capsids has given rise to the isolation of self assembly proteins to give rise to the formation of stable structures generically referred to as virus like particles (VLPs) (Kingsman and Kingsman, 1988; Murray and Eaton, 1996, Casal et al., 1999; Maranga et al., 2002). VLPs obtained by means of expressing a large number of viral proteins in the development of non-infective vaccines (subunit) against various diseases (Yao et al., 2003; Maclean et al., 2005; Hammonds et al., 2007). The genetic modification of some of these proteins has allowed obtaining chimeric VLPs characterized by the insertion of heterologous amino acid regions corresponding to immunogenically relevant proteins of different origin (Niikura et al., 2002; Varsani et al., 2003). Chimeric VLPs have essentially been used for the development of subunit vaccines which can induce a specific response against heterologous epitopes introduced in the protein which can self assemble (Xu et al., 2006; Mejia et al., 2006). The viability of these types of vaccines is conditioned by the technical difficulty required for introducing a repertoire of epitopes that is wide enough so as to assure the induction of an immune response against a sufficient number of antigenic determinants of the pathogen of interest.

Various systems for the transport and presentation of products of interest based on protein assemblies are known.

EP1849799 describes VLPs based on SV40 protein V1 in which different epitopes are inserted in an internal region of said protein, including the myc, FLAG, HA, Tat-PTD, hexahistidine, RGD epitopes, etc., as well as VLBs formed by the assembly of said protein and conjugates formed by VLPs containing the FLAG epitope and anti-FLAG antibodies.

US2004/033585 describes VLPs based on tobacco mosaic virus (TMV) coat protein U1 in which epitopes have been introduced in an internal region of said protein forming an exposed loop in the VLP. Said epitopes include the V5 epitope, the HA epitope and the myc epitope, as well as the formation of complexes between the VLP and a ligand that specifically recognizes the epitope inserted in the protein U1.

US2006/0121468 describes VLPs based on infectious pancreatic necrosis virus (IPNV) proteins VP2 and VP3 containing HIV-1 epitopes in an internal region of said sequences resulting in their presentation in a loop exposed to the solvent.

US2006/0210587 describes VLPs based on bovine papillomavirus type 1 (BPV-1) protein L1 comprising the sequence of an extracellular CCR5 loop wherein said region is inserted in an internal region of protein L1.

WO0069907 describes oligomers formed from subunit cpn10 of chaperonin (GroES and protein Gp31 of phage T4) in which an antigenic epitope is inserted in an internal region of said sequence (the so-called mobile loop) such that after the oligomer is assembled, the antigenic epitope is exposed to the exterior; conjugates formed by VLPs based on Gp31 with an antibody are furthermore described.

In addition, the possibility of modifying different polypeptides by means of introducing the sequence corresponding to the biotin acceptor peptide (BAP) recognized by biotin ligase (BL) encoded by *Escherichia coli* gene BirA (Barker et al., 1981). The presence of this peptide in recombinant proteins allows the specific ligation of a biotin molecule to such proteins (Parrott and Barry, 2001). This approach has allowed developing systems for purifying soluble proteins and protein complexes (Nesbeth et al., 2006; Barat and Wu, 2007).

Although several systems for the transport and presentation of products of interest based on protein assemblies are known, it is still necessary to develop alternative systems to the currently existing ones for the purpose of increasing the stock of such systems.

### Brief Description of the Drawings

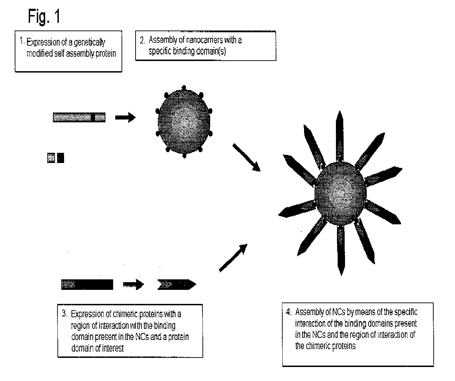

Figure 1 schematically shows the production of nanocarriers (NCs) loaded with recombinant proteins. The strategy briefly consists of: on one hand, (1) expressing a genetically modified self assembly protein (gray lines) by means of introducing a peptide insert or heterologous sequence (black) comprising a specific binding domain (black) in an exposed region of the protein assembly (2); on the other hand, constructing vectors for expressing bipartite recombinant proteins (3) comprising a region (dotted) with the capacity to interact with the exposed specific binding domain in the NCs and a region comprising a protein of interest (white); and, finally, (4) the interaction between the NCs and the recombinant proteins leads to the formation of stable complexes wherein the recombinant proteins coat the surface of the NC.

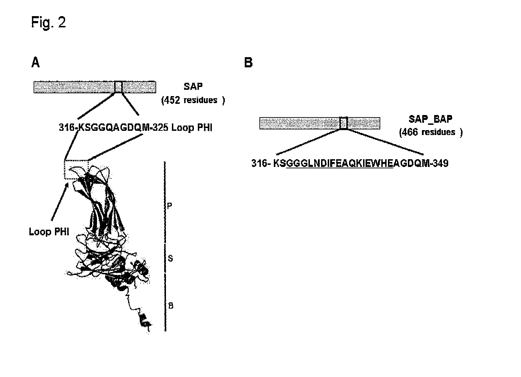

Figure 2 shows the experimental design for constructing the self assembly fusion protein SAP_BAP (SEQ ID NO: 4). In the depicted case, the self assembly protein (SAP) is the protein identified as IBDV pVP2_452 [protein comprising amino acids 1-452 of infectious bursal disease virus(IBDV) protein VP2] and the BAP peptide is a biotin acceptor peptide having the amino acid sequence shown in SEQ ID NO: 3. Figure 2A schematically shows the position of the flexible loop P_{HI} inside the primary sequence of said SAP as well as its position (marked in a box) inside the atomic structure of the latter (lower part of the panel); the corresponding image was generated by using the Chimera computer program with the access code of the Protein Data Base PDB: 2GSY, corresponding to pVP2_452 (Garriga et al., 2006). The amino acid sequence of loop P_{HI} as well as the numbers corresponding to the initial and terminal residues of said loop P_{HI} in the sequence of the SAP (pVP2_452) are shown. The different domains, protuberant (P), capsid (S), base (B), of said BAP peptide are also indicated. The diagram shown in Figure 2B schematically shows the position of the BAP peptide inside the flexible loop P_{HI} in fusion protein SAP_BAP. The amino acid sequence of the modified loop P_{HT} as well as the numbers corresponding to the initial and terminal residues of the mentioned loop in the sequence of fusion protein SAP_BAP are shown.

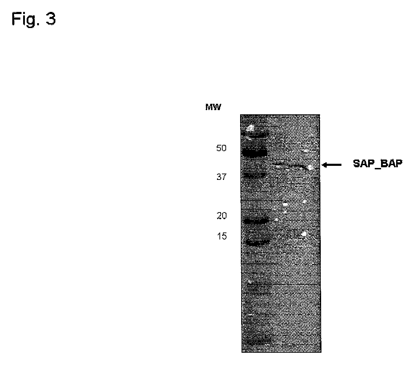

Figure 3 shows the expression of self assembly fusion protein SAP_BAP (SEQ ID NO: 4) [Example 1.2]. Briefly, an extract of a culture of insect cells infected with the recombinant baculovirus rBV/FB_SAP_BAP was analyzed by means of SDS-PAGE and immunoblotting using a specific serum against the SAP (IBDV anti-pVP2_452). The positions of the band recognized by the anti-serum (SAP_BAP) corresponding to fusion protein SAP_BAP and the positions of the bands corresponding to the pre-stained molecular weight (MW) markers used in the assay are indicates. The molecular mass of the markers is indicated in kDa. The SAP is IBDV pVP2_452 and the BAP peptide has the amino acid sequence shown in SEQ ID NO: 3.

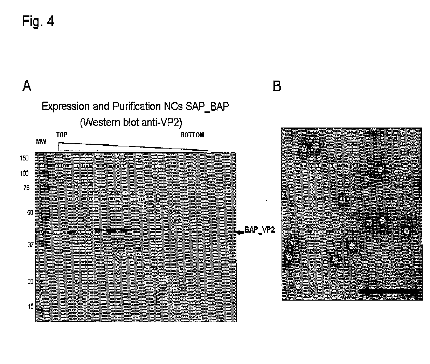

Figure 4 shows the purification and structural characterization of NCs produced by means of the expression of fusion protein SAP_BAP (SEQ ID NO: 4). An extract of a culture of insect cells infected with the recombinant baculovirus rBV/FB_SAP_BAP was used to purify the NCs produced by means of centrifuging in sucrose gradients. Figure 4A shows the results of the analysis by means of SDS-PAGE and immunoblotting of the fractions collected from a 20-50% sucrose gradient. The positions of the bands recognized by the anti-serum (IBDV anti-pVP2_452) corresponding to fusion protein SAP_BAP and the positions of the bands corresponding to the pre-stained molecular weight (MW) markers used in the assay are indicated. The molecular mass of the markers is indicated in kDa. The positions of the samples corresponding to the upper and lower areas of the gradient are indicated with the words top and bottom, respectively. The box indicates the fractions used for the subsequent structural characterization of the purified NCs. The fractions corresponding to the area of the gradient with a higher content of fusion protein SAP_BAP were mixed and analyzed by means of electronic microscopy. Figure 4B is a micrograph corresponding to a samples subjected to negative staining with uranyl acetate. The bar indicates 100 Ä. The SAP is IBDV pVP2_452 and the BAP peptide has the amino acid sequence shown in SEQ ID NO: 3.

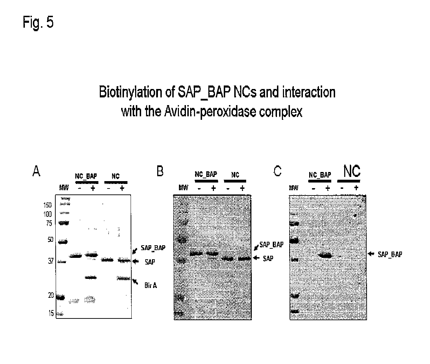

Figure 5 shows the characterization of the biotinylation of NCs produced by means of the expression of fusion protein SAP_BAP (SEQ ID NO: 4). Briefly, samples containing purified NCs from insect cells infected with the recombinant baculovirus rBV/FB_SAP_BAP were incubated with biotin in the presence (+) or absence (-) of the commercial biotin ligase (BL) enzyme. After the incubation, the samples were subjected to SDS-PAGE. The corresponding gels were stained with Coomassie blue (Figure 5A), or transferred to nitrocellulose membranes. One of these membranes was incubated with rabbit anti-SAP serum (IBDV anti-pVP2_452) followed by an incubation with goat IgG anti-rabbit IgG peroxidase-conjugate, and subsequently developed by means of incubation with 1-chloro-4-naphthol (Figure 5B). A second membrane was incubated with peroxidase-conjugated streptavidin and subsequently developed by means of incubation with 1-chloro-4-naphthol (Figure 5C). In an identical manner, purified NCs from insect cells infected with the recombinant baculovirus rBV/FB_SAP (NC_SAP) expressing SAP (without the BAP peptide) were incubated. The positions of proteins SAP_BAP, SAP and BL, as well as those of the pre-stained molecular weight markers (in kDa) used in the assays are indicated. The SAP is IBDV pVP2_452 and the BAP peptide has the amino acid sequence shown in SEQ ID NO: 3.

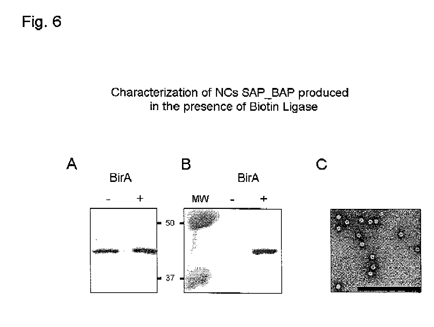

Figure 6 shows the characterization of NCs by the assembly of fusion protein SAP_BAP (SEQ ID NO: 4) produced in the presence of the biotin ligase (BL) enzyme. Samples containing purified NCs from insect cells infected with the recombinant baculovirus rBV_FS_SAF_BAP (BirA -) or rBV_FB_SAP_BAP_BirA (BirA +) were subjected to SDS-PAGE. The corresponding gels were transferred to nitrocellulose membranes. One of the membranes was incubated with rabbit anti-SAP serum (IBDV anti-pVP2_452) followed by an incubation with goat IgG anti-rabbit IgG peroxidase-conjugate, and subsequently developed by means of incubation with 1-chloro-4-naphthol (Figure 6A). A second membrane was incubated with peroxidase-conjugated streptavidin (Figure 6B) and subsequently developed by means of incubation with 1-chloro-4-naphthol. The position of the molecular weight markers (in kDa) used in the assay is indicated. Figure 6C shows a micrograph corresponding to purified NCs from cultures infected with rBV_FB_SAP_BAP_BirA subjected to negative staining with uranyl acetate and displayed by means of electronic microscopy. The bar indicates 100 Å. The SAP is IBDV pVP2_452 and the BAP peptide has the amino acid sequence shown in the SEQ ID NO: 3.

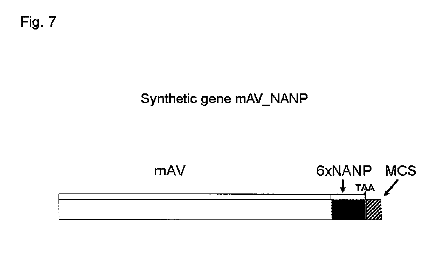

Figure 7 is a schematic diagram of chimeric gene mAV_NANP. Said diagram shows the essential elements of chimeric gene mAV_NANP (SEQ ID NO: 20) synthesized in vitro and subsequently cloned into vector pFastBacl. The region encoding a mutant form of avidin (mAV) of *G. gallus*, the region corresponding to the sequence encoding 6 consecutive repetitions of the NANP peptide (6xNANP) followed by a stop codon (TAA) and by the region corresponding to a multiple cloning site (MCS) are indicated.

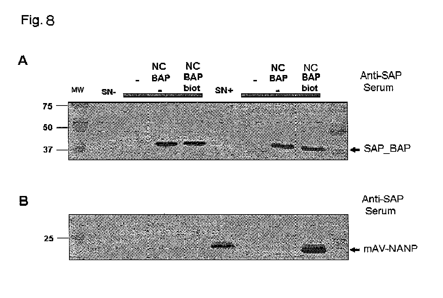

Figure 8 shows the characterization of the interaction between protein mAV_NANP (SEQ ID NO: 23) and biotinylated NCs *in vitro*. Briefly, supernatants of cultures of insect cells that are not infected (SN-) or infected with the recombinant baculovirus rBV/FB_mAV_NANP (SN+) were incubated with biotinylated NCs (NC-biot), or with non-biotinylated NCs (NC-) or with buffer lacking NCs (-). Total samples of SN- and SN+ not subjected to ultracentrifugation were analyzed as controls. For the purpose of separating soluble proteins from the proteins associated to NCs, after the incubation, the samples were deposited on a 20% sucrose cushion and subjected to ultracentrifugation. The sediments obtained were resuspended in loading buffer and subjected to SDS-PAGE and immunoblotting. The resulting nitrocellulose membranes were divided into two halves. The upper half of the membranes (A) was incubated with rabbit anti-SAP serum and the lower half (B) with rabbit anti-avidin serum. Finally, the membranes were incubated with goat IgG anti-rabbit IgG peroxidase-conjugate and subsequently developed by means of incubation with 1-chloro-4-naphthol. The positions of fusion proteins SAP_BAP (SEQ ID NO: 4) and mAV_NANP (SEQ ID NO: 23) as well as those of the pre-stained molecular weight markers (in kDa) used in the assays are indicated. The SAP is protein IBDV VP2_452 and the BAP peptide has the amino acid sequence shown in SEQ ID NO: 3.

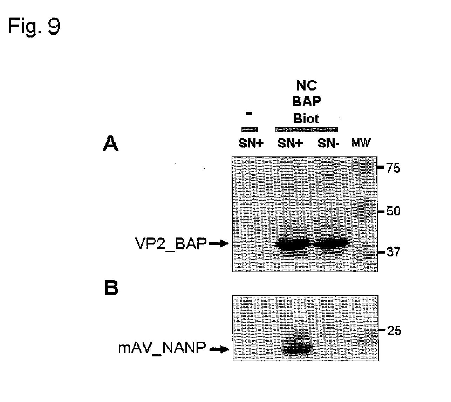

Figure 9 shows the characterization of the interaction between protein mAV_NANP (SEQ ID NO: 23) and biotinylated NCs *in vivo*. Briefly, supernatants of cultures of insect cells that are not infected (SN-) or infected with the recombinant baculovirus rBV/FB_mAV_NANP (SN+) were incubated with biotinylated NCs *in vivo* (NC-Biot). A corresponding sample of SN+ incubated in the absence of NCs (-) was analyzed as a control. For the purpose of separating soluble proteins from the proteins associated to NCs, after the incubation, the samples were deposited on a 20% sucrose cushion and subjected to ultracentrifugation. The sediments obtained were resuspended in loading buffer and subjected to SDS-PAGE and immunoblotting. The resulting nitrocellulose membranes were divided into two halves. The upper half of the membranes (A) was incubated with rabbit anti-SAP serum and the lower half (B) with rabbit anti-avidin serum. Finally, the membranes were incubated with goat IgG anti-rabbit IgG peroxidase-conjugate and subsequently developed by means of incubation with 1-chloro-4-naphthol.

### Detailed Description of the Invention

The invention generally relates to a nanocarrier (NC) which can be obtained by the assembly of fusion proteins comprising
(i) a self assembly protein and (ii) a heterologous peptide comprising an amino acid sequence with the capacity to bind to a ligand. For the sake of simplicity, the abbreviation "SAP" (self assembly protein) is occasionally used in this description to refer to the self assembly protein and the term "peptide insert" is used to refer to said heterologous peptide comprising an amino acid sequence with the capacity to bind to a ligand.

In a particular embodiment, said peptide insert (heterologous peptide comprising an amino acid sequence with the capacity to bind to a ligand) is inserted inside the amino acid sequence of said self assembly protein (SAP), whereas in another particular embodiment, said peptide insert is bound to the amino or carboxyl terminal end of said SAP.

In another particular embodiment, said NC further comprises a product of interest. Said product of interest forms part of a conjugate comprising, in addition to the product of interest, a ligand with the capacity to bind to said peptide insert present in the NC (as a result of the assembly of the fusion proteins forming said NC). The binding of said product of interest to said NC is carried out through said ligand by means of the specific interaction between the specific binding domain present in the peptide insert of the NC and the region of interaction with said specific binding domain present in the peptide insert, contained in the ligand which is present in the conjugate comprising the product of interest. The binding of said ligand to said peptide insert can be carried out either directly or indirectly through a connector.

The different components of said NC will be described in detail below.

### 1. Fusion protein (I) of the invention

In one aspect, the invention relates to a fusion protein (I), hereinafter fusion protein (I) of the invention, comprising
(i) a self assembly protein (occasionally identified in this description as "SAP"), and
(ii) a heterologous peptide comprising an amino acid sequence with the capacity to bind to a ligand (occasionally identified in this description as "peptide insert").

In a particular embodiment, said peptide insert is inserted inside the amino acid sequence of said self assembly protein (SAP), whereas in another particular embodiment, said peptide insert is bound to the amino or carboxyl terminal end of said SAP.

### 1.1 Self assembly protein

As used herein, the term "self assembly protein" or "SAP" includes any protein with self assembly capacity and the capacity to form macromolecular structures, for example, capsids, tubular assemblies (tubules), rings, ("donuts"), chaperone complexes, caveolin complexes, etc. Virtually any prokaryotic or eukaryotic protein with self-assembly capacity can be used in the present invention, for example, viral proteins, chaperones, caveolins, etc., as well as functional variants thereof, i.e. mutant forms of said proteins obtained by the addition of one or more amino acids to the corresponding native protein, or by the (conservative or non-conservative) substitution of one or more amino acids of the corresponding native protein with one or more amino acids, provided that said functional variants conserve the self assembly capacity of native proteins.

Illustrative non-limiting examples of viral proteins useful as self assembly proteins include viral structural proteins, such as proteins forming the capsids of different viruses, etc., for example, SV40 protein V1, tobacco mosaic virus (TMV) coat protein U1, bovine papillomavirus type 1 (BPV-1) protein L1, birnavirus structural proteins (e.g. infectious bursal disease virus (IBDV) protein VP2 (pVP2), infectious pancreatic necrosis virus (IPNV) proteins VP2 and VP3, etc.), etc.

In a particular embodiment, said self assembly protein comprises IBDV protein VP2 or a functional variant thereof. As used herein, the term "IBDV protein VP2 (or pVP2)" generally relates to a protein the amino acid sequence of which comprises or is formed by the amino acid sequence of the IBDV pVP2 of any IBDV strain belonging to any of the known serotypes (1 or 2) [by way of illustration see the review by van den Berg TP et al. (2000)] and includes any of the different forms of proteins pVP2 representative of any IBDV strain, according to the definition made by Sánchez and Rodriguez (1999) as well as to proteins that are substantially homologous to said IBDV proteins pVP2, i.e. proteins having a good alignment with the sequence of a certain IBDV pVP2, for example, proteins the amino acid sequences of which have a degree of freedom with respect to said IBDV proteins pVP2 of at least 60%, preferably of at least 80%, more preferably of at least 90% and even more preferably of at least 95%. Sequences that are homologous to a sequence of IBDV protein pVP2 can be easily identified a person skilled in the art with the aid of a suitable computer program for comparing sequences, for example, the BLAST program (Altschul et al. (1997)). In a particular embodiment, IBDV protein pVP2 is the Soroa strain IBDV protein pVP2 the amino acid sequence of which, complete length, is deposited in the NCBI with accession number AAD30136.

In another particular embodiment, said self assembly protein comprises a 1-n fragment of IBDV pVP2, wherein "n" is an integer comprised between 441 and 512. The term "1-n fragment of the/said IBDV pVP2, wherein "n" is an integer comprised between 441 and 512", generally relates to a peptide the amino acid sequence of which is formed by the contiguous amino acid sequence comprised between residue 1 and residue "n" of IBDV protein pVP2, wherein "n" is a integer comprised between 441 and 512. Therefore, said 1-n fragment of IBDV pVP2 has an amino acid sequence formed by or comprising between 441 and 512 contiguous amino acid residues, counted from the amino acid residue number 1, of a pVP2 representative of any IBDV strain, for example, of the Soroa strain IBDV pVP2 [NCBI, accession number AAD30136]. Particular 1-n fragments of IBDV pVP2 are called according to the "pVP2_n" format, wherein "n" is that defined above. In a specific embodiment, said self assembly protein (SAP) is that identified as IBDV pVP2_452, the amino acid sequence of which is shown in SEQ ID NO: 1, and is formed by the amino acid sequence comprised between residue 1 and residue 452 of IBDV pVP2. Said protein contains loop P_{HI} in which the peptide insert can be inserted, if desired. The assembly of the proteins identified as pVF2_441, pVP2_452, pVP2_456 and pVP2-466 gives rise to NCs, whereas the assembly of proteins pVP2_476, pVP2_487, pVP2_494 and pVP2_501 form tubular structures or tubules.

Illustrative non-limiting examples of chaperones useful as self assembly proteins include proteins Hsp60 (GroEL/GroES complex in *E. coli*), Hsp70 (DnaK in *E. coli*), Hsp90 (HtpG in *E. coli*) and Hsp100 (Clp family (e.g. ClpA, ClpX, ClpP, etc.) in E. coli), etc. Illustrative non-limiting examples of caveolins useful as self assembly proteins include proteins caveolin-1, caveolin-2, caveolin-3, etc. The amino acid sequences of these proteins, as well as relevant information about them, are known and can be obtained in the corresponding database known by persons skilled in the art (e.g. Protein Data Bank, Swissprot, etc.).

### 1.2 Peptide insert

The fusion protein (I) of the invention comprises, in addition to said SAP, a peptide insert or heterologous peptide comprising an amino acid sequence with the capacity to bind to a ligand (i.e. a specific binding domain).

As used herein, the term "peptide insert" [i.e. heterologous peptide comprising an amino acid sequence with the capacity to bind to a ligand] does not involve any particular size or any other technical structural characteristic other than that said peptide insert (comprising a specific binding domain) can bind to a ligand (comprising the region of interaction corresponding to said specific binding domain present in the peptide insert); nevertheless, in a particular embodiment, for its use in the present invention, said peptide insert must not adversely affect the assembly capacity of the SAP (i.e. the SAP in which said peptide insert is introduced or which it binds to must not lose its assembly capacity). The size of the peptide insert can vary within a wide range, for example, between 1 and 50 amino acid residues; advantageously, said peptide insert has less than 40 amino acid residues, preferably less than 30 amino acid residues, more preferably less than 20 amino acid residues, for example, between 1 and 15 amino acids.

Therefore, virtually any heterologous peptide with the capacity to bind to a ligand can be used in the present invention. By way of a non-limiting illustration, said peptide can be a member of a specific binding pair, for example, an amino acid tag (e.g. a histidine tag (his-tag), an arginine tag (arg-tag), etc.), a peptide epitope which can be recognized by an antibody (e.g. c-myc-tag, etc.), a biotin acceptor peptide (BAP), a linear domain of interaction with another or other protein or proteins (e.g. proteins SH3, signal transduction proteins, etc.), a post-translational modification sequence (e.g. myristoylation, methylation, phosphorylation, etc.), a sequence of transport to a cell compartment, etc.

In a particular embodiment, said peptide with the capacity to bind to a ligand is a BAP; the term "BAP", as used herein, relates to a peptide with the capacity to bind to biotin or to an analog thereof (e.g. 2-iminobiotin, etc.); illustrative examples of BAP are mentioned in EP 711303 (incorporated in its entirety in this description as a reference) and includes peptides comprising the following amino acid sequence (SEQ ID NO: 2):

Leu xaa1 Xaa2 Ile Xaa3 Xaa4 Xaa5 Xaa6 Lys Xaa7 Xaa8 Xaa9 Xaa10
wherein Xaa1 is any amino acid; Xaa2 is an amino acid other than Leu, Val, Ile, Trp, Phe, or Tyr; Xaa3 is Phe or Leu; Xaa4 is Glu or Asp; Xaa5 is Ala, Gly, Ser, or Thr; Xaa6 is Gln or Met; Xaa7 is Ile, Met, or Val; Xaa8 is Glu, Leu, Val, Tyr, or Ile; Xaa9 is Trp, Tyr, Val, Phe, Leu, or Ile; and Xaa10 is any amino acid other than Asp or Glu.

In a specific embodiment, said peptide with the capacity to bind to a ligand is a BAP with 15 amino acids having the amino acid sequence shown in SEQ ID NO: 3.

In a particular embodiment, said peptide insert is inserted inside the amino acid sequence of said SAP. In this case, the invention provides a fusion protein (I) of the invention comprising, in addition to said SAP, a peptide insert (heterologous peptide) inserted inside the amino acid sequence of said SAP, said peptide insert comprising an amino acid sequence with the capacity to bind to a ligand (i.e. a specific binding domain). Said peptide insert is introduced in said SAP such that the assembly capacity of said SAP is not substantially altered; and said peptide insert must furthermore be located in a region of said self assembly protein accessible for the solvent in the macromolecular structures formed after the self-assembly of the fusion protein (I) of the invention.

In this particular embodiment, given that the peptide insert is inserted inside the amino acid sequence of the SAP, the fusion protein (I) of the invention maintains the amino and carboxyl terminal ends of said SAP and the peptide insert is included inside the amino acid sequence of said SAP, for example, between two contiguous amino acids of said SAP or, alternatively, substituting one or more amino acids of said SAP. By way of illustration, in a particular embodiment, said peptide insert is located between 2 contiguous amino acids of the SAP, i.e. between residue "x" and residue "x+1" of the amino acid sequence of said SAP; whereas in another particular embodiment, due to the strategy followed to introduce the peptide insert, one or more residues located in the regions adjacent to the insertion point may be lost. Furthermore, given that the peptide insert must be located in a region of said SAP accessible for the solvent in the macromolecular structures formed after the assembly of the fusion protein (I) of the invention, the peptide insert must be inserted in a suitable region. Particularly suitable regions of the SAP for introducing the peptide insert are the loops present in said self assembly proteins (SAPs), particularly those loops which, in the macromolecular structures that they form, are exposed to the solvent. Illustrative examples of loops present in SAPs which can be used to implement the present invention include the IBDV pVP2 loop P_{HI}, the human papilloma virus capsid protein loops (Bishop et al., 2007), the adeno-associated virus AAV5 loops (Walters et al., 2004), the *Galleria mellonella* densovirus (GMDNV) loops (Simpson et al., 1999), the *E*. *coli* GroES mobile loop, located between amino acids 15 and 34 of said protein, the T4 chaperonin Gp31 mobile loop, located between amino acids 22 and 45 of said protein, etc.

To introduce the peptide insert in the suitable site, i.e. in the suitable region and between the suitable amino acids, it is convenient to know the atomic structure of the SAP to be genetically modified. The atomic structure of a SAP can be determined by means of using X-ray diffraction. The use of suitable computer programs, such as for example the UCSF Chimera program (http://www.cgl.ucsf.edu/chimera/) allows locating the regions of the protein being studied which can potentially be genetically modified. These merely theoretical predictions are initially useful for: i) discarding regions that are not exposed to the solvent; and ii) identifying regions the modification of which could cause a serious alteration in the secondary structure of the protein. Once the atomic structure of the SAP is known, a strategy can be designed to incorporate the peptide insert in the suitable site, for example, in a loop of said SAP which is exposed to the solvent when said SAP self-assembles and forms the macromolecular structure.

In a particular embodiment, said SAP is IBDV pVP2, or a functional variant thereof, such as the protein identified as IBDV pVP2_452 (SEQ ID NO: 1). In this case, for the purpose of designing the strategy for introducing the peptide insert into the suitable region, an in-depth study was conducted of the already described atomic structure of said IBDV pVP2_452, (Garriga et al., 2006) (Protein Data Bank accession no. 2GSY), by means of using the UCSF Chimera computer program (http://www.cgl.ucsf.edu/chimera/). After this analysis, a strategy was designed to incorporate, in the region corresponding to loop P_{HI}, located in the region exposed to the solvent of said IBDV pVP2_452 in the macromolecular structures formed after its assembly (Figure 2). Therefore, in a specific embodiment, the SAP is IBDV pVP2, or a functional variant thereof, such as the protein identified as IBDV pVP2_452 (SEQ ID NO: 1), and said peptide insert is located inside loop P_{HI} of said IBDV pVP2 or functional variant thereof (IBDV pVP2_452) either between 2 contiguous amino acids or substituting one or more of the amino acids of said loop P_{HI}.

In another specific embodiment, the SAP is IBDV pVP2, or a functional variant thereof, such as the protein identified as IBDV pVP2_452 (SEQ ID NO: 1), and said peptide insert comprises the amino acid sequence of a BAP. In an even more specific embodiment, said BAP comprises the amino acid sequence shown in SEQ ID NO: 3.

In another specific embodiment, the SAP is IBDV pVP2, or a functional variant thereof, such as the Protein identified as IBDV pVP2_452 (SEQ ID NO: 1), said peptide insert comprises the amino acid sequence of a BAP and said peptide insert is located inside loop P_{HI} of said IBDV pVP2 or functional variant thereof (IBDV pVP2_452) either between 2 contiguous amino acids or substituting one or more amino acids of said loop P_{HI.} In an even more specific embodiment, said SAP comprises the amino acid sequence shown in SEQ ID NO: 3 and the insertion of said peptide insert in said SAP (IBDV pVP2, or a functional variant thereof, such as the protein identified as IBDV pVP2_452) is carried out between residues 319 and 321 of the amino acid sequence of said SAP, which involves eliminating residue 320 (Gln) [Figure 2].

Therefore, in a particular embodiment, the fusion protein (I) of the invention comprises a SAP and a peptide insert or heterologous peptide inserted inside the amino acid sequence of said SAP, said heterologous peptide comprising an amino acid sequence with the capacity to bind to a ligand, wherein said SAP is IBDV pVP2, or a functional variant thereof. In a specific embodiment, said functional variant of IBDV pVP2 is the protein identified as IBDV pVP2_452 (SEQ ID NO: 1). In another specific embodiment, said peptide insert comprises a BAP. In an even more specific embodiment, said BAP comprises the amino acid sequence shown in SEQ ID NO: 3.

In another particular embodiment, the fusion protein (I) of the invention comprises a SAP and a peptide insert or heterologous peptide inserted inside the amino acid sequence of said SAP, said heterologous peptide comprising an amino acid sequence with the capacity to bind to a ligand, wherein said peptide insert comprises a BAP. In a specific embodiment, said BAP comprises the amino acid sequence shown in SEQ ID NO: 3. In another even more specific embodiment, said SAP is IBDV pVP2, or a functional variant thereof, such as IBDV pVP2_452.

In another particular embodiment, the fusion protein (I) of the invention comprises a SAP and a peptide insert or heterologous peptide inserted inside the amino acid sequence of said SAP, said heterologous peptide comprising an amino acid sequence with the capacity to bind to a ligand, wherein said SAP is IBDV pVP2, or a functional variant thereof, in which said peptide insert is located inside loop P_{HI} of said IBDV pVP2 or functional variant thereof (IBDV pVP2_452), either between 2 contiguous amino acids or substituting one or more amino acids of said loop P_{HI}. In a specific embodiment, said peptide insert comprises a BAP; in an even more specific embodiment, said peptide insert comprises a SAP comprising the amino acid sequence shown in SEQ ID NO: 3 and the insertion of said peptide insert in said SAP (IBDV pVP2, or a functional variant thereof, such as the protein identified as IBDV pVP2_452 of) is located in loop P_{HI} of said SAP.

In another particular embodiment, the fusion protein (I) of the invention comprises a SAP and a heterologous peptide inserted inside the amino acid sequence of said SAP comprising an amino acid sequence with the capacity to bind to a ligand, in which said heterologous peptide comprises a BAP. In a specific embodiment, said BAP comprises the amino acid sequence shown in SEQ ID NO: 3. In another more particular embodiment, said SAP is an IBDV pVP2, or a functional variant thereof, such as IBDV pVP2_452. In an even more specific embodiment, said BAP is located inside loop P_{HI} of said IBDV pVP2 or functional variant thereof (IBDV pVP2_452) either between 2 contiguous amino acids or substituting one or more amino acids of said loop P_{HI}.

In another particular embodiment, said peptide insert is bound to the amino- or carboxyl-terminal end of said SAP. Therefore, in a particular embodiment, said peptide insert is bound to the amino-terminal end of said SAP, whereas, in another particular embodiment, said peptide insert is bound to the carboxyl-terminal end of said SAP. In another particular embodiment, the possibility is contemplated that the fusion protein (I) of the invention comprises two peptide inserts bound to a SAP, wherein each of said peptide inserts is bound to each of the amino- and carboxyl-terminal ends of said SAP.

In this case, the invention provides a fusion protein (I) of the invention comprising, in addition to said SAP, a peptide insert (heterologous peptide) bound to the amino- or carboxyl-terminal end of said SAP, or, alternatively, to both ends, said peptide insert comprising an amino acid sequence with the capacity to bind to a ligand. Said peptide insert is bound to said SAP such that the assembly capacity of said SAP is not substantially altered; and said peptide insert must furthermore be located in a region of said SAP accessible for the solvent in the macromolecular structures formed after the self assembly of the fusion protein (I) of the invention.

In a particular embodiment, the fusion protein (I) of the invention comprises a SAP and a heterologous peptide bound to the amino- or carboxyl-terminal end of said SAP comprising an amino acid sequence with the capacity to bind to a ligand, in which said heterologous peptide comprises a BAP. In a specific embodiment, said BAP comprises the amino acid sequence shown in SEQ ID NO: 3. In another more particular embodiment, said SAP is an IBDV pVP2, or a functional variant thereof, such as IBDV pVP2_452.

### 1.3 Production of the fusion protein (I) of the invention

The fusion protein (I) of the invention can be obtained by means of the gene expression of the nucleotide sequence encoding said fusion protein (I) in suitable host cells. Said suitable host cells are cells containing the nucleotide sequence encoding the fusion protein (I) of the invention, for example, cells containing a polynucleotide comprising the nucleotide sequence encoding the fusion protein (I) of the invention or which have been transformed by said polynucleotide, or cells transformed, transfected or infected with a recombinant vector comprising said polynucleotide comprising the nucleotide sequence encoding the fusion protein (I) of the invention. Said polynucleotide, the expression cassettes, the recombinant vectors and the suitable host cells for obtaining the fusion protein (I) of the invention are additional aspects of the present invention and are described in detail below.

Alternatively, said fusion protein (I) of the invention can be obtained by fusing the separately obtained members of said fusion protein.

Therefore, in another aspect, the invention relates to a polynucleotide, hereinafter polynucleotide of the invention, comprising the nucleotide sequence encoding the fusion protein (I) of the invention.

In another aspect, the invention provides an expression cassette, hereinafter expression cassette of the invention, comprising said polynucleotide of the invention operatively bound to transcription and optionally translation control elements. The transcription and optionally translation control elements present in the expression cassette of the invention include promoters, directing the transcription of the nucleotide sequence to which they are operatively linked (nucleotide sequence encoding the self assembly protein in which the nucleotide sequence encoding said peptide insert has been introduced), and other sequences necessary or suitable for the transcription and their suitable regulation in time and place, for example, start and stop signals, cleavage sites, polyadenylation signal, replication origin, transcriptional enhancers, transcriptional silencers, etc. Said elements, as well as the vectors used to construct the expression cassettes and the recombinant vectors according to the invention, are generally chosen depending on the host cells which are intended to be used.

In another aspect, the invention relates to a recombinant vector, hereinafter recombinant vector of the invention, comprising a polynucleotide of the invention or an expression cassette of the invention. Virtually any vector can be used in the generation of the recombinant vector of the invention. By way of illustration, said suitable expression systems or vectors can be selected according to the conditions and needs of each specific case from plasmids, bacmids, yeast artificial chromosomes (YACs), bacterial artificial chromosomes (BACs), phage-based artificial chromosomes (PACs), such as for example, phage P1-based PACs, cosmids, or viruses, which can further contain a heterologous replication origin, for example a bacterial or yeast replication origin so that it can be amplified in bacteria or yeasts, as well as a marker which can used to screen the transfected cells different from the gene or genes of interest. These recombinant vectors can be obtained by persons skilled in the art by means of using conventional genetic engineering techniques [Sambrook & Russell (2001)] and form part of the present invention.

In a particular embodiment, said recombinant vector is a plasmid, such as a suitable plasmid for transforming yeasts, or a virus, such as a recombinant baculovirus (rBV) expressing during its replication cycle the fusion protein (I) of the invention and which, after its assembly, forms said NCs.

In another aspect, the invention provides a host cell, hereinafter host cell of the invention, comprising the polynucleotide of the invention. In a particular embodiment, said host cell is a cell which can be or has been transformed by a polynucleotide of the invention. In another particular embodiment, said host cell is a cell which can be or has been transformed, transfected or infected with a recombinant vector of the invention comprising the polynucleotide of the invention. Said host cell can be a eukaryotic or prokaryotic cell. Virtually any host cell which can be transformed by a polynucleotide of the invention, or which can be transformed, transfected or infected by a recombinant vector of the invention, can be used in the present invention, for example, animal cells (e.g. mammalian cells, bird cells, insect cells, etc.), yeasts, etc.; nevertheless, in a particular embodiment, said host cell is selected from yeasts and insect cells. Yeasts are suitable for reasons of simplicity and production cost. Insect cells are suitable when the expression system comprises an rBV. The use of rBV is advantageous for biosafety issues related to the host range of baculoviruses, which cannot replicate in cell types other than insect cell types.

In a particular embodiment, the invention provides a host cell, such as a yeast, for example, a yeast of the *Saccharomyces* genus e.g. S. cerevisae, S. pombe, etc., or of the *Pichia* genus, e.g. *P. pastoris*, etc., transformed with a recombinant vector of the invention, such as a plasmid comprising the polynucleotide of the invention or an expression cassette of the invention.

In another particular embodiment, the invention provides a host cell, such as an insect cell, infected with a recombinant vector of the invention, such as an rBV comprising a polynucleotide of the invention or an expression cassette of the invention.

In another aspect, the invention provides a process for producing the fusion protein (I) of the invention which comprises culturing a host cell of the invention, containing the nucleotide sequence encoding the fusion protein (I) of the invention, under conditions allowing the expression of said fusion protein, and, if desired, the recovery of said fusion protein (I) of the invention. After the expression of the fusion protein (I) of the invention in said cells, the expressed proteins are assembled and form the nanocarriers (NCs). If desired, the fusion protein (I) of the invention, optionally assembled forming NCs, can be isolated or removed from the medium, and optionally purified. Said fusion protein (I) of the invention, optionally assembled forming NCs, can be isolated and purified by conventional methods, for example by means of fractionation in sucrose gradients.

In a particular embodiment, the gene expression of the fusion protein (I) of the invention is carried out by means of using an rBV comprising the polynucleotide of the invention, which allows expressing the fusion protein (I) of the invention from said polynucleotide of the invention in insect cells. Therefore, in a particular embodiment, the invention provides a process for producing the fusion protein (I) of the invention in insect cells which comprises (i) culturing insect cells infected with an rBV comprising the polynucleotide of the invention, under conditions allowing the expression of said fusion protein, and (ii) if desired, isolating and optionally purifying said fusion protein (I) of the invention, optionally assembled in the form of NCs. The implementation of said process involves previously constructing an rBV comprising the polynucleotide of the invention or an expression cassette of the invention and infecting insect cells with said rBV. An rBV comprising the polynucleotide of the invention can be constructed by a person skilled in the art based on that described herein and on the state of the art on this technology [Sambrook & Russell (2001); Leusch MS *et al*. (1995); Luckow VA *et al*. (1993)]. Example 1 (section 1.2) describes the result of expressing a fusion protein (I) of the invention, identified as SAP_BAP (SEQ ID NO: 4), comprising the self assembly protein IBDV pVP2_452 (SAP) genetically modified by means of introducing a BAP (SEQ ID NO: 3), in insect cells (*Tricoplusia Ni*) infected with an rBV containing a polynucleotide comprising the nucleotide sequence (SEQ ID NO: 5) encoding said IBDV pVP2_452 genetically modified by means of introducing the nucleotide sequence encoding said BAP.

In another particular embodiment, the gene expression of the fusion protein (I) of the invention is carried out in yeast cells. Therefore, in a particular embodiment, the invention provides a process for producing the fusion protein (I) of the invention in yeast cells which comprises (i) culturing yeasts transformed with a recombinant vector comprising the polynucleotide of the invention, under conditions allowing the expression of the fusion protein of the invention, and (ii) if desired, isolating and optionally purifying said fusion protein (I) of the invention, optionally assembled in the form of NCs. The implementation of said process involves previously constructing a suitable recombinant vector for transforming yeasts. Yeasts transformed with a recombinant vector comprising the polynucleotide of the invention can be obtained by a person skilled in the art based on that described herein and on the state of the art on this technology [pESC epitope tagging vectors Instructions manual. Stratagene www.stratagene.com; Sambrook & Russell (2001)].

The fusion protein (I) of the invention can self assemble and form nanocarriers that are generically called NC (singular) or NCs (plural) in this description, which are an additional aspect of this invention and will be described in detail below.

### 2. Conjugate of the invention

In another aspect, the invention relates to a conjugate, hereinafter conjugate of the invention, comprising (i) a region A comprising a ligand with the capacity to bind to said heterologous peptide comprising an amino acid sequence with the capacity to bind to a ligand, i.e. said peptide insert contained in said fusion protein (I) of the invention, bound to (ii) a region B comprising a product of interest.

### 2.1 Ligand

As used herein, the term "ligand" relates to a molecule which can interact specifically and bind to the peptide insert present in the fusion protein (I) of the invention, and by extension, in the NC resulting from the assembly of said fusion proteins (I) of the invention, which enables the product of interest bound to the ligand to bind to said nanocarrier. The ligand can be of a peptide (protein) or non-peptide nature.

As used herein, the expression "which can interact specifically" or "specific interaction" describes the interaction between a first species and a second species **characterized in that** the nature of binding is such that an antibody, a receptor or a nucleic acid binding protein binds to its corresponding binding pair but does not substantially bind to other species. Likewise, the term "binding" or "binding to", as used herein, means the physical association between a first species and a second species, for example, the binding of a ligand by a receptor, the binding of an antigen by an antibody, the binding of a nucleic acid by a nucleic acid binding protein, etc.

The term "ligand" does not involve any particular size or any other technical structural characteristic other than that said ligand can interact and bind to the peptide insert present in said fusion protein (I) of the invention; therefore, said ligand contains a region of interaction with the specific binding domain which is present in the peptide insert contained in the fusion protein (I) of the invention and, subsequently, in the NC resulting from the assembly of said fusion protein (I) of the invention, such that the product of interest present in the conjugate of the invention is bound to said NC by means of the specific interaction between the specific binding domain present in the NC (peptide insert) and the corresponding region of interaction with said specific binding domain present in the peptide insert (NC), contained in the ligand, which is present in the conjugate comprising the product of interest. By way of illustration, the "peptide insert"-"ligand" pair acts as a specific binding pair, e.g. of the biotin-avidin, epitope-antibody or functional fragment thereof (e.g. a Fab or F(ab')₂ fragment, an scFv (single chain antibody), a chimeric antibody, etc.) type, etc., in which each of said peptide insert and ligand are the members of said specific binding pair, each of them containing the corresponding binding domains or regions of interaction; thus, the ligand which must be present in the conjugate of the invention will be chosen depending on the peptide insert present in the fusion protein (I) of the invention, and, vice versa, the peptide insert which must be present in the fusion protein (I) of the invention will be chosen depending on the ligand present in the conjugate of the invention.

Said ligand can be bound to said peptide insert directly, i.e. by means of a specific direct interaction between the specific binding domain contained in the peptide insert present in the NC and the corresponding region of interaction with said binding domain present in the ligand contained in the conjugate of the invention; the product of interest thus binds to the NC through a structure of the type: peptide insert-ligand-product of interest.

Alternatively, said ligand can be bound to said peptide insert indirectly, i.e. by means of using a "connector" or molecule comprising, on one hand, a first region of interaction with the binding domain contained in the peptide insert present in the NC, and, on the other hand, a second region of interaction (binding domain) with a region of interaction present in the ligand contained in the conjugate of the invention. Therefore, in each case, the connector binds, on one hand, to the specific binding domain contained in the peptide insert present in the NC by means of a specific interaction and, on the other hand, to the product of interest by means of a specific interaction between the binding domains present in the connector and in the ligand contained in the conjugate of the invention. The product of interest thus binds to the nanocarrier through a structure of the type: peptide insert-connector-ligand-product of interest.

In a particular embodiment, the ligand is bound to the peptide insert directly. In this case, for its use in the present invention, a ligand with the capacity to bind to said peptide insert present in the fusion protein (I) of the invention will be used, such that said peptide insert and ligand forma specific binding pair. By way of a non-limiting illustration, when said peptide insert comprises an amino acid tag (e.g. a histidine tag (his-tag), an arginine tag (arg-tag), etc.); likewise when said peptide insert comprises a peptide epitope which can be recognized by an antibody (e.g. c-myc-tag, etc.), the ligand is an antibody or a functional fragment thereof; etc.

In another particular embodiment, the ligand is bound to the peptide insert indirectly by means of using a connector. In this case, for its use in the present invention, a dual connector will be used having on one hand, a first binding domain for binding to said peptide insert present in the fusion protein (I) of the invention, such that said peptide insert and connector form a first specific binding pair, and on the other hand, a second binding domain for binding to said ligand present in the conjugate of the invention, such that said connector and ligand form a second specific binding pair. Illustrative non-limiting examples of this embodiment include the use of biotin or an analog thereof (connector) when the peptide insert present in the NC comprises a BAP (in this case, the ligand to which the product of interest is bound would be an avidin); etc. As used herein, the term "avidin" includes any eukaryotic or prokaryotic avidin, and its variants maintaining the capacity to bind to biotin; in a particular embodiment, the ligand present in the conjugate of the invention comprises a monomeric avidin (mAV) variant.

### 2.2 Product of interest

The region B present in the conjugate of the invention comprises a product of interest. As used herein, the term "product of interest" includes any molecule with biological activity in its broadest sense, i.e., any substance which, when administered to a subject, interacts with its receptor in the action site and exerts a certain effect. The product of interest can be of a peptide (protein) or non-peptide nature, for example, a protein, an antibody or a functional fragment thereof, an enzyme, a lipid, a sugar, a non-peptide drug, a nucleic acid, etc.

Illustrative non-limiting examples of products of interest include any type of bioactive molecules such as synthetic molecules (e.g. acetylsalicylic acid, paracetamol, tranexamic acid, metoprolol, etc.), natural products (e.g. taxol, taxane derivatives, paclitaxel, epibatidine, atropine, etc.), oligonucleotides (e.g. augmerosen), aptamers (e.g. pegaptanib), proteins (e.g. interleukins, interferons, erythropoietins, coagulation factors, insulins, etc.), antibodies (e.g. erbitux, rituxan, herceptin, avastin, remicade, humira, xolair, etc.), peptides (e.g. calcitonins, vasopressin analogs (e.g. arginine vasopressin, desmopressin acetate, etc.), insulin polypeptides (e.g. humulin, insulin aspart, etc.), growth hormones (e.g. somatotropin, sermorelin, recombinant growth factor, etc.), gonadotropin polypeptides (e.g. urofollitropin, recombinant follicle stimulating hormone, etc.), gonadotropins (e.g. chorionic gonadotropins), thyrotropic hormone analogs, somatostatin analogs (e.g. octreotide, lanreotide acetate, etc.), parathyroid hormone polypeptides (e.g. teriparatide acetate, recombinant parathyroid hormone (1-84), etc.), alphaatrial natriuretic peptide, brain natriuretic peptide (e.g. nesiritide, etc.), thyroid hormone (e.g. thyrotropin, etc.), luteinizing hormone polypeptides (e.g. lutropin alpha, etc.), glucagon-like peptide-1 analogs (GLP1) (e.g. exenatide, etc.), corticotropin polypeptides (e.g. corticotropin, etc.), corticotropin release factor, gastrin analogs, peptides for radiation treatment (e.g. lanreotide, etc.), peptide vaccines (e.g. malaria, cancer, HIV, HBV, multiple sclerosis, etc.), hormones, enzymes, lipids, sugars, nucleic acids (DNA, RNA, PNA, etc.), e.g. oligonucleotides, polynucleotides, genes, interfering RNA, etc.

In a particular embodiment, said product of interest is a product of interest of a peptide nature; in this case, its size can vary within a wide range, from a few amino acids up to hundreds of amino acids. Said peptide product of interest can be virtually any peptide (as used herein, the term peptide includes peptides and proteins without distinction), regardless of its origin (eukaryotic, prokaryotic, viral, etc.), which can be recombinantly expressed, for example, a peptide antigen, such as a viral, bacterial, microbial, tumor antigen, etc., against which an immune response is to be induced in a subject (such as an animal, including human beings); an enzyme, such as an enzyme intended to supplement a function in which the organism is deficient; a peptide drug intended to prevent or treat a pathological condition in a subject; an antibody or a functional fragment thereof; etc. Therefore, in a particular embodiment, said peptide product of interest is a peptide useful in vaccination, therapy or diagnosis, such as an epitope or antigenic determinant which can induce an immune response in a subject (e.g. animals, including human beings) against diseases caused by viruses, bacteria, parasites or any other types of microorganisms, or against tumor diseases, or it is a peptide useful in the therapy (prevention and/or treatment) of other pathologies, for example, heparin, a cell growth factor, an interleukin, an interferon, a protein of interaction with a membrane receptor, a peptide of interaction with a specific cell marker, etc.; in a specific embodiment, said product of interest comprises the tissue factor, or a functional variant thereof, or yeast-derived microvesicles containing the tissue factor or a functional variant thereof; as is known, the lipidized tissue factor or a functional variant thereof, as well as said yeast-derived microvesicles containing the tissue factor or a functional variant thereof, are useful in the treatment of hemorrhages.

In another particular embodiment, said product of interest is a product of a non-peptide nature. Said product of interest of a non-peptide nature can be virtually any compound of a non-peptide nature, for example, a polynucleotide, a non-peptide antigen, such as allergens, viral, bacterial, microbial, tumor antigen, etc., against which an immune response is to be induced in a subject (such as an animal, including human beings); a non-peptide drug intended to prevent or treat a pathological condition in a subject; a lipid with the capacity to interact with a molecule of interest; a sugar with the capacity to interact with a molecule of interest; etc. Therefore, in a particular embodiment, said non-peptide product of interest is a compound useful in vaccination, therapy or diagnosis, such as a non-peptide antigenic determinant which can induce an immune response in a subject (e.g. animals, including human beings) against diseases caused by viruses, bacteria, parasites or any other type of microorganisms, or against tumor diseases, or it is a compound useful in the therapy (prevention and/or treatment) of other types of pathologies.

In a particular embodiment, said region B comprises a single product of interest. However, in another specific embodiment, particularly when said product of interest is a product of interest of a peptide nature, said region B can comprise two or more identical or different products of interest, which can form tandems.

In a particular embodiment, the conjugate of the invention comprises a region A bound to a single region B; nevertheless, in another specific embodiment, particularly when said product of interest is a product of interest of a peptide nature, the conjugate of the invention can comprise a region A bound to two regions B. According to this specific embodiment, the conjugate of the invention comprises a region A bound to two regions B, one of which is bound to the amino-terminal region of the ligand present in region A and the other of which is bound to the carboxyl-terminal region of the ligand present in region A. Said regions B can be identical or different and each of them can contain one or more products of interest, which can be identical or different from one another. In a specific embodiment, the conjugate of the invention comprises a region A bound to a first region B containing a first product of interest (B1) and a second region B containing a second product of interest (B2). Said products of interest (B1) and (B2) can be identical or different. In a specific embodiment, said products of interest (B1) and (B2) are different from one another.

### 2.3 Obtaining the conjugate of the invention

The conjugate of the invention can be obtained by conventional methods depending on the (peptide or non-peptide) nature of the ligand and of the product of interest, and, where appropriate, of the connector. Indeed, given that both the ligand and the product of interest, and where appropriate, the connector can be of a peptide or non-peptide nature, different possibilities arise.

In a particular embodiment, the ligand and the product of interest are of a non-peptide nature. In this case, the conjugate of the invention can be obtained by conventional chemical methods known by persons skilled in the art, which will be chosen depending on the nature of the functional groups present in said compounds (ligand and product of interest). It may occasionally be necessary to form derivatives of the product of interest (e.g. ethers, esters, etc.) that subsequently release the product of interest in the subject. Information about the chemical interactions between the functional groups usually present in the ligands and in the products of interest of a non-peptide nature can be found in March's Advanced Organic Chemistry, 5th Ed. (2001), Michael B. Smith & Jerry March, John Wiley & Sons, Inc.

In another particular embodiment, the ligand is of a peptide nature and the product of interest is of a non-peptide nature, or, alternatively, the ligand is of a non-peptide nature and the product of interest is of a peptide nature. In this case, like in the previous case, the conjugate of the invention can be obtained by conventional chemical methods known by persons skilled in the art, which will be chosen depending on the nature of the functional groups present in said compounds (ligand and product of interest). It may occasionally be necessary to form derivatives of the product of interest (e.g. ethers, esters, etc.) that subsequently release the product of interest in the subject. Information about the chemical interactions between the functional groups present in the ligands and in the products of interest of a peptide or non-peptide nature can be found in March's Advanced Organic Chemistry, 5th Ed. (2001), Michael B. Smith & Jerry March, John Wiley & Sons, Inc.

In another particular embodiment, the ligand and the product of interest are of a peptide nature. In this case, the conjugate of the invention is a fusion protein, hereinafter "fusion protein (II)" comprising a ligand of a peptide nature (region A') and a product of interest of a peptide nature (region B'). In this case, as mentioned previously, the product of interest (region B') can be bound to the amino-terminal end of said ligand (region A'), or, alternatively, said product of interest (region B') can be bound to the carboxyl-terminal end of said ligand (region A'). Likewise, said region A' can be directly bound to said region B', or, alternatively, said region A' is not directly bound to said region B' but rather it is bound through a linker peptide between said regions A' and B'. Therefore, if desired, said fusion protein can further contain a linker peptides located between said regions A' and B'; advantageously, said linker peptide is a peptide with structural flexibility. By way of illustration, said flexible peptide can contain repetitions of amino acid residues, particularly of Gly, Ser and Ala residues or any other suitable repetition of amino acid residues. In a particular embodiment, said region B' additionally comprises a single product of interest, whereas in another particular embodiment, said region B' comprises two or more identical or different products of interest, which can form tandems. Furthermore, said fusion protein (II) [particular case of the conjugate of the invention in which both the ligand and the product of interest are of a peptide nature] comprises, in a particular embodiment, a region A' bound to a single region B', or, alternatively, in another particular embodiment, it comprises a region A' bound to two regions B', as has been previously mentioned, one of which is bound to the amino-terminal region of the ligand present in region A' and the other of which is bound to the carboxyl-terminal region of the ligand present in region A'. Said regions B' can in turn be identical or different and each of them can contain one or more products of interest, which can be identical or different from one another.

Said fusion protein (II) can be obtained by means of the gene expression of the nucleotide sequence encoding said fusion protein (II) in suitable host cells. It can alternatively be obtained by means of fusing the members of said fusion protein (II) .

### 3. Nanocarriers (NCs) of the invention

As has been mentioned above, the fusion protein (I) of the invention can self assemble and form nanocarriers that are generically called NC (singular) or NCs (plural) in this description, which are an additional aspect of this invention.

Therefore, in another aspect, the invention relates to a nanocarrier (NC), hereinafter nanocarrier (or NC) of the invention, which can be obtained by the assembly of fusion proteins (I) of the invention, comprising: (i) a self assembly protein (SAP), and (ii) a peptide insert comprising the amino acid sequence of a peptide with the capacity to bind to a ligand, i.e. a specific binding domain.

In a particular embodiment, said peptide insert is inserted inside the amino acid sequence of said self assembly protein (SAP), whereas in another particular embodiment, said peptide insert is bound to the amino- or carboxyl-terminal end of said SAP.

As can be observed, the NC of the invention is a macromolecular structure resulting from the assembly of a certain number of fusion proteins (I) of the invention. The necessary number of units of fusion proteins (I) of the invention which must be assembled to form the NC of the invention depends on the self assembly protein present in the fusion protein of the invention; in a particular embodiment, the self assembly protein is the protein identified as IBDV pVP2_452 (SEQ ID NO: 1) giving rise to the formation of an NC with a structure with icosahedral symmetry (with a triangulation number T=1), formed by 60 monomers of said protein organized into 20 identical trimers, with a diameter of 22 nm and a free inner volume of approximately 500 Å³. The structure of the trimer is stabilized by the presence of a calcium atom interacting with 6 Asp residues from the 3 molecules forming it. The structure of said NC is stabilized by means of a wide range of non-covalent interactions established between adjacent trimers.

Therefore, in a particular embodiment, the invention provides an NC formed by the assembly of a fusion protein (I) of the invention comprising a SAP and a peptide insert or heterologous peptide inserted inside the amino acid sequence of said SAP, said heterologous peptide comprising an amino acid sequence with the capacity to bind to a ligand, wherein said SAP is IBDV pVP2, or a functional variant thereof. In a particular embodiment, said functional variant of IBDV pVP2 is the protein identified as IBDV pVP2_452 (SEQ ID NO: 1). In another specific embodiment, said peptide insert comprises a BAP. In an even more specific embodiment, said BAP comprises the amino acid sequence shown in SEQ ID NO: 3.

In another particular embodiment, the invention provides an NC formed by the assembly of a fusion protein (I) of the invention comprising a SAP and a peptide insert or heterologous peptide inserted inside the amino acid sequence of said SAP, said heterologous peptide comprising an amino acid sequence with the capacity to bind to a ligand, wherein said peptide insert comprises a BAP. In a specific embodiment, said BAP comprises the amino acid sequence shown in SEQ ID NO: 3. In another even more specific embodiment, said SAP is IBDV pVP2, or a functional variant thereof, such as IBDV pVP2_452.

In another particular embodiment, the invention provides an NC formed by the assembly of a fusion protein (I) of the invention comprising a SAP and a peptide insert or heterologous peptide inserted inside the amino acid sequence of said SAP, said heterologous peptide comprising an amino acid sequence with the capacity to bind to a ligand, wherein said SAP is IBDV pVP2, or a functional variant thereof, in which said peptide insert is located inside loop P_{HI} of said IBDV pVP2 or functional variant thereof (IBDV pVP2_452), either between 2 contiguous amino acids or substituting one or more amino acids of said loop P_{HI}. In a specific embodiment, said peptide insert comprises a BAP; in an even more specific embodiment, said peptide insert comprises a BAP comprising the amino acid sequence shown in SEQ ID NO: 3 and the insertion of said peptide insert in said SAP (IBDV pVP2, or a functional variant thereof, such as the protein identified as IBDV pVP2_452) is located in loop P_{HI} of said SAP.

In another particular embodiment, the invention provides an NC formed by the assembly of a fusion protein (I) of the invention comprising a SAP and a peptide insert or heterologous peptide inserted inside the amino acid sequence of said SAP comprising an amino acid sequence with the capacity to bind to a ligand, in which said heterologous peptide comprises a BAP. In a specific embodiment, said BAP comprises the amino acid sequence shown in SEQ ID NO: 3. In another more particular embodiment, said SAP is an IBDV pVP2, or a functional variant thereof, such as IBDV pVP2_452. In an even more specific embodiment, said BAP is located inside loop P_{HI} of said IBDV pVP2 or functional variant thereof (IBDV pVP2_452), either between 2 contiguous amino acids or substituting one or more amino acids of said loop P_{HI}.

In another particular embodiment, the invention provides an NC formed by the assembly of a fusion protein (I) of the invention, identified as SAP_BAP, comprising the IBDV pVP2_452 and a BAP (SEQ ID NO: 3) located inside loop P_{HI} of said IBDV pVP2_452.

In another particular embodiment, the invention provides an NC formed by the assembly of a fusion protein (I) of the invention comprising a SAP and a peptide insert or heterologous peptide bound to the amino- or carboxyl-terminal end of said SAP, in which said heterologous peptide comprises a BAP. In a specific embodiment, said BAP comprises the amino acid sequence shown in SEQ ID NO: 3. In another more particular embodiment, said SAP is an IBDV pVP2, or a functional variant thereof, such as IBDV pVP2_452.

The NC of the invention may or may not contain a product of interest (which is bound to a ligand forming the conjugate of the invention) bound to the peptide insert present in said NC through a ligand with the capacity to bind to said peptide insert, or alternatively, through a dual connector, i.e. with two different binding domains, one of which has the capacity to bind to said peptide insert and the other of which has the capacity to bind to said ligand to which the product of interest is bound.

Said particular embodiments will be described in detail below.

### 3.1 Unloaded NC of the invention

In a particular embodiment, the NC of the invention is not bound to any product of interest and has been called "unloaded NC of the invention", to emphasize that it is not bound to any product of interest, opposed to another particular embodiment of the NC of the invention comprising a product of interest and which has been called "loaded NC of the invention".

In this particular embodiment, which is the simplest configuration of the NC of the invention, the unloaded NC of the invention is a macromolecular structure resulting from the assembly of fusion proteins (I) of the invention, said fusion proteins (I) of the invention comprising: (i) a self assembly protein and (ii) a peptide insert comprising the amino acid sequence of a peptide with the capacity to bind to a ligand, i.e. a specific binding domain. As has been mentioned previously, said peptide insert can be inserted inside the amino acid sequence of said self assembly protein (SAP) or bound to the amino- or carboxyl-terminal end of said SAP. Figure 1 shows a schematic depiction of an unloaded NC of the invention [see the drawing identified as (2) in said Figure 1, showing an NC with several specific binding domains on its surface resulting from the assembly of the genetically modified self assembly protein identified as (1) in said Figure 1].

In a particular embodiment, the unloaded NC of the invention comprises a connector bound to the peptide insert. In a specific embodiment, said peptide insert is a BAP and said connector is a biotin which allows the binding of a conjugate of the invention the ligand of which is an avidin, for example, a monoavidin (mAV). In an even more specific embodiment, said BAP has the amino acid sequence shown in SEQ ID NO: 3. Several processes are described below for producing this specific type of unloaded NC of the invention comprising a connector bound to the peptide insert, specifically, in which said connector is a biotin.

The technical characteristics of said fusion protein (I) of the invention, as well as of its components (self assembly protein and peptide insert), and, where appropriate, of the connector, have already been defined above.

The unloaded NC of the invention can be obtained by conventional techniques known by persons skilled in the art, for example, by means of the gene expression of the nucleotide sequence encoding said fusion protein (I) of the invention in suitable host cells, such as the previously defined host cells of the invention containing the polynucleotide encoding the amino acid sequence of the fusion protein (I) of the invention, and the subsequent assembly of said fusion proteins (I) of the invention.

Therefore, in another aspect, the invention relates to a process for producing an unloaded NC of the invention which comprises culturing a host cell of the invention (comprising the polynucleotide of the invention containing the nucleotide sequence encoding the fusion protein (I) of the invention) under conditions allowing the expression of said fusion protein and its self assembly to form NCs, and, if desired, the recovery of said NCs. After the expression of the fusion protein (I) of the invention in said cells, the expressed proteins are generally assembled and form the NCs which, if desired, can be isolated or removed from the medium, and optionally purified. Said NCs can be isolated and purified by conventional methods, for example by fractionation in sucrose gradients.

In the event that the unloaded NC of the invention contained a connector bound to the peptide insert, the previously described process for obtaining it would include the step of putting the NCs formed in contact with the connector. In a particular embodiment, said connector is biotin and in this case, said unloaded NC of the invention comprising biotin bound to BAP (peptide insert) can be obtained by putting said NCs in which the peptide insert is a BAP in contact with biotin in the presence of biotin ligase (BL) enzyme under conditions allowing the specific interaction (binding) of biotin to SAP. Said interaction can be achieved metabolically or non-metabolically, i.e. in vitro, as will be described in detail below.

In a particular embodiment, the production of said unloaded NC of the invention is carried out in insect cells infected with an rBV comprising the polynucleotide of the invention. Example 1 (section 1.3) describes obtaining unloaded NCs of the invention formed by the self assembly of a fusion protein (I) of the invention identified as SAP_BAP (SEQ ID NO: 4) comprising the self assembly protein IBDV pVP2_452 (identified as "SAP") genetically modified by means of introducing a BAP (SEQ ID NO: 3), in insect cells (*T. Ni*) infected with an rBV comprising a polynucleotide comprising the nucleotide sequence encoding said IBDV pVP2_452 genetically modified by means of introducing the nucleotide sequence encoding said BAP in loop P_{HI} of IBDV pVP2-452.

In another particular embodiment, the production of said unloaded NC of the invention is carried out in yeasts comprising the polynucleotide of the invention; in this case, the polynucleotide of the invention can be contained in a recombinant vector integrated in the yeast genome, or, preferably, said recombinant vector not integrated in the yeast genome.

The unloaded NC of the invention can be used, among other applications, in the production of a loaded NC of the invention.

### 3.2Loaded NC of the invention

In a particular embodiment, the NC of the invention is bound to a product of interest and has been called "loaded NC of the invention".

In this particular embodiment, the loaded NC of the invention is a macromolecular structure resulting from the assembly of fusion proteins (I) of the invention, further comprising a product of interest, in which said fusion proteins (I) of the invention comprise: (i) a self assembly protein and (ii) a peptide insert comprising the amino acid sequence of a peptide with the capacity to bind to a ligand, i.e. a specific binding domain. As has been mentioned previously, said peptide insert can be inserted inside the amino acid sequence of said self assembly protein (SAP) or bound to the amino- or carboxyl-terminal end of said SAP. Figure 1 shows a schematic depiction of a particular case of a loaded NC of the invention [see the drawing identified as (4) in said Figure 1, showing an NC with several specific binding domains on its surface (resulting from the self assembly of the genetically modified self assembly protein identified as (1) in said Figure 1), to which chimeric proteins (3) have been bound [particular case of the conjugate of the invention], which chimeric proteins comprise (i) a ligand containing a region of interaction with the binding domain present in the NC bound to (ii) a protein of interest].

The product of interest forms part of a conjugate of the invention comprising, in addition to the product of interest, a ligand. In a particular embodiment, said ligand has the capacity to bind to said peptide insert present in the NC, i.e. it contains a region of specific interaction with the specific binding domain present in said NC (peptide insert); in this case, the specific interaction (binding) between the ligand and the peptide insert is carried out directly. Alternatively, the binding of the ligand to the peptide insert can be carried out indirectly, through a connector; as has been defined previously, said connector is a dual molecule, i.e. it contains two different specific binding domains (or two regions of specific interaction) to allow on one hand, its specific interaction (binding) with the peptide insert (NC) and, on the other hand, its specific interaction (binding) with the ligand (conjugate of the invention). In this case, the ligand present in the conjugate of the invention has the capacity to bind to said connector, i..e. it contains a region interacting specifically with the corresponding specific binding domain present in said connector.

The technical characteristics of said fusion protein (I) of the invention, as well as those of its components (self assembly protein and peptide insert), those of the conjugate of the invention and its components (ligand and product of interest) and those of the connector have already been defined above.

According to the manner (directly or indirectly) whereby the product of interest can be bound to the NCs of the invention, the loaded NCs of the invention are classified into:
a) loaded NCs of the invention in which the product of interest is bound directly to the NC through the ligand;
   and
b) loaded NCs of the invention in which the product of interest is bound indirectly to the NC through a connector.

The product of interest, regardless of whether or not it is bound to the NC through a ligand or a connector, can alternatively be contained inside the NC of the invention, thus giving rise to an alternative embodiment of the loaded NC of the invention.

A loaded NC of the invention comprising a product of interest bound directly to the NC through the ligand, (or indirectly through a connector which is bound to the NC on one hand and to the conjugate of the invention on the other hand) can generally be obtained by incubating an unloaded NC of the invention in the presence of the conjugate of the invention and, where appropriate, of the connector, under suitable conditions allowing the binding between the binding domains present in said unloaded NC of the invention and the regions of interaction present in the ligand contained in the conjugate of the invention, alternatively, between the binding domains present in said unloaded NC of the invention and the regions of interaction present in the connector and between the binding domains present in said connector and the regions of interaction present in the ligand contained in the conjugate of the invention. The unloaded NC of the invention can be obtained according to the previously described methodology and the conjugate of the invention can be obtained by synthetic or recombinant methods, depending on the nature of its components, as has been mentioned previously.

Said particular embodiments are described below.

### 3.2.1 Loaded NC of the invention in which the product of interest is bound directly to the NC through the ligand

In a specific embodiment, the loaded NC of the invention comprises a product of interest bound directly to the NC through the ligand by means of a typical interaction of a specific binding pair, as has been mentioned previously in the section relating to the conjugate of the invention.

In this case, the loaded NC of the invention comprising a product of interest bound directly to NC through the ligand can be obtained by several conventional methods depending, among other issues, on the (peptide or non-peptide) nature of the ligand and of the product of interest.

By way of illustration, the loaded NC of the invention comprising a product of interest bound directly to the NC through the ligand can be obtained by incubating an unloaded NC of the invention in the presence of the conjugate of the invention under suitable conditions allowing the binding between the binding domains present in said unloaded NC of the invention and the regions of interaction present in the ligand contained in the conjugate of the invention. Said loaded NC of the invention comprising a product of interest bound directly to NC through the ligand, depending on the nature of the conjugate of the invention, can alternatively be obtained by means of expressing both the fusion protein (I) of the invention and,
where appropriate, the conjugate of the invention in a suitable expression system.

Therefore, in a particular embodiment, the invention provides a process (Process A.1) for product a loaded NC of the invention comprising a product of interest bound directly to the NC through the ligand which comprises putting an unloaded NC of the invention in contact with a conjugate of the invention comprising the ligand and the product of interest, under conditions allowing the binding between the specific binding domains present in the peptide inserts contained in said unloaded NC of the invention and the corresponding regions of interaction present in the ligands contained in said conjugates of the invention. As a result of said specific interaction between the specific binding domains present in said unloaded NC of the invention and the corresponding regions of interaction present in the ligands, the product of interest is bound to the NC by means of said ligands, a loaded NC of the invention comprising a product of interest bound directly to the NC through the ligand thus being generated.

As a result of the strategy followed in this Process A.1, it is possible that part of the product of interest (in the form of conjugate of the invention) is included inside the NC formed by the assembly of the fusion protein (I) of the invention.

This process (Process A.1) can be used to produce a loaded NC of the invention comprising a product of interest bound directly to NC through the ligand, either when the ligand and/or the product of interest are of a non-peptide nature or when the ligand and the product of interest are of a peptide nature.

Figure 1 schematically shows this process (Process A.1) for obtaining a loaded NC of the invention comprising a product of interest bound directly to the NC through the ligand. Said diagram shows in a simplified manner a strategy designed to obtain a loaded NC of the invention in which the product of interest bound directly to the NC through a ligand is a recombinant protein (particular case of the conjugate of the invention), briefly consisting of: expressing a self assembly protein genetically modified by means of the introducing a peptide insert (1) to allow the presence of a specific binding domain in an exposed region of the protein assembly or NC (2). In addition, recombinant vectors are developed for expressing a conjugate of the invention, which in this case is a fusion protein (3) comprising a region A' comprising a region of interaction with the specific binding domain exposed in said NC and a region B' comprising a product of interest of a protein nature. Finally, the specific interaction between the specific binding domains present in the NC and the corresponding region of interaction (ligand) present in said fusion protein comprising said regions A' and B' leads to the formation of stable complexes (4) where said fusion proteins coat the surface of the NC (loaded NC of the invention).

Alternatively, in another particular embodiment, in which the conjugate of the invention is of a peptide nature (due to the fact that both the ligand and the product of interest are of a peptide nature), i.e. when said conjugate of the invention is the fusion protein previously identified as "fusion protein (II)", the loaded NC of the invention comprising a product of interest bound directly to the NC through the ligand can be obtained by means of an alternative process comprising the joint expression (co-expression) of said fusion protein (I) of the invention and of said fusion protein (II), in one and the same expression system, under conditions allowing, on one hand, the expression of said fusion protein (I) of the invention and its assembly to form "'unloaded" NCs and on the other hand, the expression of said fusion protein (II), and furthermore the specific interaction (binding) between the specific binding domains present in the peptide inserts contained in said "unloaded" NCs and the corresponding regions of interaction present in the protein ligands contained in said fusion protein (II) [particular case of the conjugate of the invention comprising a ligand and a product of interest of a peptide nature]. Said specific interaction between the specific binding domains present in said unloaded NC of the invention and the corresponding regions of interaction present in the ligands is a typical interaction of a specific binding pair, for example, of an antigen-antibody type, etc.

Therefore, in another particular embodiment, in which the conjugate of the invention is the fusion protein (II), the invention provides a process (Process A.2) for producing a loaded NC of the invention comprising a product of interest bound directly to the NC through the ligand which comprises culturing a host cell of the invention comprising, in addition to the polynucleotide of the invention (comprising the nucleotide sequence encoding the fusion protein (I) of the invention), a polynucleotide, hereinafter "polynucleotide PF(II)" comprising the nucleotide sequence encoding said fusion protein (II), under conditions allowing the expression of said fusion protein (I) of the invention and its assembly to form "unloaded" NCs, the expression of said fusion protein (II), and furthermore the specific interaction (binding) between the specific binding domains present in said "unloaded" NCs and the corresponding regions of interaction present in the protein ligands present in said fusion protein (II), and, if desired, the recovery of said loaded NCs. After the expression of the fusion protein (I) of the invention in said cells, the expressed proteins are generally assembled and form the "unloaded" NCs, and the contact of said unloaded NCs with the fusion protein (II), under the aforementioned conditions, causes the binding of the product of interest to the NC by means of the ligand, a loaded NC of the invention comprising a product of interest bound directly to the NC through the ligand thus being generated.

The modification of a host cell of the invention so that it comprises, in addition to the polynucleotide of the invention, the nucleotide sequence encoding the fusion protein (II), can be carried out by means of conventional methods known by persons skilled in the art [Sambrook & Russell (2001)].

As a result of the strategy followed in this Process A.2, it is possible that part of the product of interest (in the form of conjugate of the invention) is included inside the NC formed by the assembly of the fusion protein (I) of the invention.

In a particular embodiment, the gene expression of the fusion protein (I) of the invention and the gene expression of said fusion protein (II) is carried in insect cells infected with an rBV comprising the polynucleotide of the invention and the polynucleotide identified as polynucleotide PF(II) [comprising the nucleotide sequence encoding said fusion protein (II)]. Said polynucleotides can form part of one and the same nucleic acid molecule or gene construct or they can alternatively be separated into two different constructs but contained in one and the same rBV. According to this embodiment, a single rBV comprising the polynucleotide of the invention and the polynucleotide PF(II) is constructed either in a single gene construct or, alternatively, in two separate identical or different gene constructs, and an insect cell is subsequently co-infeoted with said rBV.

In another particular embodiment, the gene expression of the fusion protein (I) of the invention and that of said fusion protein (II) is carried out in insect cells co-infected with 2 different rBVs, each of them containing one of said polynucleotides. According to this embodiment, an rBV comprising the polynucleotide of the invention and another rBV comprising the polynucleotide PF(II) are constructed, and an insect cell is subsequently co-infected with said rBVs.

Some of said gene constructs comprising the polynucleotide of the invention and the polynucleotide PF(II) form part of the present invention and are additional aspects thereof. Said gene constructs can be obtained by conventional methods known by persons skilled in the art in view of the fusion protein (I) of the invention and of the fusion protein (II). Likewise, the expression cassettes, the recombinant vectors and the host cells containing said gene constructs comprising the polynucleotide of the invention and the polynucleotide PF(II) are additional aspects of the present invention and can be obtained by conventional methods known by persons skilled in the art in view of the information contained in this description [Sambrook & Russell (2001)].

Likewise, the rBVs comprising said gene constructs comprising the polynucleotide of the invention and the polynucleotide PF(II) in a single gene construct, or comprising said polynucleotides (polynucleotide of the invention and polynucleotide PF(II)) in separate constructs, form part of the present invention, are additional aspects thereof and can be obtained by a person skilled in the art based on that described herein and on the state of the art on this technology, as has been mentioned previously in relation to the production of the fusion protein (I) of the invention.

In another particular embodiment, the gene expression of the fusion protein (I) of the invention and the gene expression of said fusion protein (II) are carried out in yeasts comprising the polynucleotide of the invention and the polynucleotide PF(II). Said polynucleotides can be integrated inside the yeast genome, or one of them can be integrated in the yeast genome and the other one not, which polynucleotide can be in a gene construct in the cytoplasm, or both polynucleotides can form part of one and the same nucleic acid molecule or gene construct not integrated in the yeast genome, or, alternatively, said polynucleotides can be separated into two different constructs but contained in one and the same yeast cell. According to this embodiment, a single recombinant yeast is produced comprising the polynucleotide of the invention and the polynucleotide PF(II), either integrated inside the yeast genome, or one of them integrated in the yeast genome and the other one not, or in a single gene construct not integrated in the yeast genome, or in two different gene constructs not integrated in the yeast genome but contained in one and the same yeast cell, and said yeast is then cultured under conditions allowing the expression of said fusion protein (I) of the invention and its assembly to form "unloaded" NCs, the expression of said fusion protein (II), and furthermore the specific interaction (binding) between the specific binding domains present in said "unloaded" NCs and the corresponding regions of interaction present in the protein ligands present in the fusion protein (II), and, (ii) if desired, the isolation and optionally the purification of said NCs "loaded" with the product of interest.

The recombinant yeasts comprising the polynucleotide of the invention and the polynucleotide PF(II), either integrated inside the yeast genome, or one of them integrated in the yeast genome and other one not, or in a single gene construct not integrated in the yeast genome, or in two different gene constructs not integrated in the yeast genome but contained in one and the same yeast cell, as well as the gene constructs and recombinant vectors necessary for obtaining said recombinant yeasts, form part of the present invention, are additional aspects thereof and can be obtained by a person skilled in the art based on that described herein and on the state of the art on this technology, as has been mentioned previously in relation to the production of the fusion protein (I) of the invention.

### 3.2.2 Loaded NC of the invention in which the product of interest is bound indirectly to the NC through the connector

In a specific embodiment, the loaded NC of the invention comprises a product of interest bound indirectly to the NC through a connector which is bound, on one hand, to the peptide insert present in the NC and, on the other hand, to the ligand present in the conjugate of the invention, by means of typical interactions of respective specific binding pairs, as has been mentioned previously in the section relating to the conjugate of the invention. In a particular embodiment, said connector is biotin which is bound to the NC by means of a specific interaction with a specific biotin binding domain contained in the peptide insert, such as a BAP and said ligand comprises a domain of specific biotin interaction, such as an avidin; the product of interest bound to the ligand is thus bound indirectly to the NC through said connector. In another particular embodiment, said connector is a fusion protein comprising an SH3 domain which binds to the NC by means of a specific interaction with a protein SH3 binding domain contained in the peptide insert. In another particular embodiment, said connector is a peptide comprising a domain of specific interaction with a protein involved in a signaling cascade. In another particular embodiment, said connector is a peptide comprising a domain of specific interaction with a post-translational modification sequence (e.g. myristoylation, methylation, phosphorylation, etc.). In another particular embodiment, said connector is a peptide comprising a domain of specific interaction with a sequence of transport to a cell compartment.

The loaded NC of the invention comprising a product of interest bound indirectly to the NC through a connector can be obtained by several conventional methods depending, among other issues, on the (peptide or non-peptide) nature of the ligand and on the product of interest, as well as on the connector, in a manner similar to how it has been described previously in relation to the production of loaded NCs of the invention comprising a product of interest bound directly to the NC through the ligand.

By way of illustration, the loaded NC of the invention comprising a product of interest bound indirectly to the NC through a connector can be obtained by incubating an unloaded NC of the invention with the conjugate of the invention in the presence of said connector under suitable conditions allowing the specific interaction (binding) between the specific binding domains present in said unloaded NC of the invention and the corresponding regions of interaction present in the connector, and, subsequently the specific interaction (binding) between the binding domains present in said connector bound to the NC and the regions of interaction present in the ligand contained in the conjugate of the invention. Although it is possible to jointly (simultaneously) incubate the unloaded NC of the invention with the connector and with the conjugate of the invention in a single (one-pot) step, in practice it may be advantageous to carry out the incubations in two steps, first incubating the unloaded NC of the invention with the connector, under conditions allowing the formation of an unloaded NC of the invention bound to the connector, and then incubating said unloaded NC of the invention bound to the connector with the conjugate of the invention. Said specific interaction between the binding domains present in said unloaded NC of the invention and the corresponding regions of interaction present in the connector, as well as said specific interaction between the binding domains present in said connector bound to the NC and the regions of interaction present in the ligand (conjugate of the invention) are typical interactions of specific binding pairs, for example, of the BAP-biotin and biotin-avidin type, etc.

Said loaded NC of the invention comprising a product of interest bound indirectly to the NC through the ligand connector, depending on the nature of the conjugate of the invention, can alternatively be obtained by means of expressing both the fusion protein (I) of the invention and where appropriate, the conjugate of the invention in a suitable expression system.

Therefore, in a particular embodiment, the invention provides a process (Process B.1) for producing a loaded NC of the invention comprising a product of interest bound indirectly to the NC through a connector which comprises:
a) putting an unloaded NC of the invention in contact with a connector, under conditions allowing the specific interaction (binding) between the binding domain present in said unloaded NC of the invention and the region of interaction corresponding to said binding domain present in the connector, to obtain an unloaded NC of the invention bound to said connector;
b) putting said unloaded NC of the invention bound to a connector in contact with a conjugate of the invention comprising a ligand and a product of interest, under conditions allowing the binding between the binding domain present in said connector and the corresponding region of interaction present in said ligand, to obtain a loaded NC of the invention comprising a product of interest bound indirectly to the NC through a connector;
   and, if desired
c) isolating and optionally purifying said loaded NC of the invention comprising a product of interest bound indirectly to the NC through a connector.

Alternatively, in a variant of said process B.1, the connector first binds to the conjugate of the invention and the resulting complex binds to the unloaded NC of the invention; thus, in a variant of said Process B.1), the invention provides a process for producing a loaded NC of the invention comprising a product of interest bound indirectly to the NC through a connector which comprises:
a) putting said connector in contact with a conjugate of the invention comprising a ligand and a product of interest, under conditions allowing the binding between the binding domain present in said connector and the corresponding region of interaction present in said ligand, to obtain a connector-conjugate complex of the invention;
b) putting an unloaded NC of the invention in contact with said connector-conjugate complex of the invention, under conditions allowing the specific interaction (binding) between the binding domain present in said unloaded NC of the invention and the region of interaction corresponding to said binding domain present in the connector, to obtain a loaded NC of the invention comprising a product of interest bound indirectly to the NC through said connector; and, if desired
c) isolating and optionally purifying said loaded NC of the invention comprising a product of interest bound indirectly to the NC through a connector.

Due to said specific interaction between the binding domain present in said unloaded NC of the invention and the region of interaction corresponding to said binding domain present in the connector, and subsequently to the specific interaction between the binding domain present in said connector bound to said unloaded NC of the invention and the corresponding region of interaction present in said ligand, the product of interest binds indirectly to the NC through the connector bound to the ligand, a loaded NC of the invention comprising a product of interest bound indirectly to the NC through the ligand thus being generated.

This process (Process B.1), in any of its 2 variants, can be used to produce a loaded NC of the invention comprising a product of interest bound indirectly to the NC through a connector when the connector and/or the ligand and/or the product of interest are of a non-peptide nature. Alternatively, if desired, this process can also be used when the connector, the ligand and the product of interest are of a peptide nature.

In a particular embodiment, said connector is biotin or an analog thereof, the peptide insert comprises a BAP and the ligand present in the conjugate of the invention comprises an avidin, preferably, a monomeric avidin (mAV) variant.

Example 1 (section 1.4) describes the results of a process for biotinylating NCs *in vitro* based on this process which comprises incubating (i) unloaded NCs of the invention formed by the self assembly of a fusion protein (I) of the invention, identified as SAP_BAP (SEQ ID NO: 4) comprising the self assembly protein IBDV pVP2_452 ("SAP") genetically modified by means of introducing a BAP (SEQ ID NO: 3), in insect cells (*T. Ni*) infected with an rBV comprising a polynucleotides comprising the nucleotide sequence encoding said IBDV pVP2_452 genetically modified by means of introducing the nucleotide sequence encoding said BAP (rBV/FB_SAP_BAP), with (ii) biotin, in the presence of (iii) BirA (BL or biotin ligase enzyme). Example 3, describing the production of NCs loaded with a recombinant protein through a system (BAP-biotin-mAv) based on using biotin as a connector, is also based on this process.

Said loaded NC of the invention comprising a product of interest bound indirectly to the NC through the connector, depending on the nature of the connector and of the conjugate of the invention, can alternatively be obtained by means of expressing both the fusion protein (I) of the invention and,
where appropriate, the connector and the conjugate of the invention in a suitable expression system. This alternative is also valid when the connector is a metabolite present in a native or recombinant manner in the expression system used to produce said NC, for example, biotin, a cofactor which is present in a native manner in multiple cells.

Therefore, in another particular embodiment, in which the conjugate of the invention is the fusion protein (II), and the connector is a peptide, the invention provides a process (Process B.2) for producing a loaded NC of the invention comprising a product of interest bound indirectly to the NC through a connector, which comprises culturing a host cell of the invention comprising, in addition to the polynucleotide of the invention, the polynucleotide PF(II) and a polynucleotide, hereinafter "polynucleotide-connector", comprising the nucleotide sequence encoding said connector, under conditions allowing the expression of said fusion protein (I) of the invention and its assembly to form "unloaded" NCs, the expression of said connector, the expression of said fusion protein (II), and furthermore the specific interaction (binding) between the specific binding domains present in said "unloaded" NCs and the corresponding regions of interaction present in said connector, and, subsequently, the specific interaction (binding) between the specific binding domains present in said connector and the corresponding regions of interaction present in the protein ligands present in said fusion protein (II), and, if desired, the recovery of said loaded NCs. After the expression of the fusion protein (I) of the invention in said cells, the expressed proteins are generally assembled and form the "unloaded" NCs, and the contact of said unloaded NCs with the connector and subsequently with the fusion protein (II), under the aforementioned conditions, causes the binding of the product of interest to the NC by means of the connector, through a connector-ligand system, a loaded NC of the invention comprising a product of interest bound indirectly to the NC through the ligand thus being generated.

The person skilled in the art will understand that in another variant of this process, the connector can first be fused to the fusion protein (II) of the invention and the resulting complex can interact with the unloaded NC of the invention to produce the loaded NC of the invention comprising a product of interest bound indirectly to the NC through a connector.

The modification of a host cell of the invention so that it comprises, in addition to the polynucleotide of the invention, the polynucleotide PF(II) and the polynucleotide-connector, can be carried out by means of conventional methods known by the persons skilled in the art [Sambrook & Russell (2001)].

As a result of the strategy followed in this Process B.2, in any of its variants, it is possible that part of the product of interest (in the form of conjugate of the invention) is included inside the NC formed by the assembly of the fusion protein (I) of the invention.

In a particular embodiment, the gene expression of the fusion protein (I) of the invention and the gene expression of said fusion protein (II) is carried out in insect cells infected with an rBV comprising the polynucleotide of the invention, the polynucleotide PF(II) and the polynucleotide-connector [comprising the nucleotide sequence encoding said connector]. Said polynucleotides can form part of one and the same nucleic acid molecule or gene construct, or, alternatively, they can be separated into 2 (containing any combination of 2 of said polynucleotides) or 3 different constructs but contained in one and the same rBV. According to this embodiment, a single rBV is constructed comprising the polynucleotide of the invention, the polynucleotide PF(II), the polynucleotide-connector, either in a single gene construct or, alternatively, in 2 or 3 separate gene constructs, and an insect cell is subsequently co-infected with said rBV.

In another particular embodiment, the gene expression of the fusion protein (I) of the invention and that of said fusion protein (II) is carried out in insect cells co-infected with 2 different rBVs, one of which rBVs contains any two of said polynucleotides (polynucleotide of the invention, polynucleotide PF(II) and polynucleotide-connector) and the other rBV contains the other polynucleotide. According to this embodiment, an rBV comprising any two of said polynucleotides (e.g. the polynucleotide of the invention and the polynucleotide-connector) and another rBV comprising the other polynucleotide (e.g. the polynucleotide PF(II)) are constructed, and an insect cell is subsequently co-infected with said rBVs.

In another particular embodiment, the gene expression of the fusion protein (I) of the invention and that of said fusion protein (II) is carried out in insect cells co-infected with 3 different rBVs, each of them containing one of said polynucleotides. According to this embodiment, an rBV comprising the polynucleotide of the invention, another rBV comprising the polynucleotide-connector and another rBV comprising the polynucleotide PF(II) are constructed, and an insect cell is subsequently co-infected with said rBVs.

Some of said gene constructs comprising the polynucleotide of the invention, the polynucleotide-connector and the polynucleotide PF(II) form part of the present invention and are additional aspects thereof. Said gene constructs can be obtained by conventional methods known by persons skilled in the art in view of the fusion protein (I) of the invention and of the fusion protein (II). Likewise, the expression cassettes, the recombinant vectors and the host cells containing said gene constructs comprising the polynucleotide of the invention, the polynucleotide-connector and the polynucleotide PF(II), or combinations of any two of said polynucleotides are additional aspects of the present invention and can be obtained by conventional methods known by persons skilled in the art in view of the information contained in this description [Sambrook & Russell (2001)].

Likewise, the rBVs comprising said gene constructs comprising the polynucleotide of the invention, the polynucleotide-connector and the polynucleotide PF(II) in a single gene construct, or comprising said polynucleotides in 2 or 3 separate constructs, form part of the present invention, are additional aspects thereof and can be obtained by a person skilled in the art based on that described herein and on the state of the art on this technology, as has been mentioned previously in relation to the production of the fusion protein (I) of the invention.

In another particular embodiment, the gene expression of the fusion protein (I) of the invention, the gene expression of said connector and the gene expression of said fusion protein (II) is carried out in yeasts comprising the polynucleotide of the invention, the polynucleotide-connector and the polynucleotide PF(II). Said polynucleotides can be integrated inside the yeast genome, or one of them can be integrated in the yeast genome and the other two not, or any two of said polynucleotides can be integrated in the yeast genome and the other one not, which polynucleotide can be in a gene construct in the cytoplasm, or said polynucleotides can form part of one and same nucleic acid molecule or gene construct not integrated in the yeast genome, or, alternatively, said polynucleotides can be separated into 2 or 3 different constructs but contained in one and the same yeast cell. According to this embodiment, a single recombinant yeast is produced comprising the polynucleotide of the invention, the polynucleotide-connector and the polynucleotide PF(II), in any of the aforementioned embodiments, and said yeast is then cultured under conditions allowing the expression of said fusion protein (I) of the invention and its assembly to form "unloaded" NCs, the expression of said connector and the expression of said fusion protein (II), and furthermore the specific interaction (binding) between the specific binding domains present in said "unloaded" NCs and the corresponding regions of interaction present in the connector, and the specific interaction (binding) between the specific binding domains present in said connector and the corresponding regions of interaction present in the protein ligands contained in the fusion protein (II), and, (ii) if desired, the isolation and optionally the purification of said NCs "loaded" with the product of interest.

The recombinant yeasts comprising the polynucleotide of the invention, the polynucleotide-connector and the polynucleotide PF(II), in any of the aforementioned specific embodiments, as well as the gene constructs and recombinant vectors necessary for obtaining said recombinant yeasts, form part of the present invention, are additional aspects thereof and can be obtained by a person skilled in the art based on that described herein and on the state of the art on this technology, as has been mentioned previously in relation to the production of the fusion protein (I) of the invention.

Alternatively, said loaded NC of the invention comprising a product of interest bound indirectly to the NC through the connector, depending on the nature of the connector and of the conjugate of the invention, can be obtained by means of expressing both the fusion protein (I) of the invention and the conjugate of the invention in a suitable expression system when the connector is a metabolite present in a native or recombinant manner in the expression system used to produce said NC, for example, biotin, a cofactor which is present in a native manner in multiple cells.

Therefore, in another particular embodiment, in which the conjugate of the invention is the fusion protein (II), the conjugate of the invention is the fusion protein (II), and the connector is a compound which is present in the chosen expression system, the invention provides a process (Process B.3) for producing a loaded NC of the invention comprising__a product of interest bound indirectly to the NC through a connector, which comprises culturing a host cell of the invention comprising, in addition to the polynucleotide of the invention, the polynucleotide PF(II) and the connector (which, as has been mentioned, is a product which is present in the chosen expression system, such as a metabolite, e.g. biotin), under conditions allowing the expression of said fusion protein (I) of the invention and its assembly to form "unloaded" NCs, the expression of said fusion protein (II), and furthermore the specific interaction (binding) between the specific binding domains present in said "unloaded" NCs and the corresponding regions of interaction present in said connector, and, subsequently, the specific interaction (binding) between the specific binding domains present in said connector and the corresponding regions of interaction present in the protein ligands present in said fusion protein (II), and, if desired, the recovery of said loaded NCs. After the expression of the fusion protein (I) of the invention in said cells, the expressed proteins are generally assembled and form the "unloaded" NCs, and the contact of said unloaded NCs with the connector and subsequently with the fusion protein (II), under the aforementioned conditions, causes the binding of the product of interest to the NC by means of the connector through a connector-ligand system, a loaded NC of the invention comprising a product of interest bound indirectly to the NC through the ligand thus being generated.

In a specific embodiment, the chosen expression system, i.e. the host cell of the invention, further expresses the compound or compounds necessary for the specific interaction (binding) between the specific binding domains present in said "unloaded" NCs and the corresponding regions of interaction present in said connector to occur or for enhancing it. Illustrative examples of said compounds include enzymes, etc. Said compounds can be natively expressed by said expression system; alternatively, if said expression system does not express said compound or compounds, it can then be suitably transformed by conventional methods in order to express said compound or compounds. By way of illustration, the connector can be biotin, a cofactor in the catalysis of a number of essential metabolic reactions for synthesizing fatty acids, in glyconeogenesis, in the leucine metabolism, etc. Biotin is present in a native manner in a number of cells and the presence of the biotin ligase enzyme (e.g. *E. coli* protein BirA or any other biotin ligase) is necessary for biotin to bind to a peptide; therefore, in the event that the connector is biotin, it is necessary for the chosen expression system for producing the NCs to produce said enzyme either constitutively or by means of introducing the nucleotide sequence encoding said protein. Thus, in a specific embodiment, the host cell of the invention chosen for producing NCs comprises, in addition to the polynucleotide of the invention, the polynucleotide PF(II) and the connector, a polynucleotide comprising the nucleotide sequence encoding the biotin ligase enzyme (e.g. *E. coli* protein BirA or any other suitable biotin ligase).

As a result of the strategy followed in this Process B.3, it is possible that part of the product of interest (in the form of conjugate of the invention) is included inside the NC formed by the assembly of the fusion protein (I) of the invention.

In a particular embodiment, the gene expression of the fusion protein (I) of the invention and the gene expression of said fusion protein (II) is carried out in insect cells producing said connector, infected with an rBV comprising the polynucleotide of the invention, the polynucleotide PF(II) and, optionally, in the event that said insect cell does not express the compound or compounds necessary for the connector to bind to the NC, a polynucleotide comprising the nucleotide sequence encoding said compound necessary for the connector to bind to the NC. Said polynucleotides can form part of one and the same nucleic acid molecule or gene construct, or, alternatively, they can be separated into different constructs, in any combination, but contained in one and the same rBV. According to this embodiment, a single rBV is constructed comprising the polynucleotide of the invention and the polynucleotide PF(II), and, optionally, the polynucleotide comprising the nucleotide sequence encoding a compound necessary for the connector to bind to the NC, in a single gene construct or, alternatively, in 2 or 3 separate identical or different gene constructs, and an insect cell is subsequently co-infected with said rBV.

In another particular embodiment, the gene expression of the fusion protein (I) of the invention and that of said fusion protein (II) is carried out in insect cells co-infected with 2 different rBVs, each of them containing one of said polynucleotides. If necessary, an rBV is constructed comprising the polynucleotide comprising the nucleotide sequence encoding a compound necessary for the connector to bind to the NC. According to this embodiment, once the necessary rBVs have been constructed, an insect cell is co-infected with said rBVs.

Some of said gene constructs used to implement this alternative process form part of the present invention and are additional aspects thereof. Said gene constructs can be obtained by conventional methods known by persons skilled in the art. Likewise, the expression cassettes, the recombinant vectors, including the generated rBVs, and the host cells containing said gene constructs are additional aspects of the present invention and can be obtained by conventional methods known by persons skilled in the art in view of the information contained in this description.

In another particular embodiment, the gene expression of the fusion protein (I) of the invention and the gene expression of said fusion protein (II), and, where appropriate, of the compound or compounds necessary for the connector to bind to the NC is carried out in yeasts comprising the polynucleotide of the invention and the polynucleotide PF(II) and, optionally, said polynucleotide comprising the nucleotide sequence encoding a compound necessary for the connector to bind to the NC. Said polynucleotide can be integrated inside the yeast genome, or only one of them and the other or others can be in or two gene constructs in the cytoplasm, or said polynucleotides can form part of one and the same nucleic acid molecule or gene construct not integrated in the yeast genome, or, alternatively, said polynucleotides can be separated into different constructs but contained in one and the same yeast cell. According to this embodiment, a single recombinant yeast is produced comprising the polynucleotide of the invention, the polynucleotide PF(II), and, if necessary, the polynucleotide comprising the nucleotide sequence encoding a compound necessary for the connector to bind to the NC, in any of the aforementioned embodiments, and said yeast is then cultured under conditions allowing the expression of said fusion protein (I) of the invention and its assembly to form "unloaded" NCs, the expression of said fusion protein (II), and furthermore the specific interaction (binding) between the specific binding domains present in said "unloaded" NCs and the corresponding regions of interaction present in the connector and between the specific binding domains present in said connector and the corresponding regions of interaction present in the protein ligands present in the fusion protein (II), and, (ii) if desired, the isolation and optionally the purification of said NCs "loaded" with the product of interest.

The recombinant yeasts used to implement this alternative process, as well as the gene constructs and recombinant vectors necessary to obtain said recombinant yeasts, form part of the present invention, are additional aspects thereof and can be obtained by a person skilled in the art based on that described herein and on the state of the art on this technology, as has been mentioned previously in relation to the production of the fusion protein (I) of the invention.

In a specific embodiment of this invention, the peptide insert present in the fusion protein (I) of the invention comprises a BAP, the connector is biotin, and the ligand present in the conjugate of the invention is an avidin; in this case, the invention provides a process (Process C.1) for producing a loaded NC of the invention comprising a product of interest bound indirectly to the NC through biotin, which comprises culturing a host cell of the invention comprising, in addition to a polynucleotide of the invention comprising the nucleotide sequence encoding a BAP (peptide insert), a polynucleotide encoding the biotin ligase (BL) enzyme in the presence of biotin under conditions allowing the expression of said fusion protein (I) of the invention and its assembly to form "unloaded" NCs, the expression of said BL enzyme, and furthermore the specific interaction (binding) between the specific binding domains present in said "unloaded" NCs (BAP) and the corresponding regions of interaction present in said connector (biotin), and, subsequently, the specific interaction (binding) between the specific binding domains present in said connector (biotin) and the corresponding regions of interaction present in the ligand (avidin) present in the conjugate of the invention (when it is put in contact with said "unloaded" NCs), and, if desired, the recovery of said loaded NCs. After the expression of the fusion protein (I) of the invention in said cells, the expressed proteins are generally assembled and form the "unloaded" NCs, and the contact of said unloaded NCs with the connector (biotin) and subsequently with the conjugate of the invention (avidin-product of interest), under the aforementioned conditions, causes the binding of the product of interest to the NC by means of the connector (biotin) through a biotin-avidin (connector-ligand) system, a loaded NC of the invention comprising a product of interest bound indirectly to the NC through the ligand thus being generated. If the host cell of the invention does not produce biotin naturally, biotin would then have to be added to the medium. Although virtually any BL can be used, in a particular embodiment, the BL enzyme is *E. coli* protein BirA. Likewise, if desired, the BL enzyme can be overexpressed simultaneously to the expression of the fusion protein (I) of the invention, for the purpose of raising the biotinylated NC levels (see, for example, Buoncristiani et al., 1998, J. Biol. Chem. 263, 1013-1016).

As a result of the strategy followed in this Process C.1, it is possible that part of the product of interest (in the form of conjugate of the invention) is included inside the NC formed by the assembly of the fusion protein (I) of the invention.

In a particular embodiment, the gene expression of the fusion protein (I) of the invention and the gene expression of said BL enzyme is carried out in insect cells infected with an rBV comprising the polynucleotide of the invention and the polynucleotide encoding the BL enzyme, and in the event that said insect cell does not produce biotin naturally, biotin is then added to the medium. Said polynucleotides can form part of one and the same nucleic acid molecule or gene construct, or, alternatively, they can be separated into different constructs, in any combination, but contained in one and the same rBV. According to this embodiment, a single rBV is constructed comprising the polynucleotide of the invention and the polynucleotide encoding the BL enzyme, in a single gene construct or, alternatively, in 2 separate identical or different gene constructs, and an insect cell is subsequently co-infected with said rBV. Depending on the nature of the conjugate of the invention, particularly if the ligand and the product of interest are of a peptide nature [fusion protein (II)], there may then be an additional gene construct comprising the polynucleotide encoding said fusion protein (II) forming part of said rBV either in the form of an independent gene construct or forming part of said gene construct comprising the polynucleotide of the invention and the polynucleotide encoding the BL enzyme, or, alternatively, either in a gene construct with the polynucleotide of the invention or in a gene construct with the polynucleotide encoding the BL enzyme.

In another particular embodiment, the gene expression of the fusion protein (I) of the invention and that of the BL enzyme is carried out in insect cells co-infected with 2 different rBVs, each of them containing one of said polynucleotides. According to this embodiment, once the necessary rBVs have been constructed, an insect cell is co-infected with said rBVs. As has been mentioned previously, depending on the nature of the conjugate of the invention, particularly if the ligand and the product of interest are of a peptide nature [fusion protein (II)], an additional rBV can containing a gene construct comprising the polynucleotide encoding said fusion protein (II) be constructed and the insect cell can furthermore be co-infected with said rBV.

Some of said gene constructs used to implement this alternative process form part of the present invention and are additional aspects thereof. Said gene constructs can be obtained by conventional methods known by persons skilled in the art. Likewise, the expression cassettes, the recombinant vectors, including the generated rBVs, and the host cells containing said gene constructs are additional aspects of the present invention and can be obtained by conventional methods known by persons skilled in the art in view of the information contained in this description.

In another particular embodiment, the gene expression of the fusion protein (I) of the invention and the gene expression of the BL enzyme, and, where appropriate, of said fusion protein (II), is carried out in yeasts comprising the polynucleotide of the invention and the polynucleotide encoding the BL enzyme, and, optionally, the polynucleotide PF(II). Said polynucleotides can be integrated inside the yeast genome, or only one of them and the other or others can be in one or two gene constructs in the cytoplasm, or said polynucleotides can form part of one and the same nucleic acid molecule or gene construct not integrated in the yeast genome, or, alternatively, said polynucleotides can be separated into different constructs but contained in one and the same yeast cell. According to this embodiment, a single recombinant yeast is produced comprising the polynucleotide of the invention and the polynucleotide encoding the BL enzyme, and, optionally, the polynucleotide PF(II), in any of the aforementioned embodiments, and said yeast is then cultured under conditions allowing the expression of said fusion protein (I) of the invention and its assembly to form "unloaded" NCs, the expression of said BL enzyme, and, optionally, the expression of said fusion protein (II), and furthermore the specific interaction (binding) between the specific binding domains present in said "unloaded" NCs and the corresponding regions of interaction present in the connector (biotin) and between the specific binding domains present in said connector and the corresponding regions of interaction present in the protein ligands present in the fusion protein (II) (avidin), and, (ii) if desired, the isolation and optionally the purification of said NCs "loaded" with the product of interest.

The recombinant yeasts used to implement this alternative process, as well as the gene constructs and recombinant vectors necessary for obtaining said recombinant yeasts form part of the present invention, are additional aspects thereof, and can be obtained by a person skilled in the art based on that described herein and on the state of the art on this technology, as has been mentioned previously in relation to the production of the fusion protein (I) of the invention.

In another alternative embodiment, the peptide insert present in the fusion protein (I) of the invention comprises a BAP, the connector is biotin, and the ligand present in the conjugate of the invention is an avidin and the production of loaded NCs of the invention with a product of interest bound indirectly to the NC through biotin is carried out by means of a non-metabolic, i.e. *in vitro,* biotinylation process. In this case, the invention provides a process (Process C.2) for producing a loaded NC of the invention comprising a product of interest bound indirectly to the NC through biotin, which comprises culturing a host cell of the invention comprising a polynucleotide of the invention comprising the nucleotide sequence encoding a BAP (peptide insert) in the presence of biotin ligase (BL) enzyme and biotin under conditions allowing the expression of said fusion protein (I) of the invention and its assembly to form "unloaded" NCs, and furthermore the specific interaction (binding) between the specific binding domains present in said "unloaded" NCs (BAP) and the corresponding regions of interaction present in said connector (biotin), and, subsequently, the specific interaction (binding) between the specific binding domains present in said connector (biotin) and the corresponding regions of interaction present in the ligand (avidin) present in the conjugate of the invention, and, if desired, the recovery of said loaded NCs. After the expression of the fusion protein (I) of the invention in said cells, the expressed proteins are generally assembled and form the "unloaded" NCs, and the contact of said unloaded NCs with the connector (biotin) in the presence of the BL enzyme, and, subsequently, with the conjugate of the invention (avidin-product of interest), under the aforementioned conditions, causes the binding of the product of interest to the NC by means of the connector (biotin) through a biotin-avidin (connector-ligand) system, a loaded NC of the invention comprising a product of interest bound indirectly to the NC through the ligand thus being generated. If the host cell of the invention does not naturally produce biotin or the BL enzyme, biotin and/or BL enzyme would then have to be added to the medium. Although virtually any BL can be used, in a particular embodiment, the BL enzyme is *E*. *coli* protein BirA.

In a particular embodiment, the gene expression of the fusion protein (I) of the invention is carried out in insect cells infected with an rBV comprising the polynucleotide of the invention, and in the event that said insect cell does not naturally produce biotin or BL enzyme, biotin and/or BL enzyme is then added to the medium. According to this embodiment, a single rBV is constructed comprising the polynucleotide of the invention and an insect cell is subsequently infected with said rBV. Depending on the nature of the conjugate of the invention, particularly if the ligand and the product of interest are of a peptide nature [fusion protein (II)], there may then be an additional gene construct comprising the polynucleotide encoding said fusion protein (II) forming part of said rBV either in the form of an independent gene construct or forming part of said gene construct comprising the polynucleotide of the invention. If the insect cell infected with the rBVs does not naturally produce biotin or the BL enzyme, biotin and/or BL enzyme would then have to be added to the medium.

In another particular embodiment, the gene expression of the fusion protein (I) of the invention and, where appropriate, of said fusion protein (II), is carried out in yeasts comprising the polynucleotide of the invention, and, optionally, the polynucleotide PF(II). Said polynucleotides can be integrated inside the yeast genome, or one of them can be integrated in said genome and the other one can be in a gene construct in the cytoplasm, or said polynucleotides can form part of one and the same nucleic acid molecule or gene construct not integrated in the yeast genome, or, alternatively, said polynucleotides can be separated into different constructs but contained in one and the same yeast cell. According to this embodiment, a single recombinant yeast is produced comprising the polynucleotide of the invention, and, optionally, the polynucleotide PF(II), in any of the aforementioned embodiments, and said yeast is then cultured under conditions allowing the expression of said fusion protein (I) of the invention and its assembly to form "unloaded" NCs, and, optionally, the expression of said fusion protein (II), in the presence of BL enzyme and biotin, and, furthermore the specific interaction (binding) between the specific binding domains present in said "unloaded" NCs and the corresponding regions of interaction present in the connector (biotin) and between the specific binding domains present in said connector and the corresponding regions of interaction present in the protein ligands present in the fusion protein (II) (avidin), and, (ii) if desired, the isolation and optionally the purification of said NCs "loaded" with the product of interest.

The invention also provides a process for obtaining a biotinylated NC which comprises culturing a host cell of the invention comprising, in addition to a polynucleotide of the invention comprising the nucleotide sequence encoding a BAP (peptide insert), a polynucleotide encoding the biotin ligase enzyme (BL) in the presence of biotin under conditions allowing the expression of said fusion protein (I) of the invention and its assembly to form "unloaded" NCs, the expression of said BL enzyme, and furthermore the specific interaction (binding) between the specific binding domains present in said "unloaded" NCs (BAP) and the corresponding regions of interaction present in said connector (biotin), and, if desired, the recovery of said biotinylated NCs. After the expression of the fusion protein (I) of the invention in said cells, the expressed proteins are generally assembled and form the "unloaded" NCs, and the contact of said unloaded NCs with the connector (biotin), under the aforementioned conditions, causes the binding of the biotin, either because the host cell of the invention produces it naturally or because it has been added to the medium, to the NC, a biotinylated NC of the invention thus being generated. Although virtually any BL can be used, in a particular embodiment, the BL enzyme is *E. coli* protein BirA.

The biotinylated NC of the invention is a useful tool for binding any product of interest, both of a peptide nature and of a non-peptide nature, through an avidin ligand (e.g. monoavidin (mAV)). In a particular embodiment, the production of said biotinylated NCs is carried out in insect cells infected with an rBV comprising the polynucleotide of the invention and the polynucleotide encoding the BL enzyme. In the event that said insect cell does not naturally produce biotin, biotin is then added to the medium. Said polynucleotides can form part of one and the same nucleic acid molecule or gene construct, or, alternatively, they can be separated into different constructs, in any combination, but contained in one and the same rBV. According to this embodiment, a single rBV is constructed comprising the polynucleotide of the invention and the polynucleotide encoding the BL enzyme in a single gene construct or, alternatively, in 2 separate identical or different gene constructs, and an insect cell is subsequently co-infected with said rBV.

In another particular embodiment, the production of said biotinylated NCs of the invention is carried out in insect cells co-infected with 2 different rBVs, each of them containing one of said polynucleotides. According to this embodiment, once the necessary rBVs have been constructed, an insect cell is co-infected with said rBVs.

In another particular embodiment, the production of said biotinylated NCs of the invention is carried out in yeasts comprising the polynucleotide of the invention and the polynucleotide encoding the BL enzyme. Said polynucleotides can be integrated inside the yeast genome, or only one of them and the other one can be in a gene construct in the cytoplasm, or said polynucleotides can form part of one and the same nucleic acid molecule or gene construct not integrated in the yeast genome, or, alternatively, said polynucleotides can be separated into different constructs but contained in one and the same yeast cell. According to this embodiment, a single recombinant yeast is produced comprising the polynucleotide of the invention and the polynucleotide encoding the BL enzyme, in any of the aforementioned embodiments, and said yeast is then cultured under conditions allowing the expression of said fusion protein (I) of the invention and its assembly to form "unloaded" NCs, the expression of said BL enzyme, and furthermore the specific interaction (binding) between the specific binding domains present in said "unloaded" NCs and the corresponding regions of interaction present in the connector (biotin), and, (ii) if desired the isolation and optionally the purification of said biotinylated NCs of the invention.

As has been mentioned previously, if the yeasts used to produce the biotinylated NCs of the invention do not naturally produce biotin, biotin would then have to be added to the medium.

The invention also provides an alternative process for obtaining a biotinylated NC which comprises culturing a host cell of the invention comprising a polynucleotide of the invention comprising the nucleotide sequence encoding a BAP (peptide insert), in a medium comprising biotin ligase (BL) and biotin under conditions allowing the expression of said fusion protein (I) of the invention, its secretion and assembly to form "unloaded" NCs, and furthermore the specific interaction (binding) between the specific binding domains present in said "unloaded" NCs (BAP) and the corresponding regions of interaction present in said connector (biotin), and, if desired, the recovery of said biotinylated NCs. After the expression of the fusion protein (I) of the invention in said cells, the expressed proteins are generally assembled and form the "unloaded" NCs, and the contact of said unloaded NCs with the connector (biotin) in the presence of the BL enzyme, in the aforementioned conditions, causes the binding of the biotin to the NC, a biotinylated NC of the invention thus being generated. Although virtually any BL can be used, in a particular embodiment, the BL enzyme is *E. coli* protein BirA.

In a particular embodiment, the production of said biotinylated NCs of the invention is carried out in insect cells infected with an rBV comprising the polynucleotide of the invention in a medium comprising BL and biotin. According to this embodiment, a single rBV is constructed comprising the polynucleotide of the invention, and an insect cell is subsequently infected with said rBV and cultured in a medium comprising BL and biotin.

In another particular embodiment, the production of said biotinylated NCs is carried out in yeasts comprising the polynucleotide of the invention. Said polynucleotide can be integrated inside the yeast genome, or can be contained in a gene construct in the cytoplasm not integrated in the yeast genome. According to this embodiment, a single recombinant yeast is produced comprising the polynucleotide of the invention and is cultured in a medium with BL and biotin under conditions allowing the expression of said fusion protein (I) of the invention and its assembly to form "unloaded" NCs, and furthermore the specific interaction (binding) between the specific binding domains present in said "unloaded" NCs and the corresponding regions of interaction present in the connector (biotin), and, (ii) if desired the isolation and optionally the purification of said biotinylated NCs of the invention.

In another alternative embodiment, the invention provides another alternative process for obtaining a biotinylated NC which comprises putting in contact an unloaded NC of the invention obtained by the assembly of a fusion protein (I) of the invention comprising a SAP and a peptide insert, said peptide insert comprising a biotin acceptor peptide (BAP), in a medium comprising biotin ligase (BL) enzyme and biotin under conditions allowing the specific interaction between said BAP present in said NCs and biotin, and, if desired, the recovery of said biotinylated nanocarriers. Although virtually any BL can be used, in a particular embodiment, the BL enzyme is *E. coli* protein BirA.

### 4. Applications

The NCs of the invention can be used in research; they can also be used as vectors of products of interest, i.e. as systems for delivering products of interest, such as molecules with biological activity both of a peptide nature and of a non-peptide nature, for example, drugs, peptides, proteins, antibodies, hormones, enzymes, lipids, sugars, nucleic acids (DNA, RNA, PNA, etc.), e.g. oligonucleotides, polynucleotides, genes, interfering RNA, etc., useful in the treatment and/or prevention of diseases as well as in diagnosis. In a particular embodiment, said products of interest are peptide antigens or inducers of immune responses in the subject to which they are administered, they can therefore be used in the preparation of (monovalent, bivalent or polyvalent) vaccines against animal and human disease caused by viruses, bacteria, parasites or any other types of microorganisms or against tumor diseases, etc.; in another particular embodiment, said product of interest is a molecule with biological activity, e.g. a drug, a peptide, an antibody or a functional fragment thereof, a hormone, an enzyme, etc.; in a particular embodiment, said product of interest comprises lipidized tissue factor or a functional variant thereof for the treatment of hemorrhages, etc.; in another particular embodiment, said product of interest is a nucleic acid, e.g. an oligonucleotide, a polynucleotide, a gene, an interfering RNA, etc. Therefore, in another aspect, the invention relates to the use of the NCs of the invention formed by the assembly of the fusion protein (I) of the invention as a carrier for products of interest or as a vector in gene therapy.

For their administration to a subject, the NCs of the invention, particularly the loaded NCs of the invention, are formulated with pharmaceutically acceptable carriers and excipients. As used herein, the term "subject" relates to a member of a mammalian species and includes, but is not limited to, domestic animals, primates and humans; the subject is preferably a male or female human being of any age or race.

Therefore, in another aspect, the invention relates to a pharmaceutical composition comprising a loaded NC of the invention together with a pharmaceutically acceptable excipient. In a particular embodiment, said pharmaceutical composition is a (mono- or polyvalent) vaccine comprising a therapeutically effective amount of loaded NCs of the invention together with, optionally, one or more pharmaceutically acceptable excipients and/or adjuvants. Said vaccine can be used to protect animals (including human beings) against diseases caused by pathogenic agents (e.g. viruses, bacteria, parasites, etc.), or against tumor diseases. In another particular embodiment, said pharmaceutical composition is a composition intended for its use in gene therapy.

In the sense used in this description, the expression "therapeutically effective amount" relates to the amount of NCs of the invention, calculated to cause the desired effect and will generally be determined, among other causes, by the typical characteristics of the NCs of the invention used and the therapeutic effect to be achieved.

The pharmaceutically acceptable excipients and adjuvants which can be used in the pharmaceutical compositions provided by this invention are known by the persons skilled in the art and are usually used in the preparation of pharmaceutical compositions.

The pharmaceutical composition of the invention can be presented in any suitable form of administration, typically in the form of a suspension, and can be administered by any suitable method, for example, parenterally, etc., for which it will include the pharmaceutically acceptable excipients necessary for formulating the desired form of administration. The formulations can be single-dose formulations and can be prepared according to classic Galenic methods. The auxiliary substances, carriers and excipients used must be pharmaceutically and pharmacologically tolerable, they must be able to be combined with other components of the formulation and must not exert any adverse effect on the treated subject. A review of the different dosage forms for administering medicinal products and of the excipients necessary for obtaining them can be found, for example, in "Tratado de Farmacia Galénica", C. Faulí i Trillo, 1993, Luzán 5, S.A. Ediciones, Madrid.

In a particular embodiment, said pharmaceutical composition is prepared in the form of an aqueous solution of suspension in a pharmaceutically acceptable diluent, such as saline solution, phosphate buffered saline solution (PBS), or any other pharmaceutically acceptable diluent. Likewise, in a particular embodiment, the vaccine provided by this invention is administered parenterally, for example, intraperitoneally, subcutaneously, etc.

By way of illustration, the dose of active ingredient (NCs of the invention) to be administered to a subject will be a therapeutically effective amount and can vary within a wide range. The pharmaceutical composition provided by this invention can be administered once or more times a day for preventive or therapeutic purposes. The dose of active ingredient to be administered will depend on a number of factors, including the characteristics of the product to be administered, such as for example, its activity and biological half-life, the concentration of the product in the pharmaceutical composition, the clinical condition of the subject, the severity of the pathology, the chosen pharmaceutical dosage form, etc.

The pharmaceutical composition provided by this invention can contain either a single type of NCs of the invention or two or more different NCs of the invention together with one or more pharmaceutically acceptable excipients or carriers. In a particular embodiment, said pharmaceutical composition comprises a single type of NCs of the invention. In another particular embodiment, said pharmaceutical composition comprises two or more NCs of the invention.

The pharmaceutical composition provided by this invention, if desired, can be used together with other drugs for the purpose of increasing the efficacy of the pharmaceutical composition provided by this invention, a combination therapy thus being generated. Said additional drugs can form part of the same pharmaceutical composition or, alternatively, they can be provided as a separate pharmaceutical composition for its administration at the same time (simultaneous administration) as the pharmaceutical composition of the invention or at different times (sequential administration) with respect to the administration of the pharmaceutical composition provided by this invention.

In addition, the NCs of the invention can be contained in an inert medium, i.e. in a medium that does not substantially affect their stability, for example, in an aqueous medium such as saline solution, phosphate buffered saline solution (PBS), etc. Therefore, in another aspect, the invention relates to a composition comprising an NC of the invention and an inert medium.

The following examples illustrate the invention and must not be considered as limiting the scope thereof.

### EXAMPLES

The following examples describe the production of nanocarriers (NCs) (Example 1), the production of conjugates or recombinant protein adapters (RPAs) that can be coupled to NCs (Example 2) and the production of NCs loaded with said recombinant protein adapters (Example 3).

The self assembly protein identified as SAP that is referred to in said examples is the recombinant protein identified as IBDV pVP2_452, the amino acid sequence (SEQ ID NO: 1) of which is formed by the contiguous amino acid sequence comprised between residue 1 and residue 452 of the IBDV protein VP2; said protein VP2_452 (SAP) gives rise to the formation of a structure with icosahedral symmetry (with a triangulation number T=1) formed by 60 monomers of the protein organized into 20 identical trimers forming nanocarriers (NCs).

The Materials and Methods used in said examples are described below.

### Materials and Methods

### 1. Cell Cultures

The recombinant baculoviruses (rBVs) that are referred to in this description were grown using monolayers of HighFive (HF) cells (Invitrogen) from *Tricoplusia Ni* larvae. The cultures were maintained in TC100 medium (Gibco-BRL) supplemented with 10% fetal serum (Gibco-BRL).

The cultures used for producing recombinant protein mAV_NANP (SEQ ID NO: 23) were, after the infection with recombinant baculovirus rBV_mAV_NANP, maintained in Schneider's medium (Sigma) without biotin and not supplemented with fetal serum.

The cultures of *S. cerevisiae* strain YPH499 (Stratagene) were maintained and grown according to the previously described protocol (Garriga et al., 2006).

### 2. Generation of recombinant plasmids

**Plasmid pFB_SAP.** Gene SAP (SEQ ID NO: 6) was constructed by means of PCR using the primers: Bap 1 (SEQ ID NO: 7) and Bap 4 (SEQ ID NO: 8). Purified DNA corresponding to plasmid VT7LacOI/VP2 was used as a template (Fernández-Arias et al., 1997). The resulting DNA product was purified from an agarose gel and subjected to digestion with enzymes NdeI and BamHI. After the digestion, the DNA was ligated to plasmid pFastBacl (Invitrogen) previously digested with enzymes NdeI and BamHI. The resulting plasmid, called pFB_SAP, was analyzed by means of nucleotide sequencing.

**Plasmid pFB_SAP_BAP.** Chimeric gene SAP_BAP (SEQ ID NO: 5) was constructed by means of PCR using, respectively, 2 pairs of primers: Bap 1 (SEQ ID NO: 7) and Bap 2 (SEQ ID NO: 9); and Bap 3 (SEQ ID NO: 10) and Bap 4 (SEQ ID NO: 8). Purified DNA corresponding to plasmid pFB_SAP was used as a template. The resulting DNAs were isolated from agarose-TBE gels (TBE: 89 mM Tris, 89 mM boric acid, 2 mM EDTA (ethylenediaminetetraacetic acid), precipitated with isopropanol in the presence of ammonium acetate and resuspended in water. Both DNA fragments were combined in a single tube and subjected to a new PCR round using the primers Bap 1 (SEQ ID NO: 7) and Bap 4 (SEQ ID NO: 8). The resulting DNA product (685 bp) was purified from an agarose gel and subjected to digestion with enzymes NdeI and BamHI. After the digestion, the DNA was ligated to plasmid pFastBac1 (Invitrogen) previously digested with enzymes NdeI and BamHI. The resulting plasmid, called pFB_SAP_BAP (SEQ ID NO: 11), was analyzed by means of nucleotide sequencing.

**Plasmid pFBD_BirA_SAP_BAP.** The gene BirA nucleotide sequence (SEQ ID NO: 12) encoding biotin ligase (BL) enzyme [specifically, *E. coli* protein Bir A] was amplified by means of PCR from DNA isolated from an *Escherichia coli* (strain DH5α) culture, using primers Bir A_1 (SEQ ID NO: 13) and Bir A_2 (SEQ ID NO: 14). The resulting DNA fragment was digested with restriction enzymes XhoI and *SphI*, and cloned, under the transcriptional control of promoter p10, into plasmid pFastBacDual (Invitrogen) digested with the same enzymes. The resulting plasmid, pFastBacDual_Bir A (SEQ ID NO: 15), was purified and analyzed by means of nucleotide sequencing for the purpose of verifying the correctness of the sequence corresponding to gene BirA. A DNA fragment containing the sequence corresponding to chimeric gene SAP_SAP (SEQ ID NO: 5) was then generated using primers Bap 1 (SEQ ID NO: 7) and Bap4 4 (SEQ ID NO: 8). This DNA was digested with enzymes BglII and HindIII and cloned, under the transcriptional control of promoter pPH, into plasmid pFastBacDual_BirA (SEQ ID NO: 15) digested with the same enzymes. The resulting plasmid, called pFBD_BirA_SAP_BAP (SEQ ID NO: 16), was used to generate the recombinant baculovirus (rBV) called rEV/FBD_BirA_SAP_BAP.
**pESCURA_BirA_SAP_BAP.** Plasmid pESCURA-VP2 (Garriga et al., 2006) was digested with enzyme SexAI, giving rise to the release of 2 DNA fragments with 7,435 and 482 base pairs (bp), respectively. The 7,435 bp fragment was purified from an agarose-TBE gel and subsequently used for its ligation to a 524 bp DNA fragment obtained by means of the SexAI digestion of plasmid pFB_SAP_BAP (SEQ ID NO: 11). After the ligation, a plasmid called pESCURA_SAP_BAP, with a size of 7,959 bp, was obtained. A 985 bp DNA fragment containing the sequence encoding gene BirA was then obtained by means of PCR, using primers BirA Lev 1 (SEQ ID NO: 17) and BirA Lev 2 (SEQ ID NO: 18), and plasmid pFBD_BirA_SAP_BAP (SEQ ID NO: 16) as a template. This fragment was digested with enzymes EcoRI and BglII and inserted in plasmid pESCURA_SAP-BAP digested with the same enzymes to generate plasmid pESCURA_BirA_SAP_BAP (SEQ ID NO: 19).

**Plasmid pFB_mAV_NANP.** Chimeric gene mAV_NANP (SEQ ID NO: 20) was synthesized by means of PCR (Genescript Co). The corresponding DNA contains unique restriction sites *Bgl*II and HindIII at its 5' and 3' ends, respectively. The DNA corresponding to chimeric gene mAV_NANP (SEQ ID NO: 20) was subjected to restriction with enzymes *Bgl*II and *Hind*III and cloned into plasmid pFastBacl (Invitrogen) digested with enzymes *Bam*HI and HindIII. The resulting plasmid, pFB_mAV_NANP (SEQ ID NO: 21), was analyzed by means of nucleotide sequencing for the purpose of determining that the inserted sequence corresponded to the designed chimeric gene mAV_NANP (SEQ ID NO: 20).

**Plasmid pFB_mAV**. Plasmid pFB_mAV_NANP (SEQ ID NO: 21) was subjected to restriction with enzyme *Bam*HI and the resulting 5,228 bp fragment was purified and incubated with DNA ligase. The region corresponding to the repetition containing six tandem copies of the NANP peptide (SEQ ID NO: 22, the sequence of the peptide is NANP=Asn Ala Asn Pro) was thus eliminated. The resulting plasmid, called pFB_mAV (SEQ ID NO: 25), contains the chimeric gene mAV lacking the stop codon, under the transcriptional control of promoter pPH, fused to a multiple cloning site.

### 3. Generation and analysis of the recombinant baculoviruses rBV_SAP_BAP

The recombinant baculoviruses (rBVs) were generated according to the protocol based on the use of bacmids, Bac-to-bac (Invitrogen). The bacmids obtained were used to transfect HF cells (Invitrogen). The culture medium of the transfected cells was collected at 96 hours post-transfection and analyzed to determine the viral titer according to the instructions of Invitrogen.

### 4. Purification and biochemical and structural analysis of NCs derived from the expression of protein SAP_BAP (SEQ ID NO: 4)

The purifications were carried out from cultures of HF cells infected with the recombinant baculoviruses rBV_SAP_BAP or rBV_FBD_BirA_SAP_BAP. The multiplicity of infection used for all the assays was 1 plaque forming unit (PFU) of rBV_SAP_BAP per cell. The infected cultures were collected at 72 hours post-infection (hpi) and subjected to the previously described purification protocol by means of ultracentrifugation in sucrose gradients (Garriga et al., 2006). The NCs produced in *S. cerevisiae* cultures transformed with plasmid pESCURA_BirA_SAP_BAP (SEQ ID NO: 19) were purified from cultures maintained for 24 hours at 30°C.

The corresponding samples obtained were analyzed by means of:
i) immunoblotting using specific anti-SAP serum (i.e. IBDV anti-VP2 _452) according to the previously described protocol (Saugar et al., 2005); and
ii) transmission electron microscopy, after they were stained with 2% diluted uranyl acetate in water.

The capacity to bind to biotin of the NCs produced by means of the co-expression of genes SAP_BAP (SEQ ID NO: 5) and BirA (SEQ ID NO: 12) was determined in nitrocellulose membranes. To that end, the purified NCs were separated by means of SDS-PAGE, transferred to cellulose membranes and incubated with a 1/500 dilution of peroxidase-coupled streptavidin (Sigma). The membranes were then developed by means of incubating with a 0.6 mg/ml solution of the chromogenic substrate 1-chloro-4 -naphthol diluted in phosphate buffered solution (PBS) in the presence of 0.15% H₂O₂,

### 5. In vitro NC biotinylation assays

The purified NCs were resuspended to a concentration of 5 ug/ml in Biomix-A biotinylation buffer (50 mM bicine buffer, pH 8.3; 10 mM ATP, 10 mM MgOAc, 50 µM d-biotin) (Avidity, LLC) in the presence of 50 µM of biotin and 2.5 µg of purified biotin ligase (BL) (Avidity, LLC). The samples were incubated for 30 minutes at 30°C. After this period, the samples were separated by means of sodium dodecyl sulfate polyacrylamide gel electrophoresis (SDS-PAGE), transferred to the cellulose membranes and incubated with a 1/500 dilution of peroxidase-coupled streptavidin (Sigma). The membranes were then developed by means of incubating with a 0.6 mg/ml solution of the chromogenic substrate 1-chloro-4-naphthol diluted in phosphate buffered solution (PBS) in the presence of 0.15% H₂O₂,

### 6. Production of recombinant protein mAV_NANP (SEQ ID NO: 23)

Monolayers of H5cells [*E. coli* (strain DH5α)], grown in TC100 medium supplemented with 10% fetal serum, were washed 3 times with Schneider's medium and infected with recombinant baculovirus rBV/FB_mAV_NANP. The supernatant of the cultures was collected at 72 hpi and, after eliminating the cell remains by means of centrifugation (10,000xg for 15 minutes), it was used to determine the presence of protein mAV_NANP (SEQ ID NO: 23). To that end, 15 µl aliquots of the sample were mixed with 3.5 µl of loading buffer (10 mM Tris-HCl (pH 7.6), 0.03% bromophenol blue, 0.03% xylene cyanol FF, 0.15% orange G, 60% glycerol, 60 mM EDTA), and subsequently subjected to SDS-PAGE and electro-transferred to a nitrocellulose membrane. The membranes were incubated with rabbit anti-avidin serum (Sigma) and developed by means of incubating with goat IgG anti-rabbit IgG peroxidase-conjugate (Sigma) followed by an incubation with 0.6 mg/ml 1-chloro-4-naphthol diluted in PBS in the presence of 0.15% H₂O₂.

### EXAMPLE 1

### Production of Nanocarriers (NC)

### 1.1 Genetic modification of a self assembly protein (SAP) by means of introducing the sequence corresponding to the biotin acceptor peptide (BAP)

The possibility of generating genetically modified versions of a self assembly protein (SAP) by means of incorporating heterologous amino acid sequences has been analyzed. As has been mentioned previously, the SAP on which the different analyses have been conducted has been the protein identified as IBDV pVP2_452 (SEQ ID NO: 1), giving rise to the formation of a structure with icosahedral symmetry (with a triangulation number T=1) formed by 60 monomers of the protein organized into 20 identical trimers.

The modifications introduced must meet two essential requirements: i) the introduction of the heterologous sequences should not alter the self assembly capacity of said SAP; and ii) the heterologous sequences introduced should be located in a region of said SAP accessible for the solvent in the NCs.

For this purpose, an in-depth study of the atomic structure of said previously described SAP (Garriga et al., 2006) (Protein Data Bank accession no. 2GSY) was conducted by means of using the UCSF Chimera computer program (http://www.cgl.ucsf.edu/chimera/). After this analysis, a strategy was designed to incorporate the heterologous amino acid sequences in the region corresponding to loop P_{HI}, located in the region exposed to the solvent of said SAP in the assemblies (Figure 2). As an illustrative example for verifying the efficiency of the designed strategy, the decision was made to insert the nucleotide sequence corresponding to the biotin acceptor peptide, generically called BAP (SEQ ID NO: 3).

The nucleotide sequence corresponding to the BAP was inserted by means of PCR using "megaprimers" according to the methodology described in the Materials and Methods section. The genetically modified gene SAP, called SAP_BAP (SEQ ID NO: 5) was cloned into insertion vector pFastBac1, under the transcriptional control of promoter pPH, and analyzed by means of nucleotide sequencing to verify the correctness of the generated sequence. The plasmid obtained [pFB_SAP_BAP (SEQ ID NO: 11)] was used to produce the recombinant baculovirus (rBV) called rBV/FB_SAP_BAP according to the protocol described in Materials and Methods.

### 1.2 Expression of gene SAP_SAP (SEQ ID NO: 5)

*Tricoplusia Ni* cell cultures were infected with rSV/FS_SAP_BAP with a multiplicity of infection (MOI) of 1 PFU per cell. At 72 hpi, the cultures were collected and the corresponding extracts were subjected to SDS-PAGE and immunoblotting using the specific anti-SAP serum (IBDV anti-pVP2), described as anti_VP2 by Martínez-Torrecuadrada *et al*. (2000). IBDV pVP2 and its variants, e.g. VP2_452, herein called SAP, has an electrophoretic mobility in SDS-PAGE gels greater than that expected taking into account its molecular mass. As shown in Figure 3, the infected cells accumulate a protein with an electrophoretic mobility corresponding to a molecular mass of 43 kDa, specifically recognized by the anti-SAP serum.

### 1.3 Biochemical and structural characterization of the protein assemblies produced by means of the expression of chimeric peptide SAP_BAP (SEQ ID NO: 4)

For the purpose of determining if chimeric protein SAP_BAP (SEQ ID NO: 4) maintains the self assembly capacity described for the parental protein SAP (SEQ ID NO: 1), cultures of insect cells were infected with rBV/FB_SAP_BAP. The cultures were collected at 72 hpi and subjected to the purification protocol based on ultracentrifugation in sucrose gradients for NCs described in the Materials and Methods section. The samples collected during the fractionation of the gradients were analyzed by means of SDS-PAGE and immunoblotting using specific anti-SAP serum (IBDV anti-pVP2) (Martinez-Torrecuadrada *et al*., (2000), Fernández-Arias et al., 1998). As shown in Figure 4A, the antiserum used to detect the presence of a protein with an electrophoretic mobility of approximately 43 kDa (the molecular mass of protein SAP_BAP is 50 kDa in the upper region (top) of the gradient, where the NCs must be located (Saugar et al., 2005). The samples corresponding to this area of the gradient were dialyzed against a PES buffer (25 mM PIPES, pH 6.2, 150 mM NaCl, 20 mM CaCl₂), deposited on grids for electron microscopy, stained with uranyl acetate and viewed by means of transmission electron microscopy. As observed in Figure 4B, the selected samples contain macromolecular assemblies with a size and morphology similar to the previously described NCs (Saugar et al., 2005). The group of the described results shows that the expression of gene SAP_BAP (SEQ ID NO: 5) in insect cells gives rise to the synthesis of a protein with the expected electrophoretic mobility, which can self assemble to give rise to structures similar to the T=1, subviral particles of IBDV. A corollary of these results is the demonstration of the possibility of genetically modifying, by means of introducing heterologous sequences, the gene encoding protein SAP (SEQ ID NO: 1) without altering its self assembly capacity.

### 1.4 In vitro NC biotinylation

The functionality of the proposed design directly depends on the capacity of the NCs to covalently and specifically bind biotin molecules. For the purpose of analyzing this aspect, NCs were purified according to the methodology described in previous section 1.3. The purified NCs from insect cells infected with recombinant baculovirus rBV/FB_SAP_BAP were incubated with biotin in the presence (+) or absence (-) of purified biotin ligase (BL) enzyme [occasionally identified in this description as protein BL (biotin ligase)] according to the protocol described in the Materials and Methods section. As a control for this experiment, non-genetically modified NCs obtained by means of the expression of gene SAP (SEQ ID NO: 6) using rBV_FS_SAP were identically incubated. After the incubation with the BL enzyme, the samples were analyzed by means of SDS-PAGE and stained with Coomassie blue (Figure 5A) or transferred to nitrocellulose membranes. The membranes were developed by means of immunoblotting using anti-SAP serum (Martinez-Torrecuadrada et al., (2000), Fernández-Arias et al., 1998) (Figure 5B) or incubated in the presence of peroxidase-coupled streptavidin (Figure 5C), for the purpose of locating the presence of proteins with the capacity to bind to streptavidin [the latter by means of incubating the membranes with the peroxidase chromogenic substrate (1-chloro-4-naphthol)].

As observed in Figure 5A, the samples corresponding to the purified NCs from insect cells infected with recombinant baculovirus rBV/FB_SAP_BAP (NC_SAP_BAP) (lane 1) and NC (lane 3) contain a single band corresponding to proteins SAP_BAP (SEQ ID NO: 4) (estimated electrophoretic mobility of 43 kDa) and SAP (SEQ ID NO: 1) (estimated electrophoretic mobility of 45 kDa), respectively. The samples incubated in the presence of biotin ligase (BL) (lanes 2 and 4) contain a second band of approximately 30 kDa corresponding to this protein (BL). The immunoblotting (Figure 5B) confirms the identity of the bands corresponding to proteins SAP_BAP and SAP, respectively.

Figure 5C shows that the single protein which can specifically bind the streptavidin-peroxidase complex is protein SAP_BAP (SEQ ID NO: 4) previously incubated with biotin in the presence of protein BL (lane 2). This result shows, in a conclusive manner, that the NCs produced by means of the expression of chimeric protein SAP_BAP (SEQ ID NO: 4) subjected to *in vitro* biotinylation by means of incubation with protein BL, can specifically interact with the streptavidin-peroxidase complex.

### 1.5 Development of expression systems for producing metabolically biotinylated NCs

Once the methodology for producing biotinylated NCs *in vitro* has been established, the decision was made to analyzed the possibility of developing systems for producing metabolically biotinylated NCs. The strategy for producing these NCs was based on the co-expression of the genes encoding proteins SAP_BAP (SEQ ID NO: 4) and BL using the recombinant baculovirus (rBV) rBV_BirA_SAP_BAP, the construction of which is detailed in the Materials and Methods section. This rBV was used to infect cultures of insect cells (MOI of 1 PFU/cell). At 72 hpi, the infected cultures were collected and subjected to the purification protocol for NCs by means of ultracentrifugation in sucrose gradients (Materials and Methods). The samples from the gradients were analyzed by means of SDS-PAGE and immunoblotting using anti-SAP serum (IBDV anti-pVP2) (Martinez-Torrecuadrada et al., (200D), Fernández-Arias et al., 1998). As observed in Figure 6, the gradient contains a protein with the expected molecular mass, specifically recognized by the anti-SAP serum. A sample containing a mixture of the gradient fractions with a larger content of fusion protein SAP_BAP (SEQ ID NO: 4) was subjected to SDS-PAGE and transferred to nitrocellulose. The corresponding membrane was incubated in the presence of peroxides-coupled streptavidin and developed in the presence of 1-chloro-4-naphthol for the purpose of determining the capacity of fusion protein SAP_BAP (SEQ ID NO: 4) to specifically bind to streptavidin. As shown in Figure 6B, fusion protein SAP_BAP (SEQ ID NO: 4) expressed in the presence of the BL enzyme specifically binds the streptavidin-peroxidase complex. An aliquot of the aforementioned sample was analyzed by means of transmission electron microscopy (Figure 6C, the presence of metabolically biotinylated NCs with a morphology and size similar to those of the NCs generated by means of the expression of fusion protein SAP_BAP (SEQ ID NO: 4) expressed in the absence of the BL enzyme was determined.

Similar results were obtained by means of the co-expression of genes SAP_BAP (SEQ ID NO: 5) and BirA (SEQ ID NO: 12) in *S. cerevisiae* cultures transformed with expression vector pESCURA_BirA-SAP_BAP (SEQ ID NO: 19). The construction of this vector and the generation of transformed yeasts are detailed in the Materials and Methods section.

The group of the described results shows that the co-expression of proteins SAP_BAP (SEQ ID NO: 4) and BL gives rise to the assembly of metabolically biotinylated NCs.

### EXAMPLE 2 Production of recombinant protein adapters (RPAs) that can be coupled to NCs

Once the phase of developing NCs has been completed, the generation of systems for producing recombinant proteins, generically called RPAs (recombinant proteins adapters), a particular case of the conjugates of the invention in which both the ligand and the product of interest are of a peptide nature, which can be specifically and stably coupled to biotinylated NCs, was required.

### 2.1 Design, construction and characterization of a vector for producing RPAs with the capacity to bind to biotin

The experimental strategy for producing RPAs was based on the development of systems allowing the expression of hybrid molecules formed by the fusion of: i) a protein domain providing the capacity to bind to biotin; and ii) the amino acid sequence corresponding to the protein (or proteins) of interest which are to be coupled to the NCs. For this purpose, a chimeric gene called mAV_NANP (SEQ ID NO: 20) (Figure 7) was designed and synthesized, formed by the region encoding a mutant form of the avidin protein (*G. gallus*) (Laitinen et al., 2003), lacking a stop codon (mAV), fused in phase (maintaining the reading phase corresponding to avidin) to a nucleotide sequence encoding 6 consecutive repetitions of the NANP peptide (SEQ ID NO: 22) followed by a stop codon (TAA), after which a multiple cloning site (MCS) was introduced in its terminal 3' region. The nucleotide sequence of said chimeric gene mAV_NANP corresponds to SEQ ID NO: 20 and the amino acid sequence of said fusion protein mAV_NANP corresponds to SEQ ID NO: 23. In the context of the chimeric gene, the tandem repetition of the NANP peptide (SEQ ID NO: 22), including the stop codon, is flanked by restriction sites *Bam*HI. The synthesized chimeric gene contains unique restriction sites *Bgl*II and HindIII in its 5' and 3' ends, respectively. Plasmid pFB_mAV_NANP (SEQ ID NO: 21), the construction of which is detailed in the Materials and Methods section, was used to generate the rBV called rBV/FB_mAV_NANP. This rBV was used to infect H5 cells [*E. coli* (strain DH5α)]. The supernatant of the cultures was collected at 72 hpi and, after eliminating the cell remains, it was analyzed by means of SDS-PAGE and immunoblotting using specific anti-avidin serum. As shown in Figure 8, the supernatant (SN) of the cultures infected with rBV/FB_mAV_NANP (SN+) contains a protein with the expected electrophoretic mobility (19.5 kDa) for the chimeric secretion protein mAV_NANP (SEQ ID NO: 23) specifically recognized by the anti-avidin serum. In contrast, the antiserum does not recognize any protein in a control sample obtained from a culture infected with an irrelevant rBV called rBV/His-VP3 (Kochan et al., 2003). These results show that the expression of gene mAV_NANP (SEQ ID NO: 20) in insect cells gives rise to the synthesis of a recombinant protein with the correct size which is secreted to the extracellular medium.

### 2.2 Generation of a universal vector for producing RPAs

The development of a universal vector universal for producing RPAs, called pFB_mAV (SEQ ID NO: 24), was based on the manipulation of vector pFB_mAV_NANP (SEQ ID NO: 21), the construction of which is described in the Materials and Methods section. The nucleotide sequence of this new 5,228 nucleotide construct contains unique restriction targets *Bgl*II (nucleotides 1-6) and HindIII (nucleotides 712-717) for cloning heterologous sequences into vector pFB_mAV (SEQ ID NO: 24).

### EXAMPLE 3

### Production of NCs loaded with recombinant protein adapters

For the purpose of determining the viability of the proposed strategy for producing NCs loaded with recombinant proteins, the NCs were incubated with the BL enzyme in the presence of absence of biotin. For the purpose of eliminating the biotin that is not bound to the NCs as soluble BL enzyme, after the incubation, the samples were deposited on sucrose cushions (25% in PES buffer [(25 mM PIPES pH 6.2, 150 mM NaCl, 20 mM CaCl₂]) and centrifuged (27,000 rpm in a SW40rotor [Beckman] for 2 hours). The sediments were then collected, resuspended in PES buffer and incubated with supernatants of cultures of insect cells containing or not containing the recombinant protein mAV_NANP (SEQ ID NO: 23). The samples thus prepared were incubated at room temperature for 30 minutes. They were then subjected to centrifugation on sucrose cushions, as described above, for the purpose of eliminating the protein that is not coupled to the NCs. The resulting sediments were resuspended in PES buffer and analyzed by means of SDS-PAGE and immunoblotting using specific anti-avidin and anti-SAP serums. As shown in Figure 9, the biotinylated NCs (NC-biot) incubated with supernatants containing mAV_NANP (SN+) are specifically coupled to protein mAV_NANP (lane 8). In contrast, the samples containing non-biotinylated NCs (NC-) incubated with supernatant (SN) containing mAV_NANP (lane 7) or non-biotinylated NCs incubated with an SN lacking mAV_NANP (lane 6) are not coupled to protein mAV_NANP. These results show that the produced protein adapter mAV_NANP (SEQ ID NO: 23) is exclusively coupled in the specific and stable manner to biotinylated NCs.

Assays similar to that described above were conducted for the purpose of determining the protein adaptor coupling capacity of mAV_NANP (SEQ ID NO: 23) to metabolically biotinylated NCs. To that end, NCs produced and purified from *S*. *cerevisiae* cultures transformed with plasmid pESCURA_BirA_SAP_BAP (SEQ ID NO: 19) were incubated with supernatants containing or not containing protein mAV_NANP (SEQ ID NO: 23). The results obtained are shown in Figure 8. As can be seen, metabolically biotinylated NCs are specifically coupled to protein adaptor mAV_NANP (SEQ ID NO: 23) (lane 2).

### LITERATURE

Kingsman SM, Kingsman AJ. Polyvalent recombinant antigens: a new vaccine strategy. Vaccine. 1988. 6:304-6.

Murray PK, Eaton BT. Vaccines for bluetongue. Aust Vet J. 1996. 73:207-10.

Casal JI, Rueda P, Hurtado A. Parvovirus-like particles as vaccine vectors. Methods. 1999. 19:174-86.

Maranga L, Cruz PE, Aunins JG, Carrondo MJ. Production of core and virus-like particles with baculovirus infected insect cells. Adv Biochem Eng Biotechnol. 2002. 74:183-206.

Yao Q, Bu z, Vzorov A, Yang C, Compans RW. Virus-like particle and DNA-based candidate AIDS vaccines. Vaccine. 2003. 21:638-43.

Maclean J, Rybicki EP, Williamson AL. Vaccination strategies for the prevention of cervical cancer. Expert Rev Anticancer Ther. 2005. 5:97-107.

Hammonds J, Chen X, Zhang X, Lee F, Spearman P. Advances in methods for the production, purification, and characterization of HIV-1 Gag-Env pseudovirion vaccines. Vaccine. 2007. 25:8036-48.

Niikura M, Takamura S, Kim G, Kawai S, Saijo M, Morikawa S, Kurane I, Li TC, Takeda N, Yasutomi Y. Chimeric recombinant hepatitis E virus-like particles as an oral vaccine vehicle presenting foreign epitopes. Virology. 2002. 293:273-80.

Varsani A, Williamson AL, de Villiers D, Becker I, Christensen ND, Rybicki EP. Chimeric human papillomavirus type 16 (HPV-16) L1 particles presenting the common neutralizing epitope for the L2 minor capsid protein of HPV-6 and HPV-16. J Virol. 2003. 77:8386-93.

Xu YF, Zhang YQ, Xu XM, Song GX. Papillomavirus virus-like particles as vehicles for the delivery of epitopes or genes. Arch Virol. 2006. 151:2133-48.

Mejia AF, Culp TD, Cladel NM, Balogh KK, Budgeon LR, Buck CB, Christensen ND. Preclinical model to test human papillomavirus virus (HPV) capsid vaccines in vivo using infectious HPV/cottontail rabbit papillomavirus chimeric papillomavirus particles. J Virol. 2006 80:12393-7.

Barker DF, Campbell AM. The birA gene of Escherichia coli encodes a biotin holoenzyme synthetase. J Mol Biol. 1981. 146:451-67.

Parrott MB, Barry MA. Metabolic biotinylation of secreted and cell surface proteins from mammalian cells. Biochem Biophys Res Commun. 2001. 281:993-1000.

Nesbeth D, Williams SL, Chan L, Brain T, Slater NK, Farzaneh F, Darling D. Metabolic biotinylation of lentiviral pseudotypes for scalable paramagnetic microparticle-dependent manipulation. Mol Ther. 2006. 13:814-22.

Barat B, Wu AM. Metabolic biotinylation of recombinant antibody by biotin ligase retained in the endoplasmic reticulum. Biomol Eng. 2007. 24:283-91.

Fernández-Arias A, Risco C, Martinez S, Albar JP, Rodriguez JF. Expression of ORF Al of infectious bursal disease virus results in the formation of virus-like particles. J Gen Virol. 1998 79:1047-54.

Saugar I, Luque D, Oña A, Rodriguez JF, Carrascosa JL, Trus BL, Castón JR. Structural polymorphism of the major capsid protein of a double-stranded RNA virus: an amphipathic alpha helix as a molecular switch. Structure. 2005. 13:1007-17.

Laitinen OH, Nordlund HR, Hytönen VP, Uotila ST, Marttila AT, Savolainen J, Airenne KJ, Livnah o, Bayer EA, Wilchek M, Kulomaa MS. Rational design of an active avidin monomer. Biol Chem. 2003. 278:4010-4.

Kochan G, Gonzalez D, Rodriguez JF. Characterization of the RNA-binding activity of VP3, a major structural protein of Infectious bursal disease virus. Arch Virol. 2003. 148:723-44.

Garriga D, Querol-Audi J, Abaitua F, Saugar I, Pous J, Verdaguer N, Castón JR, Rodriguez JF. The 2.6-Angstrom structure of infectious bursal disease virus-derived T=l particles reveals new stabilizing elements of the virus capsid. J Virol. 2006. 80:6895-905.

Castón JR, Martinez-Torrecuadrada JL, Maraver A, Lombardo E, Rodríguez JF, Casal JI, Carrascosa JL. C terminus of infectious bursal disease virus major capsid protein VP2 is involved in definition of the T number for capsid assembly. J Virol. 2001. 75:10815-28.

Beckett D, Kovaleva E, Schatz PJ. A minimal peptide substrate in biotin holoenzyme synthetase-catalyzed biotinylation. Protein Sci. 1999. 8:921-9.

Walters RW, Agbandje-McKenna M, Bowman VD, Moninger TO, Olson NH, Seiler M, Chiorini JA, Baker TS, Zabner J. Structure of adeno-associated virus serotype 5.J Virol. 2004. 78:3361-71.

Simpson AA, Chipman PR, Baker TS, Tijssen P, Rossmann MG. The structure of an insect parvovirus (Galleria mellonella densovirus) at 3.7 A resolution. Structure. 1998. 6:1355-67.

Bishop B, Dasgupta J, Klein M, Garcea RL, Christensen ND, Zhao R, Chen XS. Crystal structures of four types of human papillomavirus L1 capsid proteins: understanding the specificity of neutralizing monoclonal antibodies. J Biol Chem. 2007. 282:31803-11.

van den Berg TP et al. 2000. Rev Sci Tech. 19:509-43.

Sánchez AB & Rodriguez JF. Proteolytic processing in infectious bursal disease virus: identification of the polyprotein cleavage sites by site-directed mutagenesis. Virology. 1999 Sep 15; 262(1):190-199.

Altschul et al. 1997. Nucleic Acids Res. 25:3389.

Sambrook and Russell. 2001. Molecular Cloning: A Laboratory Manual. 3rd Ed. Cold Spring Harbor Laboratory Press, Cold Spring Harbor, N.Y.

Leusch MS et al. 1995. A novel host-vector system for direct selection of recombinant baculoviruses (bacmids) in Escherichia coli. Gene 160:91-4.

Luckow VA et al. 1993. Efficient generation of infectious recombinant baculoviruses by site-specific transposon-mediated insertion of foreign genes into a baculovirus genome propagated in Escherichia coli. J Virol 67:4566-79.

## Claims

1. A fusion protein comprising
(i) a self assembly protein, and
(ii) a heterologous peptide comprising an amino acid sequence with the capacity to bind to a ligand.

2. A fusion protein according to claim 1, wherein said heterologous peptide comprising an amino acid sequence with the capacity to bind to a ligand comprises a biotin acceptor peptide (BAP).

3. A fusion protein according to claim 2, wherein said EAP comprises the amino acid sequence shown in SEQ ID NO: 3.

4. A fusion protein according to any of claims 1 to 3, wherein said heterologous peptide comprising an amino acid sequence with the capacity to bind to a ligand is inserted inside the amino acid sequence of said self assembly protein.

5. A fusion protein according to any of claims 1 to 3, wherein said heterologous peptide comprising an amino acid sequence with the capacity to bind to a ligand is bound to the amino- or carboxyl-terminal end of said self assembly protein.

6. A fusion protein according to claim 1, wherein said self assembly protein comprises infectious bursal disease virus (IBDV) protein VP2 (pVP2) or a functional variant thereof, preferably a protein comprising the amino acid sequence comprised between residue 1 and residue 452 of IBDV pVP2 (IBDV pVP2_452).

7. A fusion protein according to claim 6, wherein said heterologous peptide comprising an amino acid sequence with the capacity to bind to a ligand comprises a biotin acceptor peptide (BAP).

8. A fusion protein according to claim 7, wherein said BAP comprises the amino acid sequence shown in SEQ ID NO: 3.

9. A fusion protein according to any of claims 6 to 8, wherein said heterologous peptide comprising an amino acid sequence with the capacity to bind to a ligand is inserted inside the amino acid sequence of said self assembly protein.

10. A fusion protein according to claim 9, wherein said peptide insert is inside loop P_{HI} of said IBDV pVP2 or functional variant thereof.

11. A fusion protein according to claim 1, wherein said ligand comprises a conjugate comprising a product of interest.

12. A nanocarrier which can be obtained by the assembly of a fusion protein according to any of claims 1 to 11.

13. A nanocarrier according to claim 12, further comprising a product of interest bound to said fusion protein through a ligand with the capacity to bind to the peptide insert contained in said fusion protein.

14. A nanocarrier according to claim 12, further comprising a product of interest bound to said fusion protein through a dual connector binding to the peptide insert contained in said fusion protein on one hand and to a ligand to which said product of interest is bound on the other hand.

15. A nanocarrier according to claim 12 or 14, wherein said peptide insert comprises a biotin acceptor peptide (BAP).

16. A nanocarrier according to claim 12, wherein said peptide insert comprises a biotin acceptor peptide (BAP), and said BAP is bound to biotin.

17. A composition comprising a nanocarrier according to any of claims 12 to 16 in an inert medium.

18. A pharmaceutical composition comprising a therapeutically effective amount of a nanocarrier according to any of claims 12 to 16, together with, optionally, a pharmaceutically acceptable carrier and/or adjuvant.

19. The use of a nanocarrier according to any of claims 12 to 16 as a carrier for products of interest or as a vector in gene therapy.

20. A process for producing a nanocarrier according to claim 12, which comprises culturing a host cell containing a polynucleotide comprising the nucleotide sequence encoding the fusion protein defined in any of claims 1 to 11, under conditions allowing the expression of said polynucleotide, and, if desired, the recovery of said nanocarrier.

21. A process for producing a nanocarrier according to claim 13, which comprises putting a nanocarrier according to claim 12 in contact with a conjugate comprising (i) a ligand with the capacity to bind to the peptide insert contained in a fusion protein according to claim 1, and (ii) a product of interest, under conditions allowing the binding of the ligand to the peptide insert, and, if desired, the recovery of said nanocarrier.

22. A process for producing a nanocarrier according to claim 14, which comprises putting a nanocarrier according to claim 12 in contact with a connector and a conjugate comprising (i) a ligand with the capacity to bind to the peptide insert contained in a fusion protein according to claim 1, and (ii) a product of interest, under conditions allowing the binding of the ligand to the peptide insert through said connector, and, if desired, the recovery of said nanocarrier.

23. A process for obtaining a nanocarrier according to claim 16, wherein said peptide insert comprises a biotin acceptor peptide (BAP), and said BAP is bound to biotin, which comprises
culturing a host cell containing (i) a polynucleotide comprising the nucleotide sequence encoding a fusion protein defined in any of claims 1 to 11, wherein said peptide insert comprises a BAP and, furthermore, (ii) a polynucleotide encoding the biotin ligase (BL) enzyme, in the presence of biotin, under conditions allowing the expression of said fusion protein and its assembly to form nanocarriers, the expression of said BL enzyme, and furthermore the specific interaction between said BAP present in said nanocarriers and biotin, and, if desired, the recovery of said biotinylated nanocarriers; or alternatively,
culturing a host cell of the invention comprising a polynucleotide comprising the nucleotide sequence encoding a fusion protein defined in any of claims 1 to 11, wherein said peptide insert comprises a BAP, in a medium comprising the biotin ligase (BL) enzyme and biotin, under conditions allowing the expression of said fusion protein, its secretion and assembly to form nanocarriers, and furthermore the specific interaction between said BAP present in said nanocarriers and biotin, and, if desired, the recovery of said biotinylated nanocarriers; or alternatively,
putting in contact a nanocarrier according to claim 15 the peptide insert of which comprises a biotin acceptor peptide (BAP), in a medium comprising the biotin ligase (BL) enzyme and biotin, under conditions allowing the specific interaction between said BAP present in said nanocarriers and biotin, and, if desired, the recovery of said biotinylated nanocarriers.

24. A polynucleotide comprising the nucleotide sequence encoding the fusion protein defined in any of claims 1 to 11.

25. An expression system comprising a polynucleotide according to claim 24, operatively bound to transcription and, optionally, translation control elements.

26. A host cell containing a polynucleotide according to claim 24, or a vector according to claim 25.
